# EUROPEAN PATENT SPECIFICATION

(11) **EP 3 286 212 B1**
(45) Date of publication and mention of the grant of the patent: **02.06.2021**
(21) Application number: 16718817.6
(22) Date of filing: 14.04.2016
(51) Int. Cl.: C07K 14/78, A61K 38/00

(54) **POLYPEPTIDES TARGETING HIV FUSION**
POLYPEPTIDE ZUM TARGETING DER HIV-FUSION
POLYPEPTIDES CIBLANT UNE FUSION DU VIH

(30) Priority: 24.04.2015 US 201562152271 P; 19.11.2015 US 201562257474 P
(43) Date of publication of application: 28.02.2018
(73) Proprietor: VIIV Healthcare UK (No.5) Limited, Brentford Middlesex TW8 9GS (GB)
(72) Inventor: KRYSTAL, Mark, R., Westport, CT 06880 (US); WENSEL, David, L., Waltham, MA 02453 (US); DAVIS, Jonathan, Waltham, MA 02453 (US)
(74) Representative: Learoyd, Stephanie Anne
(86) International application number: PCT/US2016/027424
(87) International publication number: WO 2016/171980

(56) References cited:
- WO-A1-2011/077093
- WO-A1-2011/130354
- WO-A1-2014/206336
- US-A1- 2009 022 720
- HAQQANI AIMAN A ET AL: "Entry inhibitors and their use in the treatment of HIV-1 infection", ANTIVIRAL RESEARCH, ELSEVIER BV, NL, vol. 98, no. 2, 28 March 2013 (2013-03-28) , pages 158-170, XP028531684, ISSN: 0166-3542, DOI: 10.1016/J.ANTIVIRAL.2013.03.017

## Description

### FIELD OF THE INVENTION

The invention is directed to polypeptides comprising a CD4 binding moiety, a gp41 binding moiety, a HIV fusion peptide inhibitor moiety and combinations thereof. More specifically, the present invention relates to polypeptides comprising a fibronectin-based scaffold domain protein that binds CD4, a fibronectin-based scaffold domain protein that binds the N17 domain of gp41, and a HIV fusion peptide inhibitor or combinations thereof. The invention also relates to the use of the innovative proteins in therapeutic applications to treat HIV.

### BACKGROUND OF THE INVENTION

Acquired immunodeficiency syndrome (AIDS) is the result of infection by the retrovirus known as human immunodeficiency virus (HIV). It remains a major medical problem, with an estimated 35 million people infected worldwide at the end of 2013. During that year, there were 2.1 million new infections, with 1.5 million people dying from complications due to AIDS.

Current therapy for HIV-infected individuals consists of a combination of approved anti-retroviral agents. Over two dozen drugs are currently approved for HIV infection, either as single agents, fixed dose combinations or single tablet regimens, the latter two containing 2-4 approved agents. These agents belong to a number of different classes, targeting either a viral enzyme or the function of a viral protein during the virus life cycle. Thus, agents are classified as either nucleotide reverse transcriptase inhibitors (NRTIs), non-nucleotide reverse transcriptase inhibitors (NNRTIs), protease inhibitors (PIs), integrase inhibitors (INIs), or entry inhibitors (one entry inhibitor, maraviroc, targets the host CCR5 protein, while the other, enfuvirtide, is a peptide that targets the gp41 region of the viral gp160 protein). In addition, a pharmacokinetic enhancer with no antiviral activity (cobicistat) has been approved for use in combinations with antiretroviral agents (ARVs) that require boosting.

Despite the armamentarium of agents and drug combinations, there remains a medical need for new anti-retroviral agents, due in part to the need for chronic dosing to combat infection. Significant problems related to long-term toxicities are documented, creating a need to address and prevent these co-morbidities (e.g., CNS, CV/metabolic, renal disease). Also, increasing failure rates on current therapies continue to be a problem, due either to the presence or emergence of resistant strains or to non-compliance attributed to drug holidays or adverse side effects. For example, despite therapy, it has been estimated that 63% of subjects receiving combination therapy remained viremic, as they had viral loads >500 copies/ml (Oette, M. et al., "Primary HIV Drug Resistance and Efficacy of First-Line Antiretroviral Therapy Guided by Resistance Testing", J. Acq. Imm. Def. Synd., 41(5):573-581 (2006)). Among these patients, 76% had viruses that were resistant to one or more classes of antiretroviral agents. As a result, new drugs are needed that are more convenient, have high genetic barriers to the development of resistance and have improved safety over current agents.

It is now well known that cells can be infected by HIV through a process by which fusion occurs between the cellular membrane and the viral membrane. The generally accepted model of this process is that the viral envelope glycoprotein complex (gp120/gp41) interacts with cell surface receptors on the membranes of the target cells. Following binding of gp120 to cellular receptors (*e.g.,* CD4 in combination with a chemokine co-receptor such as CCR5 or CXCR4), a conformational change is induced in the gp120/gp41 complex that allows gp41 to insert into the membrane of the target cell and mediate membrane fusion. As these entry processes occur on the cell membrane, they are amenable for inhibition by macrocmolecules, which include biologic peptides (Haqqani et al., Antiviral Res., 98:158 (2013)). For instance, the approved antiviral peptide enfuvirtide (FUZEON®) targets a region of gp41 involved in membrane fusion. Larger polypeptides such as monoclonal antibodies can also inhibit different aspects of virus entry. A monoclonal antibody targeting the first step of virus entry, interaction with the cellular receptor CD4 (ibalizumab; Bruno et al., J. Antimicrob. Chemother., 65:1839 (2010)), as well as a monoclonal antibody targeting the co-receptor CCR5 (PRO-140; Tenorio, Curr. HIV/AIDS Rep., 8:1 (2011)) have both shown positive results in Phase 2a trials. These antibodies also have the property of being long acting antiretrovirals, with potential dosing regimens of weekly to monthly (Jacobson et al., J. Infect. Dis., 201:1481 (2010); Jacobson et al., Antimicrob. Agents Chemother., 53:450 (2009)).

Another property of peptide entry inhibitors is that enhanced or synergistic potency can be obtained if two peptide inhibitors are attached to each other, or if a single inhibitor is localized near the site of action through binding to membrane biomolecules. Thus, attaching a fusion peptide inhibitor to a monoclonal antibody targeting CCR5 (Kopetzki et al., Virol. J., 5:56 (2008)) or attaching a cholesterol moiety to the C-terminus of a peptide fusion inhibitor to place it at the surface of the target cell membrane (Ingallinela et al., Proc. Natl. Acad. Sci. USA, 106:5801 (2009); Augusto et al., J. Antimicrob. Chemother., 69:1286 (2014)) drastically increases the potency of the combined molecule compared to the separate molecules. Similarly, bispecific antibodies consisting of anti-HIV-1 neutralizing antibody fragments targeting gp120 fused to ibalizumab showed synergistic increases in potency compared to the individual inhibitors (Sun et al., J. Acquir. Immune Defic. Syndr., 66:473 (2014)). The Combinectin molecules of the invention makes use of these various properties.

WO2011/077093 discloses virus fusion inhibitors or virus entry inhibitors which are bivalent molecules comprising a first part capable of mimicking the function of a mammalian cell receptor, and a second part capable of binding to a virus, preferably HIV, resulting in the neutralization of the virus.

### SUMMARY OF THE INVENTION

The invention is directed to polypeptides comprising a CD4 binding moiety, a gp41 binding moiety, a HIV fusion peptide inhibitor moiety and combinations thereof.

One embodiment of the invention is directed to polypeptides comprising a fibronectin-based scaffold domain protein that binds CD4, a fibronectin-based scaffold domain protein that binds the N17 domain of gp41, and a HIV fusion peptide inhibitor.

In one embodiment of the invention, the three domains are connected to each other by linkers. In another embodiment of the invention, the three domains may be connected to each other in any order. In another embodiment of the disclosure, the polypeptide comprises an amino acid sequence at least 80%, 85%, 90%, 95%, 98%, 99% or 100% identical to the non-linker regions of SEQ ID NO: 3, 5, 7 or 9.

The disclosure is also directed to polypeptides comprising a fibronectin-based scaffold domain protein that binds CD4 and a fibronectin-based scaffold domain protein that binds the N17 domain of gp41. In one embodiment of the disclosure, the two domains are connected to each other by linkers. In another embodiment of the disclosure, the two domains may be connected to each other in any order.

The invention is also directed to polypeptides comprising a fibronectin-based scaffold domain protein that binds CD4 and a HIV fusion peptide inhibitor. Thus, according to one aspect of the invention, there is provided a polypeptide comprising two active domains wherein one domain is an anti-CD4 Adnectin protein, wherein the anti-CD4 Adnectin protein comprises an amino acid sequence at least 80%, 85%, 90%, 95%, 98%, 99% or 100% identical to the amino acid sequence of SEQ ID NOs: 95-114 and the other domain is a HIV fusion peptide inhibitor moiety wherein the HIV fusion peptide inhibitor comprises an amino acid sequence at least 80%, 85%, 90%, 95%, 98%, 99% or 100% identical to the amino acid sequence of SEQ ID NO: 372-392.

In one embodiment of the invention, the two domains are connected to each other by linkers. In another embodiment of the invention, the two domains may be connected to each other in any order.

The disclosure is also directed to polypeptides comprising a fibronectin-based scaffold domain protein that binds the N17 domain of gp41, and a HIV fusion peptide inhibitor. In one embodiment of the disclosure, the two domains are connected to each other by linkers. In another embodiment of the disclosure, the two domains may be connected to each other in any order. In another embodiment of the disclosure, the polypeptide comprises an amino acid sequence at least 80%, 85%, 90%, 95%, 98%, 99% or 100% identical to the non-linker regions of SEQ ID NO: 410-428.

Another embodiment of the invention is also directed to polypeptides comprising three active domains, a fibronectin-based scaffold domain protein that binds CD4, a gp41 binding moiety and a HIV fusion peptide inhibitor moiety. The invention is also directed to polypeptides comprising a fibronectin-based scaffold domain protein that binds gp41, a CD4 binding moiety and a HIV fusion peptide inhibitor moiety. The invention is also directed to polypeptides comprising a CD4 binding moiety, a gp41 binding moiety and a HIV fusion peptide inhibitor. Thus, according to a further aspect of the invention, there is provided a polypeptide comprising three active domains wherein one domain is an anti-CD4 Adnectin protein, wherein the anti-CD4 Adnectin protein comprises an amino acid sequence at least 80%, 85%, 90%, 95%, 98%, 99% or 100% identical to the amino acid sequence of SEQ ID NOs: 95-114, a second domain is an anti-N17 Adnectin protein, wherein the anti-N17 Adnectin protein comprises an amino acid sequence at least 80%, 85%, 90%, 95%, 98%, 99% or 100% identical to the amino acid sequence of DEQ ID NO: 115-371 and the third domain is a HIV fusion peptide inhibitor wherein the HIV fusion peptide inhibitor comprises an amino acid sequence at least 80%, 85%, 90%, 95%, 98%, 99% or 100% identical to the amino acid sequence of SEQ ID NO: 372-392.

In one embodiment of the invention, the three domains are connected to each other by linkers. In another embodiment of the invention, the three domains may be connected to each other in any order.

The disclosure is also directed to polypeptides comprising two active domains, a fibronectin-based scaffold domain protein that binds CD4 and a gp41 binding moiety. The disclosure is also directed to polypeptides comprising a fibronectin-based scaffold domain protein that binds gp41 and a CD4 binding moiety. The invention is also directed to polypeptides comprising a CD4 binding moiety and a HIV fusion peptide inhibitor. The disclosure is also directed to polypeptides comprising a gp41 binding moiety and a HIV fusion peptide inhibitor. In one embodiment of the disclosure, the two domains are connected to each other by linkers. In another embodiment of the disclosure, the two domains may be connected to each other in any order.

Another embodiment of the invention is also directed to anti-CD4 Adnectin, anti-N17 Adnectin, or HIV fusion peptide inhibitors. In another embodiment of the invention, the polypeptide comprises an amino acid sequence at least 80%, 85%, 90%, 95%, 98%, 99% or 100% identical to the non-linker regions of SEQ ID NO: 95-114 or SEQ ID NO: 115-371 or SEQ ID NO: 372-392, respectively.

In another embodiment of the invention, a pharmacokinetic (PK) moiety is attached to the polypeptides of the invention. Examples of a PK moiety include but are not limited to polyethylene glycol, sialic acid, Fc, Fc fragment, transferrin, serum albumin (HSA), a serum albumin binding protein, and a serum immunoglobulin binding protein. In one embodiment of the invention, the PK moiety may be attached to the linker regions, or the N- or C-terminus of the polypeptide of the invention. In another embodiment of the disclosure, the polypeptide comprises an amino acid sequence at least 80%, 85%, 90%, 95%, 98%, 99% or 100% identical to the non-linker regions of SEQ ID NO: 4, 6, 8 or 10.

The disclosure is also directed to a polypeptide comprising the amino acid sequence of any one of the sequences shown in SEQ ID NO: 3-10.

The invention is also directed to a pharmaceutical composition comprising one or more of the polypeptides of the invention and a carrier.

The disclosure also provides a method of treating HIV in a subject comprising administering an effective amount of the polypeptides of the invention.

In a further aspect of the invention, there is provided the polypeptide of the invention for use in therapy. In a yet further aspect of the invention, there is provided the polypeptide of the invention for use in treating HIV.

### BRIEF DESCRIPTION OF THE FIGURES

FIG. 1 is a diagram of alternative Combinectins. One of the Adnectin binds CD4, a second Adnectin binds the HR1 region of gp41. The peptide also binds in the HR1 region of gp41. The different components of the Combinectin are connected to each other in any order by linkers. Any of the Combinectins may have a PK moiety attached, such as HSA or Fc.
FIG. 2 shows the amino acid sequences of Fc fusion-Combinectin 3137(SEQ ID NO:4), 3151(SEQ ID NO:6) and human serum albumin (HSA) fusion-Combinectin 3191(SEQ ID NO:8), and 3202(SEQ ID NO:10). The Fc and HSA sequences are in bold. The anti-CD4 Adnectin sequences are underlined. The anti-N17 Adnectin sequences are double underlined. The HIV inhibitor peptide sequences are underlined in bold. The linker sequences are italicized.

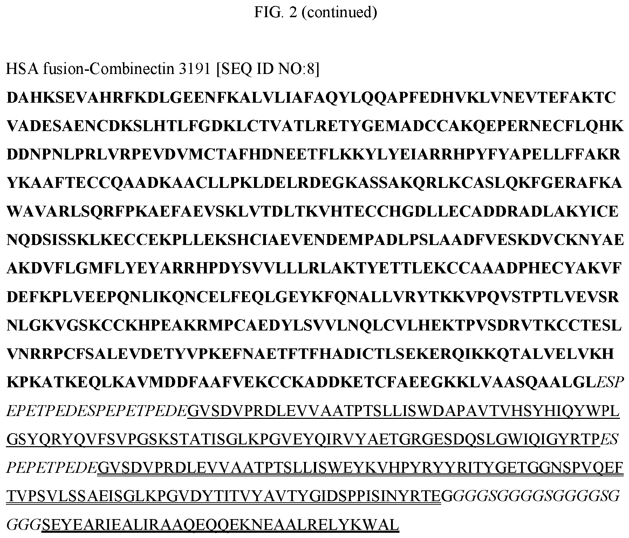

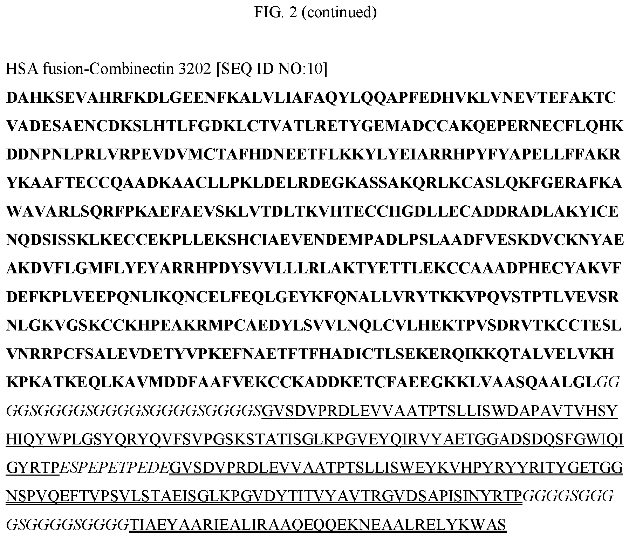

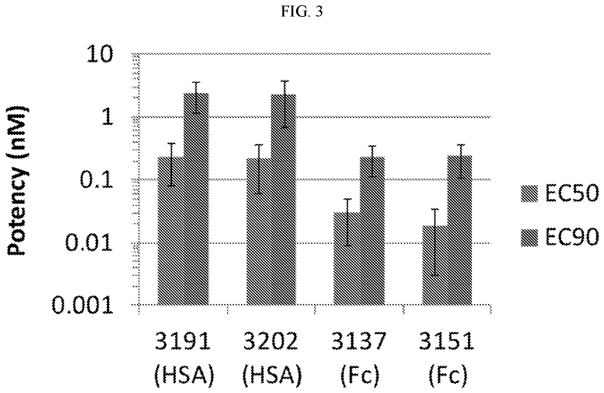

FIG. 3 shows the potency (EC₅₀ and EC₉₀) of Combinectin 3137, 3151, 3191 and 3202, as described in Example 2.

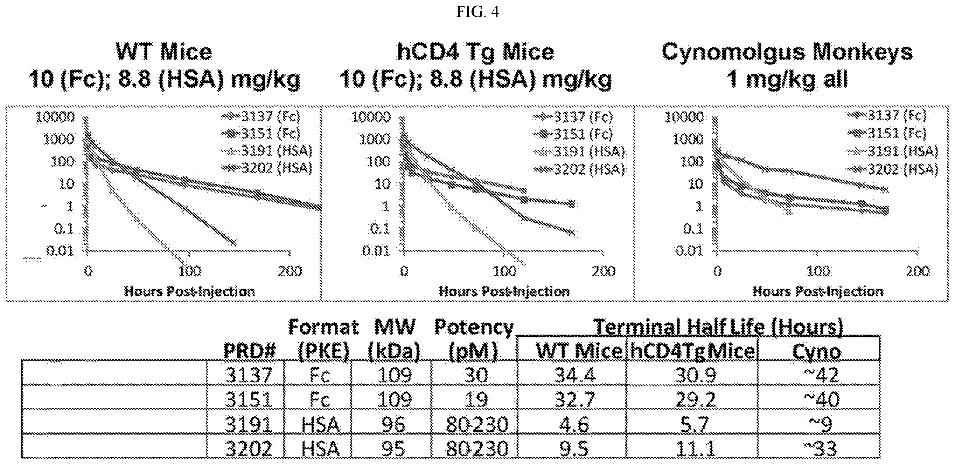

FIG. 4 shows the PK properties of Combinectin 3137, 3151, 3191 and 3202 as described in Example 3.

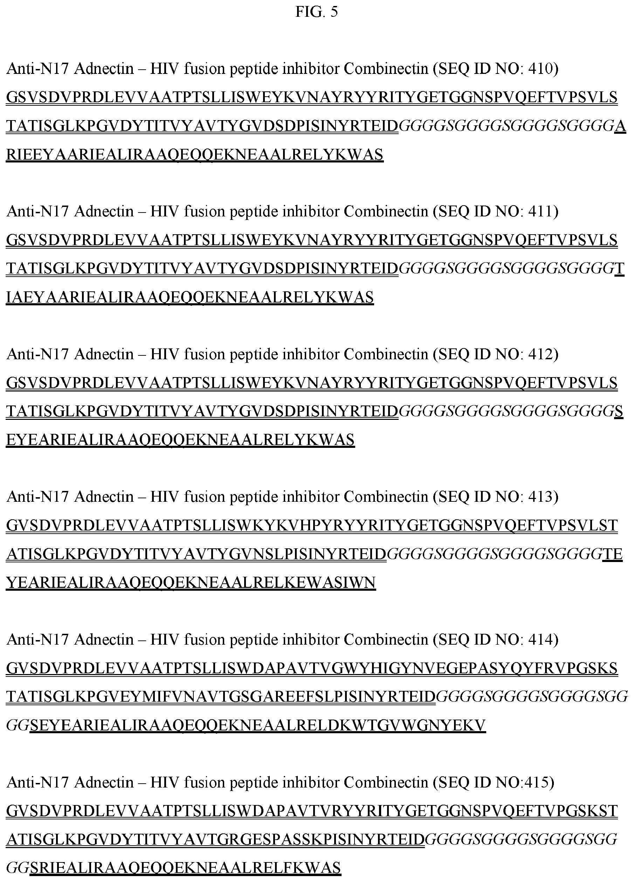

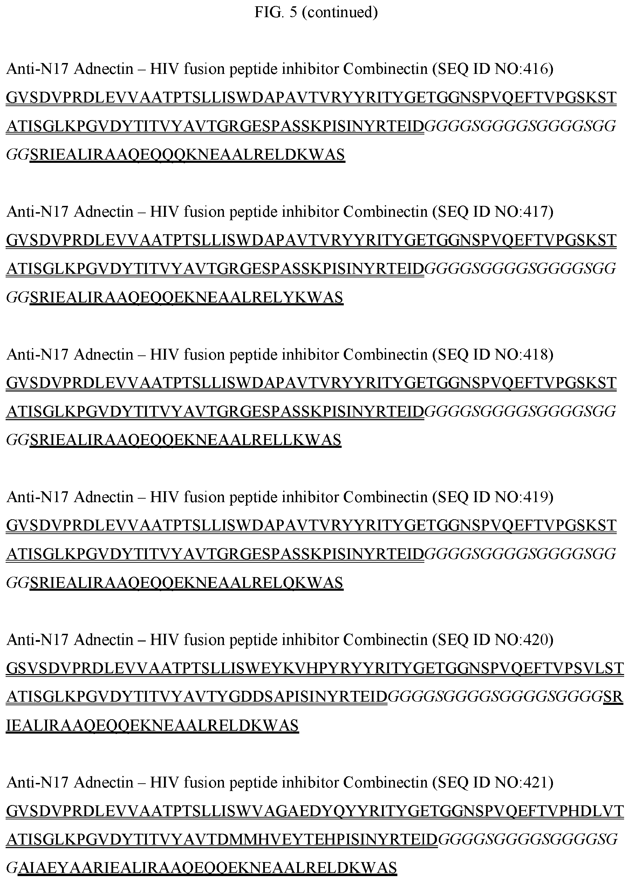

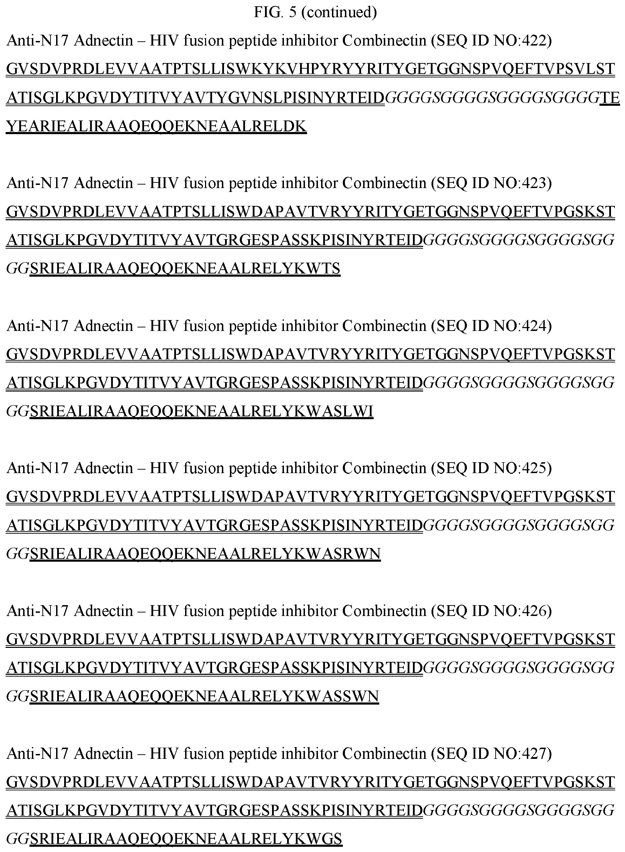

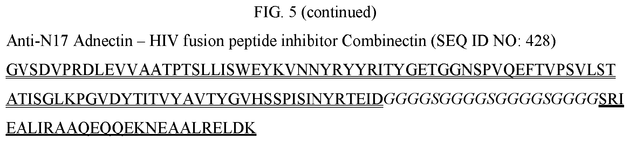

FIG. 5 shows the amino acid sequences of anti-N17 Adnectin in combination with an HIV fusion peptide inhibitor, which correspond to the sequences described in Table 4 minus the N-terminus and C-terminus extended regions. The anti-N17 Adnectin sequences are double underlined. The HIV fusion peptide inhibitor sequences are underlined in bold. The linker sequences are italicized.

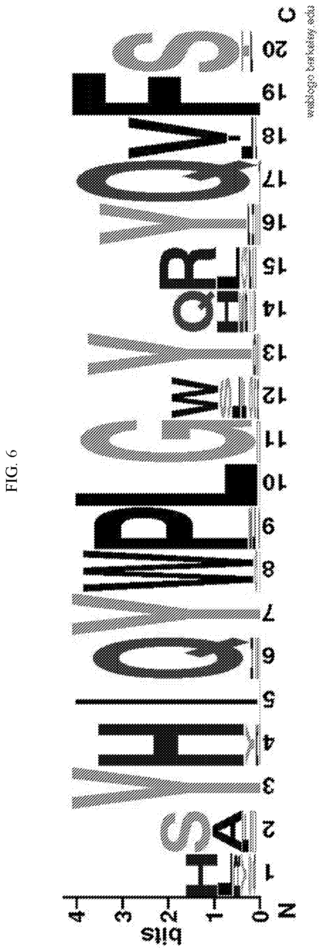

FIG. 6 shows the WebLogo for the CD loop of anti-CD4 Adnectin. WebLogo generates sequence logos, graphical representations of the patterns within a multiple sequence alignment. Each logo consists of stacks of letters, one stack for each position in the sequence. The overall height of each stack indicates the sequence conservation at that position (measured in bits), whereas the height of symbols within the stack reflects the relative frequency of the corresponding amino or nucleic acid at that position (Crooks, G.E. et al., "WebLogo: A sequence logo generator", Genome Research, 14:1188-1190 (2004)).
FIG. 7 shows the WebLogo for the FG loop of anti-CD4 Adnectin (Schneider, T.D. et al., "Sequence Logos: A New Way to Display Consensus Sequences", Nucleic Acids Res., 18:6097-6100 (1990)).
FIG. 8 shows point mutation data for the HIV fusion peptide inhibitor. The aspartic acid (D) near the C-terminus of the HIV fusion peptide inhibitor was replaced with the amino acids listed along the x axis.

### DETAILED DESCRIPTION OF THE INVENTION

### Definitions

Unless defined otherwise, all technical and scientific terms used herein have the same meaning as commonly understood by the skilled artisan. Although any methods and compositions similar or equivalent to those described herein can be used in practice or testing of the present invention, the preferred methods and compositions are described herein.

"Polypeptide" as used herein refers to any sequence of two or more amino acids, regardless of length, post-translation modification, or function. "Polypeptide", "peptide", and "protein" are used interchangeably herein. Polypeptides can be modified in any of a variety of standard chemical ways (e.g., an amino acid can be modified with a protecting group; the carboxy-terminal amino acid can be made into a terminal amide group; the amino-terminal residue can be modified with groups to, *e.g.,* enhance lipophilicity; or the polypeptide can be chemically glycosylated or otherwise modified to increase stability or *in vivo* half-life). Polypeptide modifications can include the attachment of another structure such as a cyclic compound or other molecule to the polypeptide and can also include polypeptides that contain one or more amino acids in an altered configuration (*i.e.,* R or S; or, L or D).

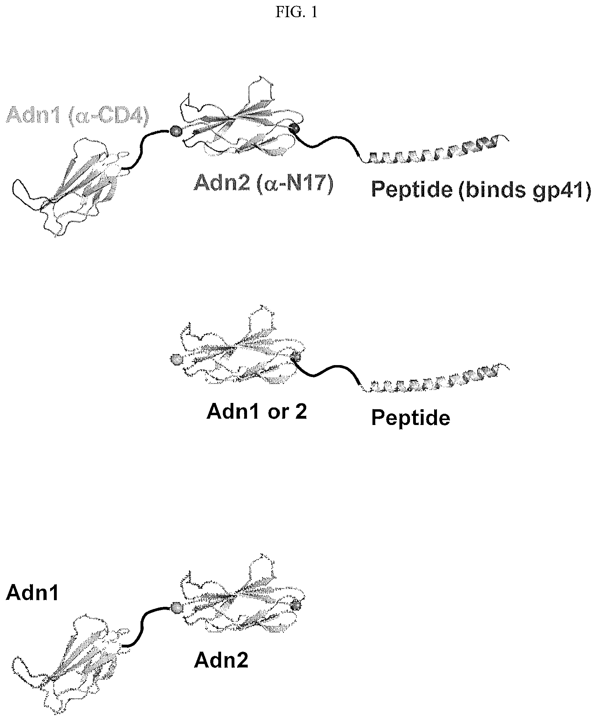

The peptides of the invention may include, for example, proteins derived from the tenth type III domain of fibronectin that have been modified to bind to CD4 or N17 domain of gp41 and are referred to herein as, "anti-CD4 Adnectin", "anti-N17 Adnectin", "CD4 Adnectin" or "gp41 Adnectin". The polypeptides of the invention may also include peptides modeled after the heptad repeat 2 (HR2) region of HIV envelope glycoprotein gp41, which inhibit fusion by binding the heptad repeat 1 (HR1) region of gp41 and are referred to herein as "HIV fusion peptide inhibitor" or "fusion peptide inhibitor". The polypeptides of the invention also include "Combinectins" comprising an anti-CD4 Adnectin linked to an anti-N17 Adnectin linked to a HIV fusion peptide inhibitor (FIG. 1). Alternatively, the Combinectin comprises an anti-CD4 Adnectin linked to an anti-N17 Adnectin or an anti-N17 Adnectin linked to a HIV fusion peptide inhibitor or an anti-CD4 Adnectin linked to a HIV fusion peptide inhibitor.

A "polypeptide chain", as used herein, refers to a polypeptide wherein each of the domains thereof is joined to other domain(s) by peptide bond(s), as opposed to noncovalent interactions or disulfide bonds.

An "isolated" polypeptide is one that has been identified and separated and/or recovered from a component of its natural environment. Contaminant components of its natural environment are materials that would interfere with diagnostic or therapeutic uses for the polypeptide, and may include recombinant host cell proteins and other proteinaceous or nonproteinaceous solutes. In one embodiment, the polypeptides will be purified (1) to greater than 95% by weight of polypeptide as determined by the Lowry method, and most preferably more than 99% by weight, or (2) to homogeneity by SDS-PAGE under reducing or nonreducing condition using Coomassie blue or, silver stain. Ordinarily, isolated polypeptide will be prepared by at least one purification step.

"Percent (%) amino acid sequence identity" herein is defined as the percentage of amino acid residues in a candidate sequence that are identical with the amino acid residues in a selected sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR®) software. Those skilled in the art can readily determine appropriate parameters for measuring alignment, including any algorithms needed to achieve maximal alignment over the full-length of the sequences being compared. For example, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

As used herein, "conservative substitution" denotes the replacement of an amino acid residue by another, without altering the overall conformation and function of the peptide, including, but not limited to, replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine. By "substituted" or "modified" the present invention includes those amino acids that have been altered or modified from naturally occurring amino acids. As such it should be understood that in the context of the present invention a conservative substitution is recognized in the art as a substitution of one amino acid for another amino acid that has similar properties.

As used herein, the term "binding site" refers to the site or portion of a protein *(e.g.,* CD4, gp41) that interacts or binds to a particular protein of the invention *(e.g.,* as an epitope is recognized by an antibody). Binding sites can be formed from contiguous amino acids or noncontiguous amino acids juxtaposed by tertiary folding of a protein. Binding sites formed by contiguous amino acids are typically retained on exposure to denaturing solvents, whereas binding sites formed by tertiary folding are typically lost on treatment of denaturing solvents.

The binding site for an anti-CD4 moiety or anti-N17moiety of the invention may be determined by application of standard techniques typically used for epitope mapping of antibodies including, but not limited to protease mapping and mutational analysis. Alternatively, a binding site can be determined by competition assay using a reference protein (*e.g.,* another Adnectin or antibody) which binds to the same polypeptide, *e.g.,* CD4 or gp41. If the test protein and reference molecule (e.g., another Adnectin or antibody) compete, then they bind to the same binding site or to binding sites sufficiently proximal such that binding of one molecule interferes with the other.

The terms "specifically binds", "specific binding", "selective binding", and "selectively binds", as used interchangeably herein refers to a protein that exhibits affinity for a CD4 or gp41, but does not significantly bind (*e.g.,* less than about 10% binding) to a different polypeptide as measured by a technique available in the art such as, but not limited to, Scatchard analysis and/or competitive binding assays (*e.g*., competition ELISA, BIACORE® SPR assay). The term is also applicable where *e.g.,* a binding domain of a protein of the invention is specific for CD4 or gp41.

The term "preferentially binds" as used herein refers to the situation in which the peptides of the invention bind CD4 or gp41at least about 20% greater than it binds a different polypeptide as measured by a technique available in the art such as, but not limited to, Scatchard analysis and/or competitive binding assays (e.g., competition ELISA, BIACORE® SPR assay).

As used herein, the term "cross-reactivity" refers to a protein which binds to more than one distinct protein having identical or very similar binding sites.

The term "*K*_{d}", as used herein, is intended to refer to the dissociation equilibrium constant of a particular Adnectin-protein, fusion peptide inhibitor-protein or Combinectin-protein (*e.g.,* CD4 and/or gp41) interaction or the affinity of an Adnectin, fusion peptide inhibitor or Combinectin for a protein (*e.g.,* CD4 and/or gp41), as measured using a surface plasmon resonance assay or a cell binding assay. A "desired *K*_{d}", as used herein, refers to a *K*_{d} of a protein of the invention that is sufficient for the purposes contemplated. For example, a desired *K*_{d} may refer to the *K*_{d} of a Combinectin required to elicit a functional effect in an *in vitro* assay, *e.g.,* a cell-based luciferase assay.

The term "*k*ₒₙ", as used herein, is intended to refer to the association rate constant for the association of, for example, a Combinectin into the Combinectin/protein complex.

The term "*k*_{off}", as used herein, is intended to refer to the dissociation rate constant for the dissociation of, for example, a Combinectin from the Combinectin/protein complex.

The term "IC₅₀", as used herein, refers to the concentration of, for example, a Combinectin that inhibits a response, either in an *in vitro* or an *in vivo* assay, to a level that is 50% of the maximal inhibitory response, *i.e.,* halfway between the maximal inhibitory response and the untreated response.

The terms "inhibit" or "neutralize" as used herein with respect to an activity of a protein of the invention means the ability to substantially antagonize, prohibit, prevent, restrain, slow, disrupt, eliminate, stop, reduce or reverse *e.g*., progression or severity of that which is being inhibited including, but not limited to, a biological activity or property, a disease or a condition. The inhibition or neutralization is preferably at least about 10%, 20%, 30%, 40%, 50%, 60%, 70%, 80%, 90%, 95% or higher.

The term "PK" is an acronym for "pharmacokinetic" and encompasses properties of a compound including, by way of example, absorption, distribution, metabolism, and elimination by a subject. A "PK modulation protein" or "PK moiety" as used herein refers to any protein, peptide, or moiety that affects the pharmacokinetic properties of a biologically active molecule when fused to or administered together with the biologically active molecule. Examples of a PK modulation protein or PK moiety include PEG, human serum albumin (HSA) binders (as disclosed in U.S. Publication No. 2005/0287153, U.S. Patent No. 7,696,320, PCT Publication Nos. WO 2009/083804 and WO 2009/133208), human serum albumin, Fc or Fc fragments and variants thereof, and sugars (e.g., sialic acid).

The term "CD4 binding moiety" refers to any moiety that blocks HIV surface protein gp120 binding to the CD4 receptor on CD4+ T cells. The CD4 binding moiety may be anti-CD4 -adnectin, -antibody(such as ibalizumab), -domain antibody (dAb), -antibody fragments (Fab), -bispecific antibody and fusion protein thereof.

The term "gp41 binding moiety" refers to any moiety that interferes with the interaction of the viral envelope glycoprotein complex (gp120/gp41) with T cells. The gp41 binding moiety may be anti-gp41 -adnectin, -antibody (Ab), -domain antibody (dAb), -antibody fragments (Fab), -bispecific antibody and fusion protein thereof.

A "HIV fusion peptide inhibitor moiety" refers to any moiety that inhibits fusion by binding the heptad repeat 1 (HR1) region of gp41. Examples of fusion peptide inhibitor moiety include peptides derived from the NHR and CHR regions of gp41, designated NHR and CHR peptides, respectively. Enfuvirtide is an example of a CHR peptide.

The peptides of the disclosure may include, for example, the CD4 monoclonal antibody ibalizumab, an anti-N17 Adnectin and a HIV fusion peptide inhibitor. Alternatively, the peptides of the invention may include an anti-CD4 Adnectin, an anti-N17 Adnectin and the HIV fusion peptide inhibitor enfuvirtide.

The "half-life" of an amino acid sequence or compound can generally be defined as the time taken for the serum concentration of the polypeptide to be reduced by 50%, *in vivo,* for example, due to degradation of the sequence or compound and/or clearance or sequestration of the sequence or compound by natural mechanisms. The half-life can be determined in any manner known per se, such as by pharmacokinetic analysis. Suitable techniques will be clear to the person skilled in the art, and may, for example, generally involve the steps of suitably administering to a subject a suitable dose of the amino acid sequence or compound of the invention; collecting blood samples or other samples from the subject at regular intervals; determining the level or concentration of the amino acid sequence or compound of the invention in said blood sample; and calculating, from (a plot of) the data thus obtained, the time until the level or concentration of the amino acid sequence or compound of the invention has been reduced by 50% compared to the initial level upon dosing. Reference is, for example, made to the standard handbooks, such as Kenneth, A. et al., Chemical Stability of Pharmaceuticals: A Handbook for Pharmacists and in Peters et al., Pharmacokinetic Analysis: A Practical Approach (1996). Reference is also made to Gibaldi, M. et al., Pharmacokinetics, Second Rev. Edition, Marcel Dekker (1982).

Half-life can be expressed using parameters such as the t_{1/2}-alpha, t_{1/2}-beta, HL_Lambda_z, and the area under the curve (AUC). In the present specification, an "increase in half-life" refers to an increase in any one of these parameters, any two of these parameters, any three of these parameters or all four of these parameters.

The notations "mpk", "mg/kg", or "mg per kg" refer to milligrams per kilogram. All notations are used interchangeably throughout the present disclosure.

The terms "individual", "subject", and "patient", used interchangeably herein, refer to an animal, preferably a mammalian (including a nonprimate and a primate), including, but not limited to, murines, simians, humans, mammalian farm animals (e.g., bovine, porcine, ovine), mammalian sport animals (e.g., equine), and mammalian pets (*e.g.,* canine and feline); preferably the term refers to humans. In a certain embodiment, the subject, is a mammal, is preferably a human and is infected with HIV.

The term "therapeutically effective amount" refers to at least the minimal dose, but less than a toxic dose, of an agent which is necessary to impart a therapeutic benefit to a subject. For example, a therapeutically effective amount of a Combinectin of the invention is an amount which in mammals, preferably humans, results in a significant decline in circulating HIV within the infected individual.

### Overview

The present invention provides novel polypeptides that bind to CD4 and/or gp41. The polypeptides comprising a CD4 binding moiety, a gp41 binding moiety, a HIV fusion peptide inhibitor moiety and combinations thereof. More specifically, the present invention relates to polypeptides comprising a fibronectin-based scaffold domain protein that binds CD4, a fibronectin-based scaffold domain protein that binds the N17 domain of gp41, and a HIV fusion peptide inhibitor or combinations thereof (herein referred to as "Combinectins ").

In order to identify CD4 and gp41 Adnectins, soluble CD4 (extracellular domain) and gp41 (various artificial constructs designed to display a triple-helical segment mimicking a portion of gp41) were presented to large synthetic libraries of Adnectins. Adnectins that survived several rounds of selection were screened for CD4 or gp41 binding, for biophysical properties, and for HIV-1 inhibitory activity. The best anti-CD4 and anti-N17 Adnectin sequences that emerged from the screenings were mutated and subjected to further rounds of selection with increased selective pressure, achieved by lowering the target concentration and/or selecting for anti-CD4 or gp41 Adnectins with fast on-rates and/or slow off-rates. From this optimization process, multiple families of Adnectins, some that targeted CD4, and others targeting gp41, were identified as HIV-1 specific inhibitors with favorable biochemical and biophysical activity.

An optimized gp41-targeting helical peptide was developed starting with sequences related to gp41 HR2 and containing changes for improving potency and breadth across HIV strains as well as increasing the resistance barrier. Peptides were produced at times synthetically and at other times as genetic fusions to inert or active Adnectins. Optimal N- and C-terminal positions were determined in fusions to a member of the gp41 Adnectin family. To identify mutations that would further increase potency, an N-terminally trimmed peptide with modest potency was used, so that improvements would be more easily detected. Small libraries of the inert Adnectin-peptide fusion were made comprising single and multiple point mutations, then the proteins were expressed and screened for biophysical properties and HIV-1 inhibitory activity. The final family of peptides consisted of optimal length peptides with various combinations of the sequences that had the most favorable profiles.

### I. Fibronectin Based Scaffolds - Adnectins

One aspect of the application provides anti-CD4 and anti-N17 Adnectins comprising a fibronectin type III (Fn3) domain in which part or all of one or more of the solvent accessible loops has been randomized or mutated. In some embodiments, one or more residues in one or more of the non-loop beta strands has also been randomized or mutated. In some embodiments, the Fn3 domain is an Fn3 domain derived from the tenth type 3 module of human fibronectin (¹⁰Fn3):
VSDVPRDLEVVAATPTSLLISWDAPAVTVRYYRITYGETGGNSPVQEFTVPGSKS TATISGLKPGVDYTITVYAVTGRGDSPASSKPISINYRT (SEQ ID NO:1) (BC, CD, DE, and FG loops are underlined)

In other embodiments, the non-ligand binding sequences of ¹⁰Fn3, *i.e.,* the "¹⁰Fn3N scaffold", may be altered, provided that the ¹⁰Fn3 retains ligand binding function and/or structural stability. A variety of mutant ¹⁰Fn3 scaffolds have been reported. In one aspect, one or more of Asp 7, Glu 9, and Asp 23 is replaced by another amino acid, such as, for example, a non-negatively charged amino acid residue (e.g., Asn, Lys, etc.). These mutations have been reported to have the effect of promoting greater stability of the mutant ¹⁰Fn3 at neutral pH as compared to the wild-type form (see, *e.g.,* PCT Publication No. WO 02/04523). A variety of additional alterations in the ¹⁰Fn3 scaffold that are either beneficial or neutral have been disclosed. See, for example, Batori et al., Protein Eng., 15(12): 1015-1020 (Dec. 2002); Koide et al., Biochemistry, 40(34): 10326-10333 (Aug. 28, 2001).

Both variant and wild-type ¹⁰Fn3 proteins are characterized by the same structure, namely seven beta-strand domain sequences designated A through G and six loop regions (AB loop, BC loop, CD loop, DE loop, EF loop, and FG loop) which connect the seven beta-strand domain sequences. The beta strands positioned closest to the N- and C-termini may adopt a beta-like conformation in solution. In SEQ ID NO: 1, the AB loop corresponds to residues 14-17, the BC loop corresponds to residues 23-31, the CD loop corresponds to residues 37-47, the DE loop corresponds to residues 51-56, the EF loop corresponds to residues 63-67, and the FG loop corresponds to residues 75-87.

Accordingly, in some embodiments, the anti-CD4 or anti-N17 Adnectin of the disclosure is a ¹⁰Fn3 polypeptide that is at least 40%, 50%, 60%, 65%, 70%, 75%, 80%, 85%, or 90% identical to the human ¹⁰Fn3 domain, shown in SEQ ID NO:1. Much of the variability will generally occur in one or more of the loops. Each of the beta or beta-like strands of a ¹⁰Fn3 polypeptide may consist essentially of an amino acid sequence that is at least 40%, 50%, 60%, 65%, 70%, 75%, 80%, 85%, 90%, 95% or 100% identical to the sequence of a corresponding beta or beta-like strand of SEQ ID NO:1, provided that such variation does not disrupt the stability of the polypeptide in physiological conditions.

In some embodiments, the invention provides one or more Adnectins comprising a tenth fibronectin type III (¹⁰Fn3) domain, wherein the ¹⁰Fn3 domain comprises a loop, AB; a loop, BC; a loop, CD; a loop, DE; a loop EF; and a loop FG; and has at least one loop selected from loop BC, CD, DE, and FG with an altered amino acid sequence relative to the sequence of the corresponding loop of the human ¹⁰Fn3 domain. In some embodiments, the Adnectins of the present invention comprise an ¹⁰Fn3 domain comprising an amino acid sequence at least 40%, 50%, 60%, 65%, 70%, 75%, 80%, 85%, 90%, 95%, 98%, 99% or 100% identical to the non-loop regions of SEQ ID NO:1, wherein at least one loop selected from BC, CD, DE, and FG is altered. In some embodiments, the BC and FG loops are altered, and in some embodiments, the BC, DE, and FG loops are altered, *i.e.,* the ¹⁰Fn3 domains comprise non-naturally occurring loops. In some embodiments, the AB, CD and/or the EF loops are altered. In some embodiments the CD and FG loops are altered. In some embodiments the solvent-accessible amino acids in the strands between loops are altered, with or without alteration of the adjoining loops. By "altered" is meant one or more amino acid sequence alterations relative to a template sequence (corresponding human fibronectin domain) and includes amino acid additions, deletions, substitutions or a combination thereof. Altering an amino acid sequence may be accomplished through intentional, blind, or spontaneous sequence variation, generally of a nucleic acid coding sequence, and may occur by any technique, for example, PCR, error-prone PCR, or chemical DNA synthesis.

In some embodiments, one or more loops selected from BC, CD, DE, and FG may be extended or shortened in length relative to the corresponding human fibronectin loop. In some embodiments, the length of the loop may be extended by 1-25 amino acids. In some embodiments, the length of the loop may be decreased by 1-11 amino acids. To optimize antigen binding, therefore, a loop of ¹⁰Fn3 may be altered in length as well as in sequence to obtain the greatest possible flexibility and affinity in antigen binding.

In some embodiments, the Adnectins comprise a Fn3 domain that comprises an amino acid sequence at least 40%, 50%, 60%, 65%, 70%, 75%, 80%, 85%, 90%, 95%, 98%, 99%, or 100% identical to the non-loop regions of SEQ ID NO:1, wherein at least one loop selected from BC, CD, DE, and FG is altered. In some embodiments, the altered BC loop has up to 1, 2, 3, 4, 5, 6, 7, 8 or 9 amino acid substitutions, up to 1, 2, 3, or 4 amino acid deletions, up to 1, 2, 3, 4, 5, 6, 7, 8, 9, or 10 amino acid insertions, or a combination thereof. In some embodiments, the altered CD loop has up to 1, 2, 3, 4, 5, 6, 7, 8, 9, 10, or 11 amino acid substitutions, up to 1, 2, 3, 4, 5, or 6 amino acid deletions, up to 1, 2, 3, 4, 5, 6, 7, 8, 9, or 10 amino acid insertions, or a combination thereof. In some embodiments, the altered DE loop has up to 1, 2, 3, 4, 5, or 6 amino acid substitutions, up to 1, 2, 3, or 4 amino acid deletions, up to 1, 2, 3, 4, 5, 6, 7, 8, 9, 10, 11, 12, or 13 amino acid insertions, or a combination thereof. In some embodiments, the FG loop has up to 1, 2, 3, 4, 5, 6, 7, 8, 9, 10, 11, 12 or 13 amino acid substitutions, up to 1, 2, 3, 4, 5, 6, 7, 8, 9, 10, or 11 amino acid deletions, up to 1, 2, 3, 4, 5, 6, 7, 8, 9, 10, 11, 12, 13, 14, 15, 16,17,18,19, 20, 21, 22, 23, 24, or 25 amino acid insertions, or a combination thereof.

### Extension Sequences

In certain embodiments, the Adnectin molecules of the present invention may be modified to comprise an N-terminal extension sequence and/or a C-terminal extension. For example, an MG sequence may be placed at the N-terminus of the ¹⁰Fn3 defined by SEQ ID NO: 1. The M will usually be cleaved off, leaving a G at the N-terminus. The Adnectins described herein may also comprise alternative C-terminal tail sequences, referred to herein as truncated C-terminal or C-terminal extension sequences. Further, truncated version may be used as therapeutic molecules in the truncated form, or alternative C-terminal extensions, such as His6 tag, may be added to the truncated version. In certain embodiments, the C-terminal extension sequences (also called "tails"), comprise E and D residues, and may be between 8 and 50, 10 and 30, 10 and 20, 5 and 10, and 2 and 4 amino acids in length. In certain embodiments, the first residue of a C-terminal extension is a proline. In certain other embodiments, the first residue of a C-terminal extension is a glutamic acid.

In some embodiments, the N-terminus may be extended by up to 1, 2, 3, 4, 5, 6, 7, 8, 9, 10, 11, 12, 13, 14,15, or more amino acids, which may be altered in any way, before or after rounds of selection, in order to improve target binding, stability, or both. In other embodiments, the C-terminus may be extended by up to 1, 2, 3, 4, 5, 6, 7, 8, 9, 10, 11, 12, 13, 14,15, or more amino acids, which may be altered in any way, before or after rounds of selection, in order to improve target binding, stability, or both. In still other embodiments, both the N- and C-termini may be extended in this manner.

### Anti-CD4 Adnectin

The amino acid sequence of anti-CD4 Adnectin loop region CD and FG of the invention include but are not limited to those listed in Table 1 below. The CD loops described in Table 1 replace R30 through T49 of ¹⁰Fn3 defined by SEQ ID NO:1. The FG loops described in Table 1 replace D67 through N91 of ¹⁰Fn3 defined by SEQ ID NO:1

Table 1 also lists the IC₅₀ values for each anti-CD4 Adnectin comprising the listed CD/FG loop combinations.

**Table 1**

| CD4 Adnectin - CD and FG loop combinations | | | |
|---|---|---|---|
| CD loop | FG loop | IC₅₀, µM | SEQ ID NO |
| | | 0.0025 | 13, 14 |
| | | 0.0060 | 15, 16 |
| | | 0.0072 | 17, 18 |
| | | 0.0075 | 19, 20 |
| | | 0.0082 | 21, 22 |
| | | 0.0087 | 23, 24 |
| | | 0.0099 | 25, 26 |
| | | 0.0115 | 27, 28 |
| | | 0.0118 | 29, 30 |
| | | 0.0158 | 31, 32 |
| | | 0.0165 | 33, 34 |
| | | 0.0213 | 35, 36 |
| | | 0.0214 | 37, 38 |
| | | 0.0230 | 39, 40 |
| | | 0.0239 | 41, 42 |
| | | 0.0260 | 43, 44 |
| | | 0.0272 | 45, 46 |
| | | 0.0287 | 47, 48 |
| | | 0.0290 | 49, 50 |
| | | 0.0297 | 51, 52 |
| | | 0.0323 | 53, 54 |
| | | 0.0353 | 55, 56 |
| | | 0.0410 | 57, 58 |
| | | 0.0411 | 59, 60 |
| | | 0.0436 | 61, 62 |
| | | 0.0509 | 63, 64 |
| | | 0.0517 | 65, 66 |
| | | 0.0562 | 67, 68 |
| | | 0.0587 | 69, 70 |
| | | 0.0604 | 71, 72 |
| | | 0.0637 | 73, 74 |
| | | 0.0688 | 75, 76 |
| | | 0.0703 | 77, 78 |
| | | 0.0715 | 79, 80 |
| | | 0.0839 | 81, 82 |
| | | 0.0869 | 83, 84 |
| | | 0.0875 | 85, 86 |
| | | 0.1059 | 87, 88 |
| | | 0.1277 | 89, 90 |
| | | 0.1600 | 91, 92 |
| | | 0.4281 | 93, 94 |

In some embodiments, anti-CD4 Adnectin of the invention comprises an amino acid sequence at least 80%, 85%, 90%, 95%, 98%, 99% or 100% identical to the CD/FG loop region combinations of SEQ ID NOs: 13-94.

In some embodiments, anti-CD4 Adnectin of the invention comprises an amino acid sequence at least 80%, 85%, 90%, 95%, 98%, 99% or 100% identical to any one of the CD loop regions of SEQ ID NOs: 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91 and 93.

In some embodiments, anti-CD4 Adnectin of the invention comprises an amino acid sequence at least 80%, 85%, 90%, 95%, 98%, 99% or 100% identical to any one of the FG loop regions of SEQ ID NOs: 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92 and 94.

WebLogo (weblogo.berkeley.edu) was used to identify consensus sequences for anti-CD4 Adnectin. Y32, I34, Y36, Q46 and F48 of the anti-CD4 Adnectin CD loop are conserved amino acids (see FIG. 6). In some embodiments, the anti-CD4 Adnectin comprises one or more of the conserved amino acids Y32, 134, Y36, Q46 and F48.

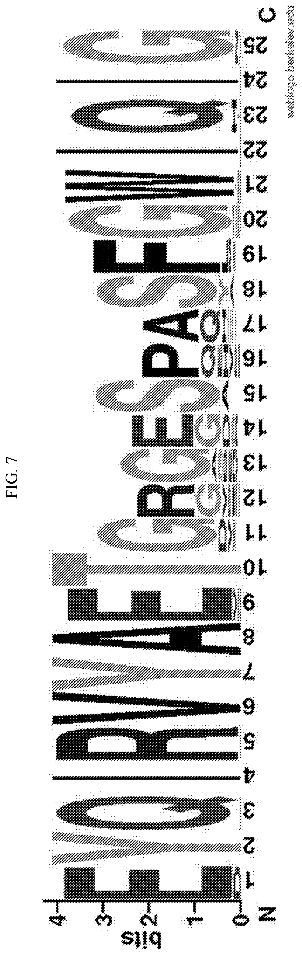

WebLogo identified Y68, I70, V72, A74, T76, I88 and 190 of the anti-CD4 Adnectin FG loop as conserved amino acids (see FIG. 7). In some embodiments, the anti-CD4 Adnectin comprises one or more of the conserved amino acids Y68, I70, V72, A74, T76, I88 and 190.

In some embodiments, the anti-CD4 Adnectin comprises one or more of the conserved amino acids Y32, I34, Y36, Q46, F48, Y68, I70, V72, A74, T76, I88 and 190.

The full length amino acid sequence of anti-CD4 Adnectin of the invention include but are not limited to those listed in Table 2 below. Table 2 also lists the antiviral EC₅₀ values for each anti-CD4 Adnectin.

**Table 2**

| Anti-CD4 Adnectin | | |
|---|---|---|
| SEQ ID NO | Antiviral EC₅₀ (nM) | Anti-CD4 Adnectin Protein Sequence |
| 95 | 48 | |
| 96 | 7.8 | |
| 97 | 11 | |
| 98 | 4.9 | |
| 99 | 8.5 | |
| 100 | 6 | |
| 101 | >400 | |
| 102 | >5500 | |
| 103 | 255 | |
| 104 | 500 | |
| 105 | 300 | |
| 106 | 2400 | |
| 107 | 500 | |
| 108 | 170 | |
| 109 | 320 | |
| 110 | 28.6 | |
| 111 | 20.0 | |
| 112 | 18.4 | |
| 113 | 15.4 | |
| 114 | 12.6 | |

In some embodiments, the anti-CD4 Adnectin of the invention comprises an amino acid sequence at least 70%, 75%, 80%, 85%, 90%, 95%, 98%, 99% or 100% identical to any one of SEQ ID NOs: 95-114.

In some embodiments, the anti-CD4 Adnectin of the invention comprises an amino acid sequence at least 70%, 75%, 80%, 85%, 90%, 95%, 98%, 99% or 100% identical to any one of SEQ ID NOs: 95-114, excluding any N-terminus extended region.

In some embodiments, the anti-CD4 Adnectin of the invention comprises an amino acid sequence at least 70%, 75%, 80%, 85%, 90%, 95%, 98%, 99% or 100% identical to any one of SEQ ID NOs: 95-114, excluding any C-terminus extended region.

In some embodiments, the anti-CD4 Adnectin of the invention comprises an amino acid sequence at least 70%, 75%, 80%, 85%, 90%, 95%, 98%, 99% or 100% identical to any one of SEQ ID NOs: 95-114, excluding both the N-terminus and C-terminus extended regions.

In other embodiments, anti-CD4 Adnectin comprises an amino acid sequence at least 80%, 85%, 90%, 95%, 98%, 99% or 100% identical to the CD loop and FG loop regions of SEQ ID NOs: 95-114.

### Anti-N17 Adnectin

The full length amino acid sequence of anti-N17 Adnectin protein of the invention include but are not limited to those listed in Table 3 below. Table 3 also lists the antiviral EC₅₀ values for each anti-N17 Adnectin.

**Table 3**

| Anti-N17 Adnectin | | |
|---|---|---|
| SEQ ID NO | Antiviral EC₅₀ (nM) | Anti-N17 Adnectin Protein Sequence |
| 115 | 130 | |
| 116 | 50 | |
| 117 | 42 | |
| 118 | 28 | |
| 119 | >1,000 | |
| 120 | 39 | |
| 121 | 57 | |
| 122 | 6 | |
| 123 | 28 | |
| 124 | 6 | |
| 125 | 4342 | |
| 126 | 8 | |
| 127 | 9 | |
| 128 | 18 | |
| 129 | 11 | |
| 130 | 42 | |
| 131 | 5 | |
| 132 | 5 | |
| 133 | 42 | |
| 134 | 5 | |
| 135 | >1,000 | |
| 136 | 5 | |
| 137 | 80 | |
| 138 | 3300 | |
| 139 | 130 | |
| 140 | 950 | |
| 141 | 3 | |
| 142 | 55 | |
| 143 | 20 | |
| 144 | 20 | |
| 145 | 11 | |
| 146 | 11 | |
| 147 | 28 | |
| 148 | 14 | |
| 149 | 3 | |
| 150 | 18 | |
| 151 | 11 | |
| 152 | 20 | |
| 153 | 8 | |
| 154 | 8 | |
| 155 | 9 | |
| 156 | 43 | |
| 157 | 43 | |
| 158 | 85 | |
| 159 | 23 | |
| 160 | 58 | |
| 161 | 13 | |
| 162 | 35 | |
| 163 | 35 | |
| 164 | 35 | |
| 165 | 42 | |
| 166 | 50 | |
| 167 | 42 | |
| 168 | 42 | |
| 169 | 5 | |
| 170 | 5 | |
| 171 | 35 | |
| 172 | 5 | |
| 173 | 5 | |
| 174 | 5 | |
| 175 | 5 | |
| 176 | >1,000 | |
| 177 | 252 | |
| 178 | 631 | |
| 179 | >1,000 | |
| 180 | >1,000 | |
| 181 | 631 | |
| 182 | >1,000 | |
| 183 | 20 | |
| 184 | 20 | |
| 185 | 252 | |
| 186 | 2830 | |
| 187 | 86 | |
| 188 | 86 | |
| 189 | >1,000 | |
| 190 | >1,000 | |
| 191 | >1,000 | |
| 192 | 2830 | |
| 193 | 252 | |
| 194 | >1,000 | |
| 195 | >1,000 | |
| 196 | 86 | |
| 197 | 81 | |
| 198 | 2830 | |
| 199 | >1,000 | |
| 200 | >1,000 | |
| 201 | 20 | |
| 202 | >1,000 | |
| 203 | 86 | |
| 204 | 0 | |
| 205 | 86 | |
| 206 | 237 | |
| 207 | >1,000 | |
| 208 | >1,000 | |
| 209 | 20 | |
| 210 | 143 | |
| 211 | 631 | |
| 212 | 86 | |
| 213 | >1,000 | |
| 214 | >1,000 | |
| 215 | >1,000 | |
| 216 | >1,000 | |
| 217 | >1,000 | |
| 218 | 62 | |
| 219 | >1,000 | |
| 220 | 86 | |
| 221 | >1,000 | |
| 222 | >1,000 | |
| 223 | >1,000 | |
| 224 | >1,000 | |
| 225 | >1,000 | |
| 226 | 631 | |
| 227 | >1,000 | |
| 228 | >1,000 | |
| 229 | >1,000 | |
| 230 | 17 | |
| 231 | 10 | |
| 232 | 47 | |
| 233 | 55 | |
| 234 | 20 | |
| 235 | 29 | |
| 236 | 85 | |
| 237 | 31 | |
| 238 | 11 | |
| 239 | 4 | |
| 240 | 5 | |
| 241 | 8 | |
| 242 | 0 | |
| 243 | 48 | |
| 244 | 58 | |
| 245 | 50 | |
| 246 | >1,000 | |
| 247 | 2 | |
| 248 | >1,000 | |
| 249 | 83 | |
| 250 | 218 | |
| 251 | 186 | |
| 252 | >1,000 | |
| 253 | >1,000 | |
| 254 | >1,000 | |
| 255 | 136 | |
| 256 | 170 | |
| 257 | >1,000 | |
| 258 | 195 | |
| 259 | 135 | |
| 260 | 188 | |
| 261 | >1,000 | |
| 262 | >1,000 | |
| 263 | >1,000 | |
| 264 | 209 | |
| 265 | 63 | |
| 266 | >1,000 | |
| 267 | >1,000 | |
| 268 | 2 | |
| 269 | 184 | |
| 270 | >1,000 | |
| 271 | >1,000 | |
| 272 | >1,000 | |
| 273 | >1,000 | |
| 274 | 115 | |
| 275 | 180 | |
| 276 | 16 | |
| 277 | >1,000 | |
| 278 | >1,000 | |
| 279 | >1,000 | |
| 280 | >1,000 | |
| 281 | >1,000 | |
| 282 | 56 | |
| 283 | >1,000 | |
| 284 | >1,000 | |
| 285 | 397 | |
| 286 | >1,000 | |
| 287 | >1,000 | |
| 288 | >1,000 | |
| 289 | >1,000 | |
| 290 | 138 | |
| 291 | >1,000 | |
| 292 | 52 | |
| 293 | >1,000 | |
| 294 | 101 | |
| 295 | >1,000 | |
| 296 | >1,000 | |
| 297 | 30 | |
| 298 | >1,000 | |
| 299 | >1,000 | |
| 300 | 132 | |
| 301 | >1,000 | |
| 302 | 4 | |
| 303 | 109 | |
| 304 | >1,000 | |
| 305 | 83 | |
| 306 | >1,000 | |
| 307 | 205 | |
| 308 | >1,000 | |
| 309 | >1,000 | |
| 310 | 62 | |
| 311 | 42 | |
| 312 | 37 | |
| 313 | >1,000 | |
| 314 | >1,000 | |
| 315 | >1,000 | |
| 316 | 331 | |
| 317 | >1,000 | |
| 318 | >1,000 | |
| 319 | >1,000 | |
| 320 | >1,000 | |
| 321 | 55 | |
| 322 | 96 | |
| 323 | 126 | |
| 324 | 487 | |
| 325 | >1,000 | |
| 326 | >1,000 | |
| 327 | >1,000 | |
| 328 | 63 | |
| 329 | 102 | |
| 330 | 99 | |
| 331 | 100 | |
| 332 | >1,000 | |
| 333 | >1,000 | |
| 334 | >1,000 | |
| 335 | 112 | |
| 336 | 171 | |
| 337 | >1,000 | |
| 338 | >1,000 | |
| 339 | >1,000 | |
| 340 | >1,000 | |
| 341 | >1,000 | |
| 342 | >1,000 | |
| 343 | 122 | |
| 344 | >1,000 | |
| 345 | >1,000 | |
| 346 | 83 | |
| 347 | >1,000 | |
| 348 | >1,000 | |
| 349 | >1,000 | |
| 350 | 191 | |
| 351 | 215 | |
| 352 | 77 | |
| 353 | 19 | |
| 354 | >1,000 | |
| 355 | >1,000 | |
| 356 | 31 | |
| 357 | 178 | |
| 358 | >1,000 | |
| 359 | >1,000 | |
| 360 | >1,000 | |
| 361 | >1,000 | |
| 362 | 121 | |
| 363 | 130 | |
| 364 | 89 | |
| 365 | >1,000 | |
| 366 | >1,000 | |
| 367 | 300 | |
| 368 | 62 | |
| 369 | 155 | |
| 370 | 335 | |
| 371 | 114 | |

In some embodiments, the anti-N17 Adnectin of the invention comprises an amino acid sequence at least 70%, 75%, 80%, 85%, 90%, 95%, 98%, 99% or 100% identical to anyone of SEQ ID NOs: 115-371.

In some embodiments, the anti-N17 Adnectin of the invention comprises an amino acid sequence at least 70%, 75%, 80%, 85%, 90%, 95%, 98%, 99% or 100% identical to any one of SEQ ID NOs: 115-371, excluding any N-terminus extended region.

In some embodiments, the anti-N17 Adnectin of the invention comprises an amino acid sequence at least 70%, 75%, 80%, 85%, 90%, 95%, 98%, 99% or 100% identical to any one of SEQ ID NOs: 115-371, excluding any C-terminus extended region.

In some embodiments, the anti-N17 Adnectin of the invention comprises an amino acid sequence at least 70%, 75%, 80%, 85%, 90%, 95%, 98%, 99% or 100% identical to any one of SEQ ID NOs: 115-371, excluding both the N-terminus and C-terminus extended regions.

In other embodiments, anti-N17 Adnectin comprises an amino acid sequence at least 80%, 85%, 90%, 95%, 98%, 99% or 100% identical to the BC loop, DE loop and FG loop regions of SEQ ID NOs: 115-371.

Analysis of the sequences above indicate that S52, V53, L54 and S55 of the anti-N17 Adnectin DE loop are conserved amino acids. In some embodiments, the anti-N17 Adnectin comprises one or more of the conserved amino acids S52, V53, L54 and S55.

Additionally, analysis of the sequences above indicate that Y24 of the anti-CD4 Adnectin BC loop is a conserved amino acid. In some embodiments, position 26 of the anti-N17 Adnectin BC loop is valine or leucine.

Analysis of the sequences above indicate that G78 and S85 of the anti-N17 Adnectin FG loop are conserved amino acids. In some embodiments, the anti-N17 Adnectin comprises one or more of the conserved amino acids G78 and S85. In some embodiments, position 79 of the anti-N17 Adnectin FG loop is valine or isoleucine.

In some embodiments, the anti-N17 Adnectin comprises one or more of the conserved amino acids Y24, V26, L26, S52, V53, L54, S55 G78, V79, I79 and S85.

Point mutation analysis of the anti-N17 Adnectin showed advantages of mutating several ¹⁰Fn3 non-loop scaffold positions. Specifically, mutating positions T56 and T58 boosted potency. In some embodiments, the anti-N17 Adnectin comprises mutating T58 to Asn, Glu, or Gln.

### II. HIV Fusion peptide inhibitors

The amino acid sequence of gp41, and its variation among different strains of HIV-1, is well known. The fusogenic domain (often called the fusion peptide, or FP) is believed to be involved in insertion into and disruption of the target cell membrane. The transmembrane domain, containing the transmembrane anchor sequence, is located toward the C-terminal end of the protein. Between the fusogenic domain and transmembrane anchor are two distinct regions, known as heptad repeat (HR) regions, each region having a plurality of heptads. The amino acid sequence comprising the HR1 region and the amino acid sequence comprising the HR2 region are each relatively conserved regions in the HIV-1 envelope protein. A representative sequence of the external domain of gp41 (clade B consensus) is as follows:
512 AVGIGAMFL GFLGAAGSTM GAASVTLTVQ ARQLLSGIVQ QQNNLLRAIE
561 AQQHLLQLTV WGIKQLQARV LAVERYLKDQ QLLGIWGCSG KLICTTAVPW
611 NASWSNKSLD EIWNNMTWME WEREIDNYTG LIYTLIEESQ NQQEKNEQEL
661 LELDKWASLW NWFDITNWLW YIK (SEQ ID NO:2)

The fusion peptide consists of approximately the first 23 amino acids, Ala512-Ser534. The HR1 region has a plurality of contiguous 7 amino acid residue stretches or "heptads" (the 7 amino acids in each heptad designated "a" through "g"), with a predominance of hydrophobic residues at the first ("a") and fourth ("d") positions which interact homotypically to form the core of the 3-helix bundle structure. Neutral polar amino acids such as Glutamine may also occupy these positions. One representative heptad begins with Leu545. A highly conserved portion of HR1 consists of the 17 residues from Leu565 to Leu581, and is called "N17".

The C-terminal portion of gp41 comprises the HR2 region, which is believed to form an alpha helical structure during the fusion process, and bind into the grooves of the HR1 triple helical structure. HR2 also comprises heptads, though they do not interact homotypically but rather interact with amino acids from HR1. One representative heptad begins at Trp628.

### HIV Fusion Peptide Inhibitor

The HIV fusion peptide inhibitor of the invention include but are not limited to the following sequences:

| HIV Fusion Peptide Inhibitor Sequence | SEQ ID NO |
|---|---|
| SEYEARIEALIRAAQEQQEKNEAALRELYKWAL | 372 |
| SEYEARIEALIRAAQEQQEKNEAALRELWKWAS | 373 |
| TIAEYAARIEALIRAAQEQQEKNEAALRELYKWAS | 374 |
| ARIEEYAARIEALIRAAQEQQEKNEAALRELYKWAS | 375 |
| SEYEARIEALIRAAQEQQEKNEAALRELYKWAS | 376 |
| TEYEARIEALIRAAQEQQEKNEAALRELKEWASIWN | 377 |
| SEYEARIEALIRAAQEQQEKNEAALRELDKWTGVWGNYEKV | 378 |
| SRIEALIRAAQEQQEKNEAALRELFKWAS | 379 |
| SRIEALIRAAQEQQQKNEAALRELDKWAS | 380 |
| SRIEALIRAAQEQQEKNEAALRELYKWAS | 381 |
| SRIEALIRAAQEQQEKNEAALRELLKWAS | 382 |
| SRIEALIRAAOEOOEKNEAALRELQKWAS | 383 |
| SRIEALIRAAQEQQEKNEAALRELDKWAS | 384 |
| AIAEYAARIEALIRAAQEQQEKNEAALRELDKWAS | 385 |
| TEYEARIEALIRAAQEQQEKNEAALRELDK | 386 |
| SRIEALIRAAQEQQEKNEAALRELYKWTS | 387 |
| SRIEALIRAAQEQQEKNEAALRELYKWASLWI | 388 |
| SRIEALIRAAQEQQEKNEAALRELYKWASRWN | 389 |
| SRIEALIRAAQEQQEKNEAALRELYKWASSWN | 390 |
| SRIEALIRAAQEQQEKNEAALRELYKWGS | 391 |
| SRIEALIRAAQEQQEKNEAALRELDK | 392 |

In some embodiments, the HIV fusion peptide inhibitor comprises an amino acid sequence at least 70%, 75%, 80%, 85%, 90%, 95%, 98%, 99% or 100% identical to any one of SEQ ID NOs: 372-392.

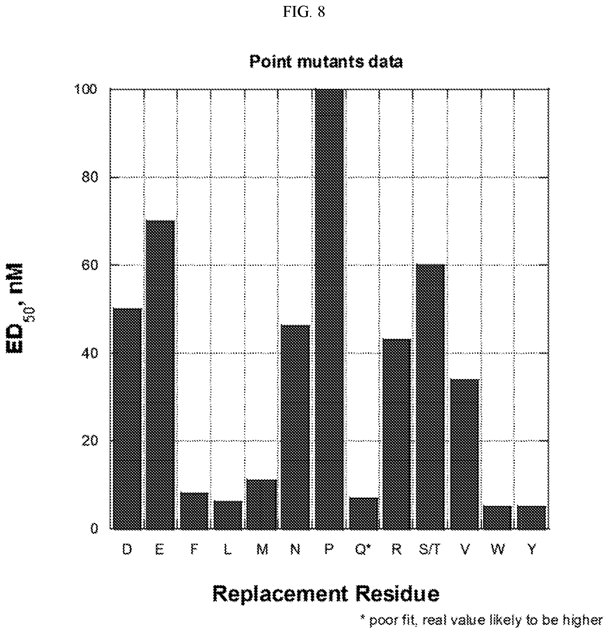

Point mutations in the C-terminal region of the HIV fusion peptide inhibitor were seen to boost potency. In some embodiments, a hydrophobic replacement of the aspartic acid (D) near the C-terminus of the HIV fusion peptide inhibitor provided at least a 10x increase in potency (FIG. 8). In some embodiments, the HIV fusion peptide inhibitor comprises replacing "DK" with "YK", "LK", "FK" or "WK".

Other point mutation studies in the C-terminal region showed how the C-terminal amino acids "WAS" can be mutated with good effects on potency. In some embodiments, the HIV fusion peptide inhibitor comprises replacing C-terminal amino acids "WAS" to "WFS" or "WAL".

### III. Linkers

The various components of the Combinectin of the invention may be covalently or non-covalently linked. In some embodiments, the PK moiety may be directly or indirectly linked to a Combinectin via a polypeptide linker.

Suitable linkers are those which allow the separate domains to fold independently of each other forming a three dimensional structure that permits high affinity binding to a target molecule.

The disclosure provides a number of suitable linkers that meet these requirements, including glycine-serine based linkers, glycine-proline based linkers. The Examples described herein demonstrate that the Combinectin domains joined via polypeptide linkers retain their target binding function. In some embodiments, the linker is a glycine-serine based linker. These linkers comprise glycine and serine residues and may be between 8 and 50, 10 and 30, and 10 and 20 amino acids in length. Examples include linkers having an amino acid sequence (GS)₇ (SEQ ID NO: 393), G(GS)₆ (SEQ ID NO: 394), and G(GS)₇G (SEQ ID NO: 395). Other linkers contain glutamic acid, and include, for example, (GSE)₅ (SEQ ID NO: 396) and GGSEGGSE (SEQ ID NO: 397). Other exemplary glycine-serine linkers include (GS)₄ (SEQ ID NO: 398), (GGGGS)₇ (SEQ ID NO: 399), (GGGGS)₅ (SEQ ID NO: 400), (GGGGS)₄ (SEQ ID NO: 401 (GGGGS)₃G (SEQ ID NO: 402). In some embodiments, the linker is a glycine-proline based linker. These linkers comprise glycine and proline residues and may be between 3 and 30, 10 and 30, and 3 and 20 amino acids in length. Examples include linkers having an amino acid sequence (GP)₃G (SEQ ID NO: 403) and (GP)₅G (SEQ ID NO: 404). In other embodiments, the linker may be a proline-alanine based linker having between 3 and 30, 10 and 30, and 3 and 20 amino acids in length. Examples of proline alanine based linkers include, for example, (PA)₃ (SEQ ID NO: 405), (PA)₆ (SEQ ID NO: 406) and (PA)₉ (SEQ ID NO: 407). In some embodiments, the linker is a glutamic acid-proline based linker. These linkers comprise glutamic acid and proline residues and may be between 3 and 30, 10 and 30, and 3 and 20 amino acids in length. Examples include linkers having an amino acid sequence ESPEPETPEDE (SEQ ID NO: 408) and (ESPEPETPED)₂E (SEQ ID NO: 409). It is contemplated, that the optimal linker length and amino acid composition may be determined by routine experimentation by methods well known in the art.

Linkers or spacers, may be introduced at the N-terminus of the anti-CD4 moiety, the C-terminus of the anti-CD4 moiety, the N-terminus of the anti-gp41 moiety, the C-terminus of the anti-gp41 moiety, the N-terminus of the HIV fusion peptide inhibitor, or combinations thereof. In some embodiments the preferred linkers between the anti-CD4 Adnectin and anti-N17 Adnectin are short glutamine-proline rich linkers. In some embodiments the preferred linker between the anti-N17 Adnectin and HIV fusion peptide inhibitor are flexible glycine-serine rich linkers.

### IV. Anti-N17 Adnectin Linked to a HIV Fusion Peptide Inhibitor Combinectin

The amino acid sequences of anti-N17 Adnectin - HIV fusion peptide inhibitor Combinectin of the disclosure include but are not limited to those listed in Table 4 below. Table 4 also lists the antiviral EC₅₀ values for each anti-N17 Adnectin-HIV fusion peptide inhibitor Combinectin.

**Table 4**

| Anti-N17 Adnectin - HIV Fusion Peptide Inhibitor Combinectin | | |
|---|---|---|
| SEQ ID NO | Antiviral EC₅₀ (nM) | Anti-N17 Adnectin - HIV Fusion Peptide Inhibitor Protein Sequence |
| 410 | 2 | |
| 411 | 4342 | |
| 412 | 2980 | |
| 413 | 6 | |
| 414 | 4 | |
| 415 | >1,000 | |
| 416 | >1,000 | |
| 417 | 428 | |
| 418 | 218 | |
| 419 | >1,000 | |
| 420 | 8 | |
| 421 | 50 | |
| 422 | 3 | |
| 423 | 122 | |
| 424 | 39 | |
| 425 | 523 | |
| 426 | 139 | |
| 427 | 143 | |
| 428 | 50 | |

In some embodiments of the disclosure, the anti-N17 Adnectin - HIV fusion peptide inhibitor Combinectin or fusion proteins thereof comprise an amino acid sequence at least 70%, 75%, 80%, 85%, 90%, 95%, 98%, 99% or 100% identical to the non-linker regions of any one of SEQ ID NOs: 410-428.

In some embodiments of the disclosure, the anti-N17 Adnectin - HIV fusion peptide inhibitor Combinectin or fusion proteins thereof comprise an amino acid sequence at least 70%, 75%, 80%, 85%, 90%, 95%, 98%, 99% or 100% identical to the non-linker regions of any one of SEQ ID NOs: 410-428, excluding any N-terminus extended region.

In other embodiments of the disclosure, anti-N17 Adnectin - HIV fusion peptide inhibitor Combinectin comprises an anti-N17 Adnectin comprising an amino acid sequence at least 80%, 85%, 90%, 95%, 98%, 99% or 100% identical to the BC loop, DE loop and FG loop regions of SEQ ID NOs: 410-428.

In other embodiments of the disclosure, the anti-N17 Adnectin - HIV fusion peptide inhibitor Combinectin comprises a HIV fusion peptide inhibitor comprising an amino acid sequence at least 80%, 85%, 90%, 95%, 98%, 99% or 100% identical to the HIV fusion peptide inhibitor of SEQ ID NOs: 410-428.

### V. Anti-CD4 Adnectin Linked to an Anti-N17 Adnectin Linked to a HIV Fusion Peptide Inhibitor Combinectin

The anti-CD4 Adnectin linked to an anti-N17 Adnectin linked to a HIV fusion peptide inhibitor Combinectin of the invention include but are not limited to the following sequences:
Combinectin 3137 (SEQ ID NO: 3)
Combinectin 3151 (SEQ ID NO: 5)
Combinectin 3191 (SEQ ID NO: 7)
Combinectin 3202 (SEQ ID NO: 9)

In the sequences above, the anti-CD4 Adnectin sequences are underlined. The anti-N17 Adnectin sequences are double underlined. The HIV fusion peptide inhibitor sequences are underlined in bold. The linker sequences are italicized.

In some embodiments, the Combinectin or fusion proteins thereof comprises an amino acid sequence at least 70%, 75%, 80%, 85%, 90%, 95%, 98%, 99% or 100% identical to any one of SEQ ID NOs: 3, 5, 7 and 9.

In some embodiments, the Combinectin or fusion proteins thereof comprises an amino acid sequence at least 70%, 75%, 80%, 85%, 90%, 95%, 98%, 99% or 100% identical to the non- linker regions of any one of SEQ ID NOs: 3, 5, 7 and 9.

In other embodiments, the Combinectin comprises an anti-CD4 Adnectin comprising an amino acid sequence at least 80%, 85%, 90%, 95%, 98%, 99% or 100% identical to the CD loop and FG loop regions of SEQ ID NOs: 3, 5, 7 and 9.

In other embodiments, the Combinectin comprises an anti-N17 Adnectin comprising an amino acid sequence at least 80%, 85%, 90%, 95%, 98%, 99% or 100% identical to the BC loop, DE loop and FG loop regions of SEQ ID NOs: 3, 5, 7 and 9.

In other embodiments, the Combinectin comprises a HIV fusion peptide inhibitor comprising an amino acid sequence at least 80%, 85%, 90%, 95%, 98%, 99% or 100% identical to the HIV fusion peptide inhibitor of SEQ ID NOs: 3, 5, 7 and 9.

### VI. Pharmacokinetic Moieties

In one aspect, the application provides for an anti-CD4 moiety, an anti-gp41 moiety, a HIV fusion peptide inhibitor and combinations thereof further comprising a pharmacokinetic (PK) moiety. Improved pharmacokinetics may be assessed according to the perceived therapeutic need. Often it is desirable to increase bioavailability and/or increase the time between doses, possibly by increasing the time that a protein remains available in the serum after dosing. In some instances, it is desirable to improve the continuity of the serum concentration of the protein over time (e.g., decrease the difference in serum concentration of the protein shortly after administration and shortly before the next administration). The Combinectin of the invention may be attached to a moiety that reduces the clearance rate of the polypeptide in a mammal (e.g., mouse, rat, or human) by at least two-fold, at least 10-fold, at least 20-fold, at least 30-fold, at least 40-fold, at least 50-fold, at least 60-fold, at least 70-fold, at least 80-fold, at least 90-fold, at least 100-fold relative to the unmodified Combinectin. Other measures of improved pharmacokinetics may include serum half-life, which is often divided into an alpha phase and a beta phase. Either or both phases may be improved significantly by addition of an appropriate moiety. For example, the PK moiety may increase the serum half-life of the polypeptide by more than 5%, 10%, 20%, 30%, 40%, 50%, 60%, 70%, 80%, 90%, 100%, 120%, 150%, 200%, 400%, 600%, 800%, 1000% or more relative to the Combinectin alone.

Moieties that slow clearance of a protein from the blood, herein referred to as "PK moieties", include polyoxyalkylene moieties *(e.g.,* polyethylene glycol), sugars (*e*.*g*., sialic acid), and well-tolerated protein moieties (e.g., Fc and fragments and variants thereof, transferrin, or serum albumin). The Combinectin of the invention may also be fused to albumin or a fragment (portion) or variant of albumin as described in U.S. Publication No. 2007/0048282, or may be fused to one or more Adnectins or other moieties that bind serum albumin, Fc, or transferrin.

Other PK moieties that can be used in the invention include those described in Kontermann et al., (Current Opinion in Biotechnology, 22:868-876 (2011)). Such PK moieties include, but are not limited to, human serum albumin fusions, human serum albumin conjugates, human serum albumin binders (e.g., Adnectin PKE, AlbudAb, ABD), XTEN fusions, PAS fusions (*i.e*., recombinant PEG mimetics based on the three amino acids proline, alanine, and serine), carbohydrate conjugates (e.g., hydroxyethyl starch (HES)), glycosylation, polysialic acid conjugates, and fatty acid conjugates.

Accordingly, in some embodiments the invention provides a Combinectin fused to a PK moiety that is a polymeric sugar. In some embodiments, the PK moiety is a polyethylene glycol moiety or an Fc region. In some embodiments the PK moiety is a serum albumin binding protein such as those described in U.S. Publication Nos. 2007/0178082 and 2007/0269422. In some embodiments the PK moiety is human serum albumin. In some embodiments, the PK moiety is transferrin.

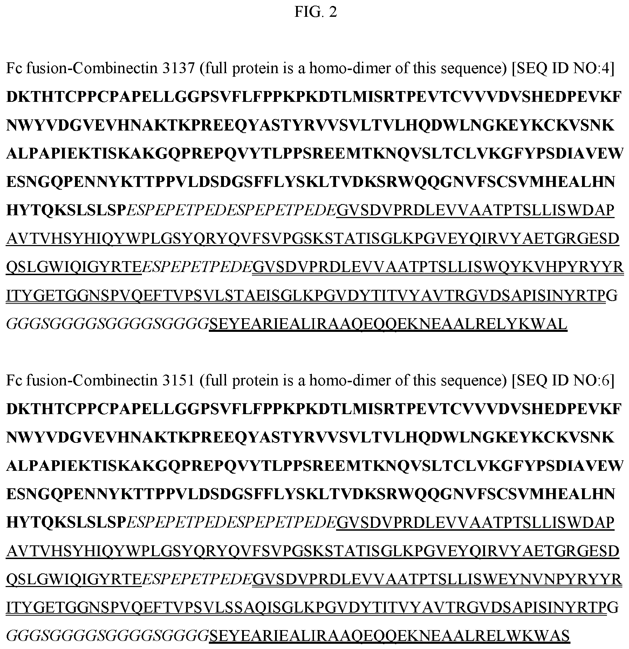

The Fc fusion-Combinectin of the invention include the sequences shown in 3137 (SEQ ID NO: 4) and 3151 (SEQ ID NO: 6) of FIG. 2.

In some embodiments, the Fc fusion-Combinectin or fusion proteins thereof comprises an amino acid sequence at least 70%, 75%, 80%, 85%, 90%, 95%, 98%, 99% or 100% identical to any one of SEQ ID NOs: 4 and 6.

In some embodiments, the Fc fusion-Combinectin or fusion proteins thereof comprises an amino acid sequence at least 70%, 75%, 80%, 85%, 90%, 95%, 98%, 99% or 100% identical to the non- linker regions of any one of SEQ ID NOs: 4 and 6.

In other embodiments, the Fc fusion-Combinectin comprises an anti-CD4 Adnectin comprising an amino acid sequence at least 80%, 85%, 90%, 95%, 98%, 99% or 100% identical to the CD loop and FG loop regions of SEQ ID NOs: 4 and 6.

In other embodiments, the Fc fusion-Combinectin comprises an anti-N17 Adnectin comprising an amino acid sequence at least 80%, 85%, 90%, 95%, 98%, 99% or 100% identical to the BC loop, DE loop and FG loop regions of SEQ ID NOs: 4 and 6.

In other embodiments, the Fc fusion-Combinectin comprises a fusion peptide inhibitor comprising an amino acid sequence at least 80%, 85%, 90%, 95%, 98%, 99% or 100% identical to the HIV fusion peptide inhibitor of SEQ ID NOs: 4 and 6.

The HSA fusion-Combinectin of the invention include the sequences shown in 3191 (SEQ ID NO: 8) and 3202 (SEQ ID NO: 10) of FIG. 2.

In some embodiments, the HSA fusion -Combinectin or fusion proteins thereof comprises an amino acid sequence at least 70%, 75%, 80%, 85%, 90%, 95%, 98%, 99% or 100% identical to any one of SEQ ID NOs: 8 and 10.

In some embodiments, the HSA fusion-Combinectin or fusion proteins thereof comprises an amino acid sequence at least 70%, 75%, 80%, 85%, 90%, 95%, 98%, 99% or 100% identical to the non-linker regions of any one of SEQ ID NOs: 8 and 10.

In other embodiments, the HSA fusion-Combinectin comprises an anti-CD4 Adnectin comprising an amino acid sequence at least 80%, 85%, 90%, 95%, 98%, 99% or 100% identical to the CD loop and FG loop regions of SEQ ID NOs: 8 and 10.

In other embodiments, the HSA fusion-Combinectin comprises an anti-N17 Adnectin comprising an amino acid sequence at least 80%, 85%, 90%, 95%, 98%, 99% or 100% identical to the BC loop, DE loop and FG loop regions of SEQ ID NOs: 8 and 10.

In other embodiments, the HSA fusion-Combinectin comprises a fusion peptide inhibitor comprising an amino acid sequence at least 80%, 85%, 90%, 95%, 98%, 99% or 100% identical to the HIV fusion peptide inhibitor of SEQ ID NOs: 8 and 10.

### Polyethylene Glycol

In some embodiments, the anti-CD4 moiety, anti-gp41 moiety, HIV fusion peptide inhibitor and combinations thereof comprises polyethylene glycol (PEG). PEG is a well-known, water soluble polymer that is commercially available or can be prepared by ring-opening polymerization of ethylene glycol according to methods well known in the art (Sandler et al., Polymer Synthesis, Vol. 3, pp. 138-161, Academic Press, New York). The term "PEG" is used broadly to encompass any polyethylene glycol molecule, without regard to size or to modification at an end of the PEG, and can be represented by the formula: X-O(CH₂CH₂O)ₙ₋₁CH₂CH₂OH, where n is 20 to 2300 and X is H or a terminal modification, e.g., a C₁₋₄ alkyl. PEG can contain further chemical groups which are necessary for binding reactions, which result from the chemical synthesis of the molecule; or which act as a spacer for optimal distance of parts of the molecule. In addition, such a PEG can consist of one or more PEG side-chains which are linked together. PEGs with more than one PEG chain are called multiarmed or branched PEGs. Branched PEGs are described in, for example, European Published Application No. 473084A and U.S. Patent No. 5,932,462.

One or more PEG molecules may be attached at different positions on the protein, and such attachment may be achieved by reaction with amines, thiols or other suitable reactive groups. The amine moiety may be, for example, a primary amine found at the N-terminus of a polypeptide or an amine group present in an amino acid, such as lysine or arginine. In some embodiments, the PEG moiety is attached at a position on the polypeptide selected from the group consisting of: a) the N-terminus; b) between the N-terminus and the most N-terminal beta strand or beta-like strand; c) a loop or strand residue positioned on a face of the polypeptide opposite the target-binding site; d) between the C-terminus and the most C-terminal beta strand or beta-like strand; e) within a linker sequence connecting two binding domains, and f) at the C-terminus.

PEGylation may be achieved by site-directed PEGylation, wherein a suitable reactive group is introduced into the protein to create a site where PEGylation preferentially occurs. In some embodiments, the protein is modified to introduce a cysteine residue at a desired position, permitting site-directed PEGylation on the cysteine. Mutations may be introduced into a protein coding sequence to generate cysteine residues. This might be achieved, for example, by mutating one or more amino acid residues to cysteine. Preferred amino acids for mutating to a cysteine residue include serine, threonine, alanine and other hydrophilic residues. Preferably, the residue to be mutated to cysteine is a surface-exposed residue. Algorithms are well-known in the art for predicting surface accessibility of residues based on primary sequence or a protein. Alternatively, surface residues may be predicted by comparing the amino acid sequences of binding polypeptides, given that the crystal structure of the framework, based on which binding polypeptides are designed and evolved, has been solved (see Himanen et al., Nature, 414:933-938 (2001)) and thus the surface-exposed residues identified. PEGylation of cysteine residues may be carried out using, for example, PEG-maleimide, PEG-vinylsulfone, PEG-iodoacetamide, or PEG-orthopyridyl disulfide.

The PEG is typically activated with a suitable activating group appropriate for coupling to a desired site on the polypeptide. PEGylation methods are well-known in the art and further described in Zalipsky, S. et al., "Use of Functionalized Poly(Ethylene Glycols) for Modification of Polypeptides" in Harris, J.M., Polyethylene Glycol Chemistry: Biotechnical and Biomedical Applications, Plenus Press, New York (1992), and in Zalipsky, Advanced Drug Reviews, 16:157-182 (1995).

PEG may vary widely in molecular weight and may be branched or linear. Typically, the weight-average molecular weight of PEG is from about 100 Daltons to about 150,000 Daltons. Exemplary weight-average molecular weights for PEG include about 20,000 Daltons, about 40,000 Daltons, about 60,000 Daltons and about 80,000 Daltons. In certain embodiments, the molecular weight of PEG is 40,000 Daltons. Branched versions of PEG having a total molecular weight of any of the foregoing can also be used. In some embodiments, the PEG has two branches. In other embodiments, the PEG has four branches. In another embodiment, the PEG is a bis-PEG (NOF Corporation, DE-200MA), to which two Adnectins are conjugated.

Conventional separation and purification techniques known in the art can be used to purify PEGylated Adnectin, such as size exclusion (e.g., gel filtration) and ion exchange chromatography. Products may also be separated using SDS-PAGE. Products that may be separated include mono-, di-, tri-, poly- and un-PEGylated Adnectins, as well as free PEG. The percentage of mono-PEG conjugates can be controlled by pooling broader fractions around the elution peak to increase the percentage of mono-PEG in the composition. About 90% mono-PEG conjugates represent a good balance of yield and activity.

In some embodiments, the PEGylated anti-CD4 moiety, anti-gp41 moiety, HIV fusion peptide inhibitor and combinations thereof will preferably retain at least about 25%, 50%, 60%, 70%, 80%, 85%, 90%, 95% or 100% of the biological activity associated with the unmodified anti-CD4 moiety, anti-gp41 moiety, Adnectin or Combinectin. In some embodiments, biological activity refers to its ability to bind to CD4 or gp41, as assessed by *K*_{d}*, k*ₒₙ*,* or *k*_{off}*.* In some embodiments, the PEGylated anti-CD4 moiety, anti-gp41 moiety, Adnectin or Combinectin thereof shows an increase in binding to CD4 or gp41 relative to unPEGylated anti-CD4 moiety, anti-gp41 moiety, Adnectin or Combinectin.

### Immunoglobulin Fc Domain (and Fragments)

In some embodiments, the peptide of the invention is fused to an immunoglobulin Fc domain, or a fragment or variant thereof. As used herein, a "functional Fc region" is an Fc domain or fragment thereof which retains the ability to bind FcRn. In some embodiments, a functional Fc region binds to FcRn, but does not possess effector functions. The ability of the Fc region or fragment thereof to bind to FcRn can be determined by standard binding assays known in the art. In other embodiments, the Fc region or fragment thereof binds to FcRn and possesses at least one "effector function" of a native Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors *(e.g.,* B cell receptor; BCR), etc. Such effector functions generally require the Fc region to be combined with a binding domain (e.g., an anti-CD4 or ant-N-17 Adnectin) and can be assessed using various assays known in the art for evaluating such antibody effector functions.

A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification. Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95% sequence identity therewith.

In an exemplary embodiment, the Fc domain is derived from an IgG1 subclass, however, other subclasses (e.g., IgG2, IgG3, and IgG4) may also be used. Shown below is the sequence of a human IgG1 immunoglobulin Fc domain:

The core hinge sequence is underlined, and the CH2 and CH3 regions are in regular text. It should be understood that the C-terminal glycine and lysine are optional in the Combinectin of the invention.

The fusion may be formed by attaching an Adnectin of the invention to either end of the Fc molecule, *i.e.,* Fc-Adnectin or Adnectin-Fc arrangements. In certain embodiments, the Fc and Adnectin are fused via a linker.

In some embodiments, the Fc region used in the Adnectin fusion comprises the hinge region of an Fc molecule. As used herein, the "hinge" region comprises the core hinge residues spanning positions 1-16 of SEQ ID NO: 11 (DKTHTCPPCPAPELLG; SEQ ID NO: 12) of the IgG1 Fc region. In certain embodiments, the Adnectin-Fc fusion adopts a multimeric structure (e.g., dimer) owing, in part, to the cysteine residues at positions 6 and 9 of SEQ ID NO: 11 within the hinge region.

In certain embodiments, an Adnectin-Fc fusion may have the following configurations: 1) Adnectin-hinge-Fc or 2) hinge-Fc-Adnectin. Therefore, any Adnectin of the present invention can be fused to an Fc region comprising a hinge sequence according to these configurations. In some embodiments, a linker may be used to join the Adnectin to the hinge-Fc moiety, for example, an exemplary fusion protein may have the configuration Adnectin-linker-hinge-Fc or hinge-Fc-linker-Adnectin. Additionally, depending on the system in which the fusion polypeptide is produced, a leader sequence may be placed at the N-terminus of the fusion polypeptide. For example, if the fusion is produced in a mammalian system, a leader sequence may be added to the N-terminus of the fusion molecule. If the fusion is produced *in E. coli,* the fusion sequence will be preceded by a methionine.

### VII. Nucleic Acid-Protein Fusion Technology

In one aspect, the invention provides an Adnectin comprising fibronectin type III domains that binds CD4 or the n17 domain of gp41. One way to rapidly make and test Fn3 domains with specific binding properties is the nucleic acid-protein fusion technology. This disclosure utilizes the *in vitro* expression and tagging technology, termed 'PROfusion' which exploits nucleic acid-protein fusions (RNA- and DNA-protein fusions) to identify novel polypeptides and amino acid motifs that are important for binding to proteins. Nucleic acid-protein fusion technology is a technology that covalently couples a protein to its encoding genetic information. For a detailed description of the RNA-protein fusion technology and fibronectin-based scaffold protein library screening methods see Szostak et al., U.S. Patent Nos. 6,258,558, 6,261,804, 6,214,553, 6,281,344, 6,207,446, 6,518,018 and 6,818,418; Roberts et al., Proc. Natl. Acad. Sci., 94:12297-12302 (1997); and Kurz et al., Molecules, 5:1259-1264 (2000).

### VIII. Vectors and Polynucleotides

Nucleic acids encoding any of the various proteins or polypeptides disclosed herein may be synthesized chemically. Codon usage may be selected so as to improve expression in a cell. Such codon usage will depend on the cell type selected. Specialized codon usage patterns have been developed for *E. coli* and other bacteria, as well as mammalian cells, plant cells, yeast cells and insect cells. See, for example, Mayfield et al., Proc. Natl. Acad. Sci. USA, 100(2):438-442 (Jan. 21, 2003); Sinclair et al., Protein Expr. Purif., 26(I):96-105 (Oct. 2002); Connell, N.D., Curr. Opin. Biotechnol., 12(5):446-449 (Oct. 2001); Makrides et al., Microbiol. Rev., 60(3):512-538 (Sep. 1996); and Sharp et al., Yeast, 7(7):657-678 (Oct. 1991).

General techniques for nucleic acid manipulation are described in, for example, Sambrook et al., Molecular Cloning: A Laboratory Manual, Second Edition, Vols. 1-3, Cold Spring Harbor Laboratory Press (1989), or Ausubel, F. et al., Current Protocols in Molecular Biology, Green Publishing and Wiley-Interscience, New York (1987) and periodic updates. Generally, the DNA encoding the polypeptide is operably linked to suitable transcriptional or translational regulatory elements derived from mammalian, viral, or insect genes. Such regulatory elements include a transcriptional promoter, an optional operator sequence to control transcription, a sequence encoding suitable mRNA ribosomal binding site, and sequences that control the termination of transcription and translation. The ability to replicate in a host, usually conferred by an origin of replication, and a selection gene to facilitate recognition of transformants is additionally incorporated.

The proteins described herein may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (*i*.*e*., cleaved by a signal peptidase) by the host cell.

For prokaryotic host cells that do not recognize and process a native signal sequence, the signal sequence is substituted by a prokaryotic signal sequence selected, for example, from the group of the alkaline phosphatase, penicillinase, 1 pp, or heat-stable enterotoxin II leaders.

For yeast secretion the native signal sequence may be substituted by, *e.g.,* a yeast invertase leader, a factor leader (including Saccharomyces and Kluyveromyces alphafactor leaders), or acid phosphatase leader, the C. albicans glucoamylase leader, or the signal sequence described in U.S. Patent No. 5,631,144. In mammalian cell expression, mammalian signal sequences as well as viral secretory leaders, for example, the herpes simplex gD signal, are available. The DNA for such precursor regions may be ligated in reading frame to DNA encoding the protein.

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2 micron plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

Expression and cloning vectors may contain a selection gene, also termed a selectable marker. Typical selection genes encode proteins that (a) confer resistance to antibiotics or other toxins, e.g., ampicillin, neomycin, methotrexate, or tracycline, (b) complement auxotrophic deficiencies, or (c) supply critical nutrients not available from complex media, *e.g.,* the gene encoding D-alanine racemase for Bacilli.

Expression and cloning vectors usually contain a promoter that is recognized by the host organism and is operably linked to the nucleic acid encoding the protein of the invention, e.g., a fibronectin-based scaffold protein. Promoters suitable for use with prokaryotic hosts include the phoA promoter, beta-lactamase and lactose promoter systems, alkaline phosphatase, a tryptophan (trp) promoter system, and hybrid promoters such as the tan promoter. However, other known bacterial promoters are suitable. Promoters for use in bacterial systems also will contain a Shine-Dalgarno (S.D.) sequence operably linked to the DNA encoding the protein of the invention. Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Transcription from vectors in mammalian host cells can be controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, e.g., the actin promoter or an immunoglobulin promoter, from heat-shock promoters, provided such promoters are compatible with the host cell systems.

Transcription of a DNA encoding protein of the invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature, 297:17-18 (1982) on enhancing elements for activation of eukaryotic promoters. The enhancer may be spliced into the vector at a position 5' or 3' to the peptide-encoding sequence, but is preferably located at a site 5' from the promoter.

Expression vectors used in eukaryotic host cells (*e*.*g*., yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of mRNA encoding the protein of the invention. One useful transcription termination component is the bovine growth hormone polyadenylation region. See WO 94/11026 and the expression vector disclosed therein.

The recombinant DNA can also include any type of protein tag sequence that may be useful for purifying the protein. Examples of protein tags include, but are not limited to, a histidine tag, a FLAG tag, a myc tag, an HA tag, or a GST tag. Appropriate cloning and expression vectors for use with bacterial, fungal, yeast, and mammalian cellular hosts can be found in Cloning Vectors: A Laboratory Manual, Elsevier, New York (1985).

The expression construct is introduced into the host cell using a method appropriate to the host cell, as will be apparent to one of skill in the art. A variety of methods for introducing nucleic acids into host cells are known in the art, including, but not limited to, electroporation; transfection employing calcium chloride, rubidium chloride, calcium phosphate, DEAE-dextran, or other substances; microprojectile bombardment; lipofection; and infection (where the vector is an infectious agent).

Suitable host cells include prokaryotes, yeast, mammalian cells, or bacterial cells. Suitable bacteria include gram negative or gram positive organisms, for example, *E. coli* or Bacillus spp. Yeast, preferably from the Saccharomyces species, such as *S. cerevisiae,* may also be used for production of polypeptides. Various mammalian or insect cell culture systems can also be employed to express recombinant proteins. Baculovirus systems for production of heterologous proteins in insect cells are reviewed by Luckow et al. (Bio/Technology, 6:47 (1988)). Examples of suitable mammalian host cell lines include endothelial cells, COS-7 monkey kidney cells, CV-1, L cells, C127, 3T3, Chinese hamster ovary (CHO), human embryonic kidney cells, HeLa, 293, 293T, and BHK cell lines. Purified polypeptides are prepared by culturing suitable host/vector systems to express the recombinant proteins. For many applications, the small size of many of the polypeptides disclosed herein would make expression in *E. coli* as the preferred method for expression. The protein is then purified from culture media or cell extracts.

### IX. Protein Production

The present invention is also directed to cell lines that express a Combinectin or fusion protein thereof. Creation and isolation of cell lines producing a Combinectin can be accomplished using standard techniques known in the art, such as those described herein.

Host cells are transformed with the herein-described expression or cloning vectors for protein production and cultured in conventional nutrient media modified as appropriate for inducing promoters, selecting transformants, or amplifying the genes encoding the desired sequences. In the examples shown here, the host cells used for high-throughput protein production (HTPP) and mid-scale production were those from the HMS174-bacterial strain.

The host cells used to produce the proteins of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma)) are suitable for culturing the host cells. In addition, many of the media described in Ham et al., Meth. Enzymol., 58:44 (1979), Barites et al., Anal. Biochem., 102:255 (1980), U.S. Patent Nos. 4,767,704, 4,657,866, 4,927,762, 4,560,655, 5,122,469, 6,048,728, 5,672,502, or U.S. Patent No. RE 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as Gentamycin drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

Proteins disclosed herein can also be produced using cell-free translation systems. For such purposes the nucleic acids encoding the polypeptide must be modified to allow *in vitro* transcription to produce mRNA and to allow cell-free translation of the mRNA in the particular cell-free system being utilized (eukaryotic such as a mammalian or yeast cell-free translation system or prokaryotic such as a bacterial cell-free translation system.

Proteins of the invention can also be produced by chemical synthesis (e.g., by the methods described in Solid Phase Peptide Synthesis, Second Edition, The Pierce Chemical Co., Rockford, III. (1984)). Modifications to the protein can also be produced by chemical synthesis.

The proteins of the present invention can be purified by isolation/purification methods for proteins generally known in the field of protein chemistry. Non-limiting examples include extraction, recrystallization, salting out *(e.g.,* with ammonium sulfate or sodium sulfate), centrifugation, dialysis, ultrafiltration, adsorption chromatography, ion exchange chromatography, hydrophobic chromatography, normal phase chromatography, reversed-phase chromatography, get filtration, gel permeation chromatography, affinity chromatography, electrophoresis, countercurrent distribution or any combinations of these. After purification, polypeptides may be exchanged into different buffers and/or concentrated by any of a variety of methods known to the art, including, but not limited to, filtration and dialysis.

The purified polypeptide is preferably at least 85% pure, or preferably at least 95% pure, and most preferably at least 98% pure. Regardless of the exact numerical value of the purity, the polypeptide is sufficiently pure for use as a pharmaceutical product.

### X. Biophysical and Biochemical Characterization

Binding of the protein of the invention to a target molecule *(e.g.,* CD4 or gp41) may be assessed in terms of equilibrium constants (e.g., dissociation, *K*_{d}) and in terms of kinetic constants (e.g., on-rate constant, *k*ₒₙ and off-rate constant, *k*_{off}). The protein of the invention will generally bind to a target molecule with a *K*_{d} of less than 500 nM, 100 nM, 10 nM, 1 nM, 500 pM, 200 pM, or 100 pM, although higher *K*_{d} values may be tolerated where the *k*_{off} is sufficiently low or the *k*ₒₙ*,* is sufficiently high.

### In Vitro Assays for Binding Affinity

Proteins that bind CD4 or gp41 can be identified using various *in vitro* assays. Preferably, the assays are high-throughput assays that allow for screening multiple candidates simultaneously.

In some embodiments, biomolecular interactions can be monitored in real time with the BIACORE® system, which uses SPR to detect changes in the resonance angle of light at the surface of a thin gold film on a glass support due to changes in the refractive index of the surface up to 300 nm away. BIACORE® analysis generates association rate constants, dissociation rate constants, equilibrium dissociation constants, and affinity constants. Binding affinity is obtained by assessing the association and dissociation rate constants using a BIACORE® surface plasmon resonance system (Biacore, Inc.). A biosensor chip is activated for covalent coupling of the target. The target is then diluted and injected over the chip to obtain a signal in response units of immobilized material. Since the signal in resonance units (RU) is proportional to the mass of immobilized material, this represents a range of immobilized target densities on the matrix. Association and dissociation data are fit simultaneously in a global analysis to solve the net rate expression for a 1:1 bimolecular interaction, yielding best fit values for *k*ₒₙ*, k*_{off} and Rₘₐₓ (maximal response at saturation). Equilibrium dissociation constants for binding, *K*_{d}'s are calculated from SPR measurements as *k*_{off}/*k*ₒₙ.

In some embodiments, the Combinectin of the invention exhibit a *K*_{d} of 100nM or less. Preferably, the *K*_{d} is 10nM or less. More preferably, the *K*_{d} is 1nM or less.

In some embodiments, the Combinectin of the invention exhibit an IC₅₀ of 5 nM or less, 4 nM or less, 3 nM or less, 2.5 nM or less, 2 nM or less, 1.5 nM or less, 1 nM or less, 0.5 nM or less, 0.2 nM or less, or 0.1 nM or less. Preferably, the IC₅₀ is 1.5 nM or less. More preferably, the IC₅₀ is 0.5 nM or less.

It should be understood that the assays described herein above are exemplary, and that any method known in the art for determining the binding affinity between proteins (*e.g*., fluorescence based-transfer (FRET), enzyme-linked immunosorbent assay, and competitive binding assays (e.g., radioimmunoassays)) can be used to assess the binding affinities of the Combinectin of the invention.

In the present invention, ELISA assays were utilized for identifying Adnectins that bind to CD4 or gp41, with affinities determined by BIACORE® SPR. FACS assays were also used to determine the EC₅₀ of binding of the CD4 Adnectin (alone and as part of the full Combinectin) to CD4 as presented naturally on T-cell surfaces. Peptide affinities were measured by BIACORE® SPR.

As described in Table 5 below, the range of binding affinities (by SPR) for CD4 Adnectins to CD4 was 0.3 nM to 140 nM; the range for N17 Adnectins binding to artificial gp41-based targets was 0.5 nM to 40 nM; the range for peptide binding was 0.2 nM to 70 nM. SPR-based affinities for the Combinectin of the invention and for the individual components thereof are shown in Table 5 below.

**Table 5**

| Binding Affinities for Combinectin and Individual Components | | | | | |
|---|---|---|---|---|---|
| Protein | ID | Target | *Kₒₙ* (1/Ms) | *K_{off}* (1/s) | *K_{d}* (nM) |
| CD4 Adnectin | ADX_6940_B01 | CD4 | 1.9E+05 | 7.6E-04 | 4 |
| N17 Adnectin | ADX_6200_A08 | gp41 (IZN24) | 7.0E+06 | 3.3E-03 | 0.5 |
| peptide | 203613-24 | gp41 (PRD-828) | 2.3E+06 | 2.5E-04 | 0.1 |
| HSA-Combinectin | BMT-180280 | CD4 | 7.6E+03 | 8.3E-04 | 109 |
| HSA-Combinectin | BMT-180280 | gp41 (IZN24) | 8.1E+05 | 1.7E-03 | 2 |
| HSA-Combinectin | BMT-180280 | gp41 (PRD-828) | 9.6E+05 | 1.8E-04 | 0.2 |

### In Vitro Assays for Inhibition Activity

Various art-recognized *in vitro* systems exist that allow for examination of the potency of the Combinectin (or of individual inhibitors or combinations thereof) against HIV-1 infection. These include systems that allow for complete replication of laboratory-derived virus or clinical isolates of various strains in cultured cells or peripheral blood monocyte cultures. In addition, systems that recapitulate the early cell entry stages of infection, without using viable virus, could be used to analyze the effectiveness of the Combinectin, individual inhibitors or combinations thereof. These include, but are not limited to, "pseudotyped" viruses that contain deletions that make them unable to produce infectious virions or cells that express only the HIV gpl60 gene that can be used to monitor the HIV-1 specific fusion reaction to target cells.

### In Vivo Models

One skilled in the art would know of various art-recognized animal models that allow for replication and in some cases recapitulate the symptoms of HIV infection. These models can be used to test the efficacy of the Combinectin, individual inhibitors or combinations thereof of the invention.

### XI. Therapeutic Applications

In one aspect, the present invention provides Combinectins useful for the treatment of HIV. Accordingly, in certain embodiments the disclosure provides methods for attenuating or inhibiting HIV fusion in a subject comprising administering an effective amount of the Combinectin of the invention to a subject. In some embodiments, the subject is a human. In some embodiments, the Combinectin of the invention is pharmaceutically acceptable to a mammal, in particular a human. A "pharmaceutically acceptable" polypeptide refers to a polypeptide that is administered to an animal without significant adverse medical consequences.

In some embodiments of the disclosure, the Combinectin of the present invention will be administered to a subject in combination (concurrently or separately) with an agent known in the art to be useful for the particular disorder or disease being treated.

In some embodiments, the target patient population for Combinectin therapy is one that is not amenable to standard therapy for the disease being treated due to, *e.g.,* age, pre-existing conditions, genetic makeup, and/or co-morbidities. The Combinectin of the invention can serve as an alternative to existing therapies that are associated with substantial side effects or safety concerns.

In some embodiments, the target patient population for Combinectin therapy is comprised of uninfected individuals at high risk of infection, due to lifestyle or other aggravating factors. The Combinectin is used to protect these individuals from getting infected by HIV (pre-exposure prophylaxis).

### XII. Pharmaceutical Compositions

The present invention further provides pharmaceutical compositions comprising a Combinectin or fusion proteins thereof described herein, wherein the composition is essentially endotoxin free, or at least contain no more than acceptable levels of endotoxins as determined by the appropriate regulatory agency (*e*.*g*., FDA).

Compositions of the present invention can be in the form of a pill, tablet, capsule, liquid, or sustained release tablet for oral administration; a liquid for intravenous, subcutaneous or parenteral administration; or a gel, lotion, ointment, cream, or a polymer or other sustained release vehicle for local administration, or an atomizable suspension suitable for inhaled or intranasal administration.

Methods well known in the art for making compositions are found, for example, in Gennaro, A.R., ed., Remington: The Science and Practice of Pharmacy, 20th Edition, Lippincott Williams & Wilkins, Philadelphia, PA (2000). Compositions for parenteral administration may, for example, contain excipients, sterile water, saline, polyalkylene glycols such as polyethylene glycol, oils of vegetable origin, or hydrogenated naphthalenes. Biocompatible, biodegradable lactide polymer, lactide/glycolide copolymer, or polyoxyethylene-polyoxypropylene copolymers may be used to control the release of the compounds. Nanoparticulate compositions (e.g., biodegradable nanoparticles, solid lipid nanoparticles, liposomes) may be used to control the biodistribution of the compounds. Other potentially useful parenteral delivery systems include ethylene-vinyl acetate copolymer particles, osmotic pumps, implantable infusion systems, and liposomes. The concentration of the compound in the composition varies depending upon a number of factors, including the dosage of the drug to be administered, and the route of administration.

Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (e.g., Zn-protein complexes); and/or non-ionic surfactants such as Tween, PLURONIC® or polyethylene glycol (PEG).

The active ingredients may also be entrapped in a microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Osol, A., ed., Remington's Pharmaceutical Sciences, 16th Edition (1980).

Sustained-release preparations may be prepared. Suitable examples of sustained-release preparations include semipermeable matrices of solid hydrophobic polymers containing the proteins of the invention, which matrices are in the form of shaped articles, *e.g.,* films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and y ethyl-L-glutamate, non-degradable ethylene-vinyl acetate, degradable lactic acid-glycolic acid copolymers such as the LUPRON DEPOT® (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated proteins of the invention may remain in the body for a long time, they may denature or aggregate as a result of exposure to moisture at 37 °C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Compositions of the present invention for oral use include tablets containing the active ingredient(s) in a mixture with non-toxic pharmaceutically acceptable excipients. These excipients may be, for example, inert diluents or fillers (e.g., sucrose and sorbitol), lubricating agents, glidants, and anti-adhesives (e.g., magnesium stearate, zinc stearate, stearic acid, silicas, hydrogenated vegetable oils, or talc). Compositions for oral use may also be provided as chewable tablets, or as hard gelatin capsules wherein the active ingredient is mixed with an inert solid diluent, or as soft gelatin capsules wherein the active ingredient is mixed with water or an oil medium.

The pharmaceutical composition to be used for *in vivo* administration typically must be sterile. This may be accomplished by filtration through sterile filtration membranes. Where the composition is lyophilized, sterilization using this method may be conducted either prior to or following lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (*e*.*g*., lyophilized) requiring reconstitution prior to administration.

The compositions herein may also contain more than one active compound as necessary for the particular indication being treated, preferably those with complementary activities that do not adversely affect each other. Such molecules are suitably present in combination in amounts that are effective for the purpose intended.

### XIII. Administration

A pharmaceutical composition comprising a Combinectin or fusion protein thereof of the present invention can be administered to a subject with HIV using standard administration techniques including oral, parenteral, pulmonary, transdermal, intramuscular, intranasal, buccal, sublingual, or suppository administration. Preferably, administration of the Combinectins of the invention is parenteral. The term parenteral as used herein includes intravenous, intramuscular, subcutaneous, rectal, vaginal, or intraperitoneal administration. Peripheral systemic delivery by intravenous or intraperitoneal or subcutaneous injection is preferred.

A therapeutically effective dose refers to a dose that produces the therapeutic effects for which it is administered. An effective amount of a pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the binding agent molecule is being used, the route of administration, and the size (body weight, body surface or organ size) and condition (the age and general health) of the patient.

For example, the therapeutically effective dose can be estimated initially either in cell culture assays or in animal models such as mice, rats, rabbits, dogs, pigs, or monkeys. An animal model may also be used to determine the appropriate concentration range and route of administration. Such information can then be used to determine useful doses and routes for administration in humans.

The exact dosage will be determined in light of factors related to the subject requiring treatment, and may be ascertained using standard techniques. Dosage and administration are adjusted to provide sufficient levels of the active compound or to maintain the desired effect. Factors that may be taken into account include the severity of the disease state, the general health of the subject, the age, weight, and gender of the subject, time and frequency of administration, drug combination(s), reaction sensitivities, and response to therapy. In general, the Combinectin of the present invention are administered at about 0.01 mg/kg to about 50 mg/kg per day, preferably about 0.01 mg/kg to about 30 mg/kg per day, most preferably about 0.01 mg/kg to about 20 mg/kg per day. In some embodiments, the Combinectin of the present invention are administered at weekly dosages of about 0.01 mg/kg to about 10 mg/kg, more preferably about 0.01 to about 5 mg/kg, most preferably about 0.01 to about 1 mg/kg. Alternatively, the Combinectin of the invention are administered at about 15 to about 100mg/week, from about 20 to about 80mg/week, from about 20 to about 60mg/week, or about 20 to about 25 mg/week.

The frequency of dosing will depend upon the pharmacokinetic parameters of the binding agent molecule in the formulation used. Typically, a composition is administered until a dosage is reached that achieves the desired effect. The composition may therefore be administered as a single dose or as multiple doses (at the same or different concentrations/dosages) over time, or as a continuous infusion. Further refinement of the appropriate dosage is routinely made. Appropriate dosages may be ascertained through use of appropriate dose-response data. For example, the Combinectin may be given daily *(e.g.,* once, twice, three times, or four times daily) or less frequently *(e.g.,* once every other day, once or twice weekly, or monthly). In addition, as is known in the art, adjustments for age as well as the body weight, general health, sex, diet, time of administration, drug interaction, and the severity of the disease may be necessary, and will be ascertainable with routine experimentation by those skilled in the art. The Combinectin is suitably administered to the patient at one time or over a series of treatments.

Administration of a Combinectin or a fusion thereof, and one or more additional therapeutic agents, whether co-administered or administered sequentially, may occur as described above for therapeutic applications. Suitable pharmaceutically acceptable carriers, diluents, and excipients for co-administration will be understood by the skilled artisan to depend on the identity of the particular therapeutic agent being administered.

### XIV. Kits and Articles of Manufacture

The Combinectin of the invention can be provided in a kit, a packaged combination of reagents in predetermined amounts with instructions for use in the therapeutic or diagnostic methods of the invention.

For example, in one embodiment of the invention, an article of manufacture containing materials useful for the treatment or prevention of the disorders or conditions described above is provided. The article of manufacture comprises a container and a label. Suitable containers include, for example, bottles, vials, syringes, and test tubes. The containers may be formed from a variety of materials such as glass or plastic. The container holds a composition of the invention which is effective for treating HIV and may have a sterile access port (for example, the container may be an intravenous solution bag or a vial having a stopper pierceable by a hypodermic injection needle). The active agent in the composition is a Combinectin of the invention. The label on, or associated with, the container indicates that the composition is used for treating HIV. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

The invention is now described by reference to the following examples, which are illustrative only, and are not intended to limit the present invention.

### EXAMPLES

### Example 1 - Combinectin Production/Purification

### HIV Combinectin Tandem - Bacterial

Transformed DNA into BL21(DE3) bacterial cells.

Cells grown up in bacterial culture at ∼37°C to a target OD₆₀₀.

Culture temperature dropped to ∼30°C, and culture is induced with IPTG and harvested after several hours.

Harvest is performed using a centrifuge.

Recovery of protein is performed using a chemical lysis and a MICROFLUIDIZER®, followed by clarification by centrifugation or tangential flow filtration. Lysate is processed immediately or frozen for later use.

Purification by Hydrophobic Interaction Chromatography followed by hydroxyapatite chromatography and/or ion exchange chromatography. Formulated and concentrated using tangential flow filtration.

### HIV Combinectin-Fc Construct; Mammalian Cell Culture

DNA is transfected into appropriate mammalian cells.

Cells grown in cell culture.

Harvested by centrifugation and/or filtration.

Purification using affinity chromatography and ion exchange chromatography.

Formulated and concentrated using tangential flow filtration.

### HIV Combinectin-HuSA construct; Mammalian Cell Culture

DNA is transfected into appropriate mammalian cells.

Cells grown in cell culture.

Harvested by centrifugation and/or filtration.

Purification by Hydrophobic Interaction Chromatography followed by hydroxyapatite chromatography and/or ion exchange chromatography.

Formulated and concentrated using tangential flow filtration.

### Example 2 - Combinectin Potency Assay

MT-2 cells, HEK 293T cells and the proviral DNA clone of NL₄₋₃ were obtained from the NIH AIDS Research and Reference Reagent Program. MT-2 cells were propagated in RPMI 1640 medium supplemented with 10% heat-inactivated fetal bovine serum (FBS), 10 mM HEPES buffer pH 7.55, and 2 mM L-glutamine. HEK 293T cells were propagated in DMEM media supplemented with 10% heat-inactivated FBS, 10 mM HEPES buffer pH 7.55 and 2 mM L-glutamine. Recombinant NL-Rluc virus, in which a section of the *nef* gene from the proviral clone of NL₄₋₃ was replaced with the *Renilla* luciferase gene, was constructed at Bristol-Myers Squibb. The replication-competent virus was harvested 3 days after transfection of HEK 293T cells with the modified pNL-Rluc proviral clone. Transfections were performed using Lipofectamine Plus (Invitrogen, Carlsbad, CA), according to manufacturer's instruction. Virus was titered in MT-2 cells using luciferase enzyme activity as a biomarker. The NL-Rluc virus used to infect MT-2 cells a multiplicity of 0.01 for 1 hour before adding to the peptides in 96-well plates. Peptides were serially diluted three-fold and 11 concentrations were plated in triplicate. After 4-5 days of incubation, cells were processed and quantitated for virus growth by the amount of expressed luciferase. Luciferase was quantitated using the Dual Luciferase kit from Promega (Madison, WI), with modifications to the manufacturer's protocol. The diluted Passive Lysis solution was pre-mixed with the re-suspended Luciferase Assay Reagent and then re-suspended in STOP & GLO® Substrate (2:1:1 ratio). A total of 50 µL of the mixture was added to each aspirated well on assay plates and luciferase activity was measured immediately on a Wallac TriLux (Perkin-Elmer, Waltham, MA). The 50% effective concentration (EC₅₀) was calculated by comparing the amount of luciferase produced in the presence of inhibitory peptide compared to wells where no peptide was added.

### Example 3 - Evaluation of Combinectin Pharmacokinetics

### Human CD4 Transgenic Mouse Model

Male and female heterozygous human CD4 mice were obtained from Jackson Laboratories, Bar Harbor, ME

### WT Mouse PK Studies

8-21 day single IV bolus dose studies were run in female C57B1/6 WT mice to assess the PK properties of the various Combinectins. Fc-Combinectin fusions were dosed at 10mg/kg and HSA-Combinectin fusions were dosed at 8.8mg/kg. Plasma samples were collected in CPD and stored at -80°C until analysis.

### hCD4 Mouse PK Studies

7-10 day single IV bolus dose studies were run in heterozygous hCD4 mice to assess the PK properties of various Combinectins in the presence of target. Combinectins doses and sample collection methods were the same as described above for the WT mice.

### Cynomolgus Monkey Studies

A 1-week single dose study was conducted in female cynos to determine the PK of Combinectins. Following a lmg/kg dose, serum samples were collected at the indicated times, aliquoted and quick frozen for MSD or LC/MS analysis.

### Pharmacokinetic Measurements

Drug levels were measured in mouse or cyno plasma using the Mesoscale technology platform or colorimetric ELISA formats. Fc-Combinectin fusions were captured via the protein PRD828 (BMS) that specifically binds the peptide component of Combinectins and detected using a Goat anti-Human IgG Fc-HRP conjugated pAb (Pierce #31413). HSA-Combinectin fusions were captured via PRD828 and detected using a goat pAb against HSA (Bethyl, TX #A80-229A) that was ruthenium labeled. Sample concentrations were calculated from a standard curve using a 5-parameter logarithmic fit. Non-compartmental analyses were performed using Phoenix WINNONLIN® 6.3 (Pharsight Corporation, Mountain View, CA) using a plasma model and linear up/log down calculation method.

Sample concentrations were calculated from a standard curve using a 5-parameter logarithmic fit. Non-compartmental analyses were performed using Phoenix WINNONLIN® 6.3 (Pharsight Corporation, Mountain View, CA) using a plasma model and linear up/log down calculation method.

## Claims

1. A polypeptide comprising two active domains wherein one domain is an anti-CD4 Adnectin protein, wherein the anti-CD4 Adnectin protein comprises an amino acid sequence at least 80%, 85%, 90%, 95%, 98%, 99% or 100% identical to the amino acid sequence of SEQ ID NOs: 95-114 and the other domain is a HIV fusion peptide inhibitor moiety wherein the HIV fusion peptide inhibitor comprises an amino acid sequence at least 80%, 85%, 90%, 95%, 98%, 99% or 100% identical to the amino acid sequence of SEQ ID NO: 372-392.

2. A polypeptide comprising two active domains wherein one domain is an anti-N17 Adnectin protein wherein the anti-N17 Adnectin protein comprises an amino acid sequence at least 80%, 85%, 90%, 95%, 98%, 99% or 100% identical to the amino acid sequence of SEQ ID NO: 115-371, and the other domain is a HIV fusion peptide inhibitor moiety wherein the HIV fusion peptide inhibitor comprises an amino acid sequence at least 80%, 85%, 90%, 95%, 98%, 99% or 100% identical to the amino acid sequence of SEQ ID NO: 372-392.

3. The polypeptide of claim 1 or 2 wherein the two domains are connected to each other in any order by linkers.

4. The polypeptide of claims 1-3 further comprising one or more pharmacokinetic (PK) moieties selected from the group consisting of polyethylene glycol, sialic acid, Fc, Fc fragment, transferrin, serum albumin, a serum albumin binding protein, and a serum immunoglobulin binding protein.

5. A polypeptide comprising three active domains wherein one domain is an anti-CD4 Adnectin protein wherein the anti-CD4 Adnectin protein comprises an amino acid sequence at least 80%, 85%, 90%, 95%, 98%, 99% or 100% identical to the amino acid sequence of SEQ ID NOs: 95-114, a second domain is an anti-N17 Adnectin protein, wherein the anti-N17 Adnectin protein comprises an amino acid sequence at least 80%, 85%, 90%, 95%, 98%, 99% or 100% identical to the amino acid sequence of SEQ ID NO: 115-371 and the third domain is a HIV fusion peptide inhibitor wherein the HIV fusion peptide inhibitor comprises an amino acid sequence at least 80%, 85%, 90%, 95%, 98%, 99% or 100% identical to the amino acid sequence of SEQ ID NO: 372-392.

6. The polypeptide of claim 5 wherein the three domains are connected to each other in any order by linkers.

7. The polypeptide of claim 5, further comprising one or more pharmacokinetic (PK) moieties selected from the group consisting of polyethylene glycol, sialic acid, Fc, Fc fragment, transferrin, serum albumin, a serum albumin binding protein, and a serum immunoglobulin binding protein.

8. The polypeptide of claim 7, wherein the PK moiety is human serum albumin.

9. The polypeptide according to claim 5 comprising an amino acid sequence at least 80%, 85%, 90%, 95%, 98%, 99% or 100% identical to the non-linker regions of SEQ ID NO: 9.

10. A polypeptide according to claim 5 comprising an amino acid sequence at least 80%, 85%, 90%, 95%, 98%, 99% or 100% identical to the non-linker regions of SEQ ID NO: 10.

11. A polypeptide comprising the amino acid sequence shown in SEQ ID NO: 9.

12. A polypeptide comprising the amino acid sequence shown in SEQ ID NO: 10.

13. A pharmaceutical composition comprising a polypeptide of any one of the preceding claims, and a carrier.

14. A polypeptide according to claims 1-12 for use in therapy.

15. A polypeptide according to claims 1-12 for use in treating HIV.

## Patentansprüche

1. Polypeptid, das zwei aktive Domänen umfasst, wobei eine Domäne ein Anti-CD4-Adnectin-Protein ist, wobei das Anti-CD4-Adnectin-Protein eine Aminosäuresequenz umfasst, die mindestens 80%, 85%, 90%, 95%, 98%, 99% oder 100% identisch mit der Aminosäuresequenz von SEQ ID NO:95-114 ist, und die andere Domäne eine HIV-Fusionspeptid-Inhibitor-Gruppe ist, wobei der HIV-Fusionspeptid-Inhibitor eine Aminosäuresequenz umfasst, die mindestens 80%, 85%, 90%, 95%, 98%, 99% oder 100% identisch mit der Aminosäuresequenz von SEQ ID NO:372-392 ist.

2. Polypeptid, das zwei aktive Domänen umfasst, wobei eine Domäne ein Anti-N17-Adnectin-Protein ist, wobei das Anti-N17-Adnectin-Protein eine Aminosäuresequenz umfasst, die mindestens 80%, 85%, 90%, 95%, 98%, 99% oder 100% identisch mit der Aminosäuresequenz von SEQ ID NO:115-371 ist, und die andere Domäne eine HIV-Fusionspeptid-Inhibitor-Gruppe ist, wobei der HIV-Fusionspeptid-Inhibitor eine Aminosäuresequenz umfasst, die mindestens 80%, 85%, 90%, 95%, 98%, 99% oder 100% identisch mit der Aminosäuresequenz von SEQ ID NO:372-392 ist.

3. Polypeptid nach Anspruch 1 oder 2, wobei die zwei Domänen miteinander in beliebiger Reihenfolge durch Linker verbunden sind.

4. Polypeptid nach den Ansprüchen 1 bis 3, des Weiteren umfassend eine oder mehrere pharmakokinetische (PK) Gruppen, die ausgewählt sind aus der Gruppe bestehend aus Polyethylenglycol, Sialinsäure, Fc, Fc-Fragment, Transferrin, Serum-Albumin, einem Serum-Albumin-Bindungsprotein und einem Serum-Immunglobulin-Bindungsprotein.

5. Polypeptid, das drei aktive Domänen umfasst, wobei eine Domäne ein Anti-CD4-Adnectin-Protein ist, wobei das Anti-CD4-Adnectin-Protein eine Aminosäuresequenz umfasst, die mindestens 80%, 85%, 90%, 95%, 98%, 99% oder 100% identisch mit der Aminosäuresequenz von SEQ ID NO:95-114 ist, eine zweite Domäne ein Anti-N17-Adnectin-Protein ist, wobei das Anti-N17-Adnectin-Protein eine Aminosäuresequenz umfasst, die mindestens 80%, 85%, 90%, 95%, 98%, 99% oder 100% identisch mit der Aminosäuresequenz von SEQ ID NO:115-371 ist, und die dritte Domäne ein HIV-Fusionspeptid-Inhibitor ist, wobei der HIV-Fusionspeptid-Inhibitor eine Aminosäuresequenz umfasst, die mindestens 80%, 85%, 90%, 95%, 98%, 99% oder 100% identisch mit der Aminosäuresequenz von SEQ ID NO:372-392 ist.

6. Polypeptid nach Anspruch 5, wobei die drei Domänen miteinander in beliebiger Reihenfolge durch Linker verbunden sind.

7. Polypeptid nach Anspruch 5, des Weiteren umfassend eine oder mehrere pharmakokinetische (PK) Gruppen, die ausgewählt sind aus der Gruppe bestehend aus Polyethylenglycol, Sialinsäure, Fc, Fc-Fragment, Transferrin, Serum-Albumin, einem Serum-Albumin-Bindungsprotein und einem Serum-Immunglobulin-Bindungsprotein.

8. Polypeptid nach Anspruch 7, wobei die PK-Gruppe humanes Serum-Albumin ist.

9. Polypeptid nach Anspruch 5, das eine Aminosäuresequenz umfasst, die mindestens 80%, 85%, 90%, 95%, 98%, 99% oder 100% identisch mit den nicht-Linker-Regionen von SEQ ID NO:9 ist.

10. Polypeptid nach Anspruch 5, das eine Aminosäuresequenz umfasst, die mindestens 80%, 85%, 90%, 95%, 98%, 99% oder 100% identisch mit den nicht-Linker-Regionen von SEQ ID NO:10 ist.

11. Polypeptid, das die in SEQ ID NO:9 gezeigte Aminosäuresequenz umfasst.

12. Polypeptid, das die in SEQ ID NO:10 gezeigte Aminosäuresequenz umfasst.

13. Arzneimittel, das ein Polypeptid nach einem der vorangehenden Ansprüche und einen Träger umfasst.

14. Polypeptid nach den Ansprüchen 1 bis 12 zur Verwendung in der Therapie.

15. Polypeptid nach den Ansprüchen 1 bis 12 zur Verwendung bei der Behandlung von HIV.

## Revendications

1. Polypeptide comprenant deux domaines actifs, où un domaine est une protéine adnectine anti-CD4, où la protéine adnectine anti-CD4 comprend une séquence d'acides aminés au moins 80 %, 85 %, 90 %, 95 %, 98 %, 99 % ou 100 % identique à la séquence d'acides aminés des SEQ ID NO : 95 à 114 et l'autre domaine est un groupement inhibiteur du peptide de fusion du VIH où l'inhibiteur du peptide de fusion du VIH comprend une séquence d'acides aminés au moins 80 %, 85 %, 90 %, 95 %, 98 %, 99 % ou 100 % identique à la séquence d'acides aminés des SEQ ID NO : 372 à 392.

2. Polypeptide comprenant deux domaines actifs, où un domaine est une protéine adnectine anti-N17, où la protéine adnectine anti-N17 comprend une séquence d'acides aminés au moins 80 %, 85 %, 90 %, 95 %, 98 %, 99 % ou 100 % identique à la séquence d'acides aminés des SEQ ID NO : 115 à 371, et l'autre domaine est un groupement inhibiteur du peptide de fusion du VIH où l'inhibiteur du peptide de fusion du VIH comprend une séquence d'acides aminés au moins 80 %, 85 %, 90 %, 95 %, 98 %, 99 % ou 100 % identique à la séquence d'acides aminés des SEQ ID NO : 372 à 392.

3. Polypeptide selon la revendication 1 ou 2, où les deux domaines sont connectés l'un à l'autre, dans un ordre quelconque, par des segments de liaison.

4. Polypeptide selon les revendications 1 à 3, comprenant en outre un ou plusieurs groupements pharmacocinétiques (PK) sélectionnés dans le groupe consistant en le polyéthylèneglycol, l'acide sialique, Fc, un fragment Fc, la transferrine, la sérumalbumine, une protéine de liaison à la sérumalbumine, et une protéine de liaison à une immunoglobuline sérique.

5. Polypeptide comprenant trois domaines actifs, où un domaine est une protéine adnectine anti-CD4, où la protéine adnectine anti-CD4 comprend une séquence d'acides aminés au moins 80 %, 85 %, 90 %, 95 %, 98 %, 99 % ou 100 % identique à la séquence d'acides aminés des SEQ ID NO : 95 à 114, un deuxième domaine est une protéine adnectine anti-N17, où la protéine adnectine anti-N17 comprend une séquence d'acides aminés au moins 80 %, 85 %, 90 %, 95 %, 98 %, 99 % ou 100 % identique à la séquence d'acides aminés des SEQ ID NO : 115 à 371, et le troisième domaine est un inhibiteur du peptide de fusion du VIH où l'inhibiteur du peptide de fusion du VIH comprend une séquence d'acides aminés au moins 80 %, 85 %, 90 %, 95 %, 98 %, 99 % ou 100 % identique à la séquence d'acides aminés des SEQ ID NO : 372 à 392.

6. Polypeptide selon la revendication 5, où les trois domaines sont connectés les uns aux autres, dans un ordre quelconque, par des segments de liaison.

7. Polypeptide selon la revendication 5, comprenant en outre un ou plusieurs groupements pharmacocinétiques (PK) sélectionnés dans le groupe consistant en le polyéthylèneglycol, l'acide sialique, Fc, un fragment Fc, la transferrine, la sérumalbumine, une protéine de liaison à la sérumalbumine, et une protéine de liaison à une immunoglobuline sérique.

8. Polypeptide selon la revendication 7, où le groupement PK est la sérumalbumine humaine.

9. Polypeptide selon la revendication 5, comprenant une séquence d'acides aminés au moins 80 %, 85 %, 90 %, 95 %, 98 %, 99 % ou 100 % identique aux régions non-segments de liaison de la SEQ ID NO : 9.

10. Polypeptide selon la revendication 5, comprenant une séquence d'acides aminés au moins 80 %, 85 %, 90 %, 95 %, 98 %, 99 % ou 100 % identique aux régions non-segments de liaison de la SEQ ID NO : 10.

11. Polypeptide comprenant la séquence d'acides aminés montrée dans la SEQ ID NO : 9.

12. Polypeptide comprenant la séquence d'acides aminés montrée dans la SEQ ID NO : 10.

13. Composition pharmaceutique comprenant un polypeptide selon l'une quelconque des revendications précédentes, et un véhicule.

14. Polypeptide selon les revendications 1 à 12 pour utilisation en thérapie.

15. Polypeptide selon les revendications 1 à 12 pour utilisation dans le traitement du VIH.


### SEQUENCE LISTING

<110> BRISTOL-MYERS SQUIBB COMPANY
<120> POLYPEPTIDES TARGETING HIV FUSION
<130> 12358-WO-PCT
<150> US 62/152,271
   <151> 2015-04-24
<150> US 62/257,474
   <151> 2015-11-19
<160> 428
<170> PatentIn version 3.5
<210> 1
   <211> 94
   <212> PRT
   <213> Artificial Sequence
<220>
   <223> Human fibronectin
<400> 1
   Val Ser Asp Val Pro Arg Asp Leu Glu Val Val Ala Ala Thr Pro Thr
   1               5                   10                  15
   Ser Leu Leu Ile Ser Trp Asp Ala Pro Ala Val Thr Val Arg Tyr Tyr
               20                  25                  30
   Arg Ile Thr Tyr Gly Glu Thr Gly Gly Asn Ser Pro Val Gln Glu Phe
           35                  40                  45
   Thr Val Pro Gly Ser Lys Ser Thr Ala Thr Ile Ser Gly Leu Lys Pro
       50                  55                  60
   Gly Val Asp Tyr Thr Ile Thr Val Tyr Ala Val Thr Gly Arg Gly Asp
   65                  70                  75                  80
   Ser Pro Ala Ser Ser Lys Pro Ile Ser Ile Asn Tyr Arg Thr
                   85                  90
<210> 2
   <211> 172
   <212> PRT
   <213> Artificial Sequence
<220>
   <223> GP41 External domain
<400> 2
   Ala Val Gly Ile Gly Ala Met Phe Leu Gly Phe Leu Gly Ala Ala Gly
   1               5                   10                  15
   Ser Thr Met Gly Ala Ala Ser Val Thr Leu Thr Val Gln Ala Arg Gln
               20                  25                  30
   Leu Leu Ser Gly Ile Val Gln Gln Gln Asn Asn Leu Leu Arg Ala Ile
           35                  40                  45
   Glu Ala Gln Gln His Leu Leu Gln Leu Thr Val Trp Gly Ile Lys Gln
       50                  55                  60
   Leu Gln Ala Arg Val Leu Ala Val Glu Arg Tyr Leu Lys Asp Gln Gln
   65                  70                  75                  80
   Leu Leu Gly Ile Trp Gly Cys Ser Gly Lys Leu Ile Cys Thr Thr Ala
                   85                  90                  95
   Val Pro Trp Asn Ala Ser Trp Ser Asn Lys Ser Leu Asp Glu Ile Trp
               100                 105                 110
   Asn Asn Met Thr Trp Met Glu Trp Glu Arg Glu Ile Asp Asn Tyr Thr
           115                 120                 125
   Gly Leu Ile Tyr Thr Leu Ile Glu Glu Ser Gln Asn Gln Gln Glu Lys
       130                 135                 140
   Asn Glu Gln Glu Leu Leu Glu Leu Asp Lys Trp Ala Ser Leu Trp Asn
   145                 150                 155                 160
   Trp Phe Asp Ile Thr Asn Trp Leu Trp Tyr Ile Lys
                   165                 170
<210> 3
   <211> 251
   <212> PRT
   <213> Artificial Sequence
<220>
   <223> Combinectin
<400> 3
   Gly Val Ser Asp Val Pro Arg Asp Leu Glu Val Val Ala Ala Thr Pro
   1               5                   10                  15
   Thr Ser Leu Leu Ile Ser Trp Asp Ala Pro Ala Val Thr Val His Ser
               20                  25                  30
   Tyr His Ile Gln Tyr Trp Pro Leu Gly Ser Tyr Gln Arg Tyr Gln Val
           35                  40                  45
   Phe Ser Val Pro Gly Ser Lys Ser Thr Ala Thr Ile Ser Gly Leu Lys
       50                  55                  60
   Pro Gly Val Glu Tyr Gln Ile Arg Val Tyr Ala Glu Thr Gly Arg Gly
   65                  70                  75                  80
   Glu Ser Asp Gln Ser Leu Gly Trp Ile Gln Ile Gly Tyr Arg Thr Glu
                   85                  90                  95
   Glu Ser Pro Glu Pro Glu Thr Pro Glu Asp Glu Gly Val Ser Asp Val
               100                 105                 110
   Pro Arg Asp Leu Glu Val Val Ala Ala Thr Pro Thr Ser Leu Leu Ile
           115                 120                 125
   Ser Trp Gln Tyr Lys Val His Pro Tyr Arg Tyr Tyr Arg Ile Thr Tyr
       130                 135                 140
   Gly Glu Thr Gly Gly Asn Ser Pro Val Gln Glu Phe Thr Val Pro Ser
   145                 150                 155                 160
   Val Leu Ser Thr Ala Glu Ile Ser Gly Leu Lys Pro Gly Val Asp Tyr
                   165                 170                 175
   Thr Ile Thr Val Tyr Ala Val Thr Arg Gly Val Asp Ser Ala Pro Ile
               180                 185                 190
   Ser Ile Asn Tyr Arg Thr Pro Gly Gly Gly Gly Ser Gly Gly Gly Gly
           195                 200                 205
   Ser Gly Gly Gly Gly Ser Gly Gly Gly Gly Ser Glu Tyr Glu Ala Arg
       210                 215                 220
   Ile Glu Ala Leu Ile Arg Ala Ala Gln Glu Gln Gln Glu Lys Asn Glu
   225                 230                 235                 240
   Ala Ala Leu Arg Glu Leu Tyr Lys Trp Ala Leu
                   245                 250
<210> 4
   <211> 497
   <212> PRT
   <213> Artificial Sequence
<220>
   <223> Combinectin
<400> 4
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Ala Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Glu Glu Met Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Glu Ser Pro Glu Pro Glu Thr Pro Glu Asp Glu Ser Pro Glu Pro
   225                 230                 235                 240
   Glu Thr Pro Glu Asp Glu Gly Val Ser Asp Val Pro Arg Asp Leu Glu
                   245                 250                 255
   Val Val Ala Ala Thr Pro Thr Ser Leu Leu Ile Ser Trp Asp Ala Pro
               260                 265                 270
   Ala Val Thr Val His Ser Tyr His Ile Gln Tyr Trp Pro Leu Gly Ser
           275                 280                 285
   Tyr Gln Arg Tyr Gln Val Phe Ser Val Pro Gly Ser Lys Ser Thr Ala
       290                 295                 300
   Thr Ile Ser Gly Leu Lys Pro Gly Val Glu Tyr Gln Ile Arg Val Tyr
   305                 310                 315                 320
   Ala Glu Thr Gly Arg Gly Glu Ser Asp Gln Ser Leu Gly Trp Ile Gln
                   325                 330                 335
   Ile Gly Tyr Arg Thr Glu Glu Ser Pro Glu Pro Glu Thr Pro Glu Asp
               340                 345                 350
   Glu Gly Val Ser Asp Val Pro Arg Asp Leu Glu Val Val Ala Ala Thr
           355                 360                 365
   Pro Thr Ser Leu Leu Ile Ser Trp Gln Tyr Lys Val His Pro Tyr Arg
       370                 375                 380
   Tyr Tyr Arg Ile Thr Tyr Gly Glu Thr Gly Gly Asn Ser Pro Val Gln
   385                 390                 395                 400
   Glu Phe Thr Val Pro Ser Val Leu Ser Thr Ala Glu Ile Ser Gly Leu
                   405                 410                 415
   Lys Pro Gly Val Asp Tyr Thr Ile Thr Val Tyr Ala Val Thr Arg Gly
               420                 425                 430
   Val Asp Ser Ala Pro Ile Ser Ile Asn Tyr Arg Thr Pro Gly Gly Gly
           435                 440                 445
   Gly Ser Gly Gly Gly Gly Ser Gly Gly Gly Gly Ser Gly Gly Gly Gly
       450                 455                 460
   Ser Glu Tyr Glu Ala Arg Ile Glu Ala Leu Ile Arg Ala Ala Gln Glu
   465                 470                 475                 480
   Gln Gln Glu Lys Asn Glu Ala Ala Leu Arg Glu Leu Tyr Lys Trp Ala
                   485                 490                 495
   Leu
<210> 5
   <211> 251
   <212> PRT
   <213> Artificial Sequence
<220>
   <223> Combinectin
<400> 5
   Gly Val Ser Asp Val Pro Arg Asp Leu Glu Val Val Ala Ala Thr Pro
   1               5                   10                  15
   Thr Ser Leu Leu Ile Ser Trp Asp Ala Pro Ala Val Thr Val His Ser
               20                  25                  30
   Tyr His Ile Gln Tyr Trp Pro Leu Gly Ser Tyr Gln Arg Tyr Gln Val
           35                  40                  45
   Phe Ser Val Pro Gly Ser Lys Ser Thr Ala Thr Ile Ser Gly Leu Lys
       50                  55                  60
   Pro Gly Val Glu Tyr Gln Ile Arg Val Tyr Ala Glu Thr Gly Arg Gly
   65                  70                  75                  80
   Glu Ser Asp Gln Ser Leu Gly Trp Ile Gln Ile Gly Tyr Arg Thr Glu
                   85                  90                  95
   Glu Ser Pro Glu Pro Glu Thr Pro Glu Asp Glu Gly Val Ser Asp Val
               100                 105                 110
   Pro Arg Asp Leu Glu Val Val Ala Ala Thr Pro Thr Ser Leu Leu Ile
           115                 120                 125
   Ser Trp Glu Tyr Asn Val Asn Pro Tyr Arg Tyr Tyr Arg Ile Thr Tyr
       130                 135                 140
   Gly Glu Thr Gly Gly Asn Ser Pro Val Gln Glu Phe Thr Val Pro Ser
   145                 150                 155                 160
   Val Leu Ser Ser Ala Gln Ile Ser Gly Leu Lys Pro Gly Val Asp Tyr
                   165                 170                 175
   Thr Ile Thr Val Tyr Ala Val Thr Arg Gly Val Asp Ser Ala Pro Ile
               180                 185                 190
   Ser Ile Asn Tyr Arg Thr Pro Gly Gly Gly Gly Ser Gly Gly Gly Gly
           195                 200                 205
   Ser Gly Gly Gly Gly Ser Gly Gly Gly Gly Ser Glu Tyr Glu Ala Arg
   210 215 220
   Ile Glu Ala Leu Ile Arg Ala Ala Gln Glu Gln Gln Glu Lys Asn Glu
   225 230 235 240
   Ala Ala Leu Arg Glu Leu Trp Lys Trp Ala Ser
   245 250
<210> 6
   <211> 497
   <212> PRT
   <213> Artificial Sequence
<220>
   <223> Combinectin
<400> 6
   Asp Lys Thr His Thr Cys Pro Pro Cys Pro Ala Pro Glu Leu Leu Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Ala Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Pro Ala Pro Ile
   100 105 110
   Glu Lys Thr Ile Ser Lys Ala Lys Gly Gln Pro Arg Glu Pro Gln Val
   115 120 125
   Tyr Thr Leu Pro Pro Ser Arg Glu Glu Met Thr Lys Asn Gln Val Ser
   130 135 140
   Leu Thr Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Glu Ser Pro Glu Pro Glu Thr Pro Glu Asp Glu Ser Pro Glu Pro
   225                 230                 235                 240
   Glu Thr Pro Glu Asp Glu Gly Val Ser Asp Val Pro Arg Asp Leu Glu
                   245                 250                 255
   Val Val Ala Ala Thr Pro Thr Ser Leu Leu Ile Ser Trp Asp Ala Pro
               260                 265                 270
   Ala Val Thr Val His Ser Tyr His Ile Gln Tyr Trp Pro Leu Gly Ser
           275                 280                 285
   Tyr Gln Arg Tyr Gln Val Phe Ser Val Pro Gly Ser Lys Ser Thr Ala
       290                 295                 300
   Thr Ile Ser Gly Leu Lys Pro Gly Val Glu Tyr Gln Ile Arg Val Tyr
   305                 310                 315                 320
   Ala Glu Thr Gly Arg Gly Glu Ser Asp Gln Ser Leu Gly Trp Ile Gln
                   325                 330                 335
   Ile Gly Tyr Arg Thr Glu Glu Ser Pro Glu Pro Glu Thr Pro Glu Asp
               340                 345                 350
   Glu Gly Val Ser Asp Val Pro Arg Asp Leu Glu Val Val Ala Ala Thr
           355                 360                 365
   Pro Thr Ser Leu Leu Ile Ser Trp Glu Tyr Asn Val Asn Pro Tyr Arg
       370                 375                 380
   Tyr Tyr Arg Ile Thr Tyr Gly Glu Thr Gly Gly Asn Ser Pro Val Gln
   385                 390                 395                 400
   Glu Phe Thr Val Pro Ser Val Leu Ser Ser Ala Gln Ile Ser Gly Leu
                   405                 410                 415
   Lys Pro Gly Val Asp Tyr Thr Ile Thr Val Tyr Ala Val Thr Arg Gly
   420 425 430
   Val Asp Ser Ala Pro Ile Ser Ile Asn Tyr Arg Thr Pro Gly Gly Gly
   435 440 445
   Gly Ser Gly Gly Gly Gly Ser Gly Gly Gly Gly Ser Gly Gly Gly Gly
   450 455 460
   Ser Glu Tyr Glu Ala Arg Ile Glu Ala Leu Ile Arg Ala Ala Gln Glu
   465 470 475 480
   Gln Gln Glu Lys Asn Glu Ala Ala Leu Arg Glu Leu Trp Lys Trp Ala
   485 490 495
   Ser
<210> 7
   <211> 251
   <212> PRT
   <213> Artificial Sequence
<220>
   <223> Combinectin
<400> 7
   Gly Val Ser Asp Val Pro Arg Asp Leu Glu Val Val Ala Ala Thr Pro
   1 5 10 15
   Thr Ser Leu Leu Ile Ser Trp Asp Ala Pro Ala Val Thr Val His Ser
   20 25 30
   Tyr His Ile Gln Tyr Trp Pro Leu Gly Ser Tyr Gln Arg Tyr Gln Val
   35 40 45
   Phe Ser Val Pro Gly Ser Lys Ser Thr Ala Thr Ile Ser Gly Leu Lys
   50 55 60
   Pro Gly Val Glu Tyr Gln Ile Arg Val Tyr Ala Glu Thr Gly Arg Gly
   65 70 75 80
   Glu Ser Asp Gln Ser Leu Gly Trp Ile Gln Ile Gly Tyr Arg Thr Pro
   85 90 95
   Glu Ser Pro Glu Pro Glu Thr Pro Glu Asp Glu Gly Val Ser Asp Val
   100 105 110
   Pro Arg Asp Leu Glu Val Val Ala Ala Thr Pro Thr Ser Leu Leu Ile
   115 120 125
   Ser Trp Glu Tyr Lys Val His Pro Tyr Arg Tyr Tyr Arg Ile Thr Tyr
   130 135 140
   Gly Glu Thr Gly Gly Asn Ser Pro Val Gln Glu Phe Thr Val Pro Ser
   145 150 155 160
   Val Leu Ser Ser Ala Glu Ile Ser Gly Leu Lys Pro Gly Val Asp Tyr
   165 170 175
   Thr Ile Thr Val Tyr Ala Val Thr Tyr Gly Ile Asp Ser Pro Pro Ile
   180 185 190
   Ser Ile Asn Tyr Arg Thr Glu Gly Gly Gly Gly Ser Gly Gly Gly Gly
   195 200 205
   Ser Gly Gly Gly Gly Ser Gly Gly Gly Gly Ser Glu Tyr Glu Ala Arg
   210 215 220
   Ile Glu Ala Leu Ile Arg Ala Ala Gln Glu Gln Gln Glu Lys Asn Glu
   225 230 235 240
   Ala Ala Leu Arg Glu Leu Tyr Lys Trp Ala Leu
   245 250
<210> 8
   <211> 857
   <212> PRT
   <213> Artificial Sequence
<220>
   <223> Combinectin
<400> 8
   Asp Ala His Lys Ser Glu Val Ala His Arg Phe Lys Asp Leu Gly Glu
   1 5 10 15
   Glu Asn Phe Lys Ala Leu Val Leu Ile Ala Phe Ala Gln Tyr Leu Gln
   20 25 30
   Gln Ala Pro Phe Glu Asp His Val Lys Leu Val Asn Glu Val Thr Glu
   35 40 45
   Phe Ala Lys Thr Cys Val Ala Asp Glu Ser Ala Glu Asn Cys Asp Lys
   50 55 60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu Glu Ser Pro Glu Pro Glu Thr
               580                 585                 590
   Pro Glu Asp Glu Ser Pro Glu Pro Glu Thr Pro Glu Asp Glu Gly Val
           595                 600                 605
   Ser Asp Val Pro Arg Asp Leu Glu Val Val Ala Ala Thr Pro Thr Ser
       610                 615                 620
   Leu Leu Ile Ser Trp Asp Ala Pro Ala Val Thr Val His Ser Tyr His
   625                 630                 635                 640
   Ile Gln Tyr Trp Pro Leu Gly Ser Tyr Gln Arg Tyr Gln Val Phe Ser
                   645                 650                 655
   Val Pro Gly Ser Lys Ser Thr Ala Thr Ile Ser Gly Leu Lys Pro Gly
               660                 665                 670
   Val Glu Tyr Gln Ile Arg Val Tyr Ala Glu Thr Gly Arg Gly Glu Ser
           675                 680                 685
   Asp Gln Ser Leu Gly Trp Ile Gln Ile Gly Tyr Arg Thr Pro Glu Ser
       690                 695                 700
   Pro Glu Pro Glu Thr Pro Glu Asp Glu Gly Val Ser Asp Val Pro Arg
   705                 710                 715                 720
   Asp Leu Glu Val Val Ala Ala Thr Pro Thr Ser Leu Leu Ile Ser Trp
                   725                 730                 735
   Glu Tyr Lys Val His Pro Tyr Arg Tyr Tyr Arg Ile Thr Tyr Gly Glu
               740                 745                 750
   Thr Gly Gly Asn Ser Pro Val Gln Glu Phe Thr Val Pro Ser Val Leu
           755                 760                 765
   Ser Ser Ala Glu Ile Ser Gly Leu Lys Pro Gly Val Asp Tyr Thr Ile
       770                 775                 780
   Thr Val Tyr Ala Val Thr Tyr Gly Ile Asp Ser Pro Pro Ile Ser Ile
   785                 790                 795                 800
   Asn Tyr Arg Thr Glu Gly Gly Gly Gly Ser Gly Gly Gly Gly Ser Gly
                   805                 810                 815
   Gly Gly Gly Ser Gly Gly Gly Gly Ser Glu Tyr Glu Ala Arg Ile Glu
   820 825 830
   Ala Leu Ile Arg Ala Ala Gln Glu Gln Gln Glu Lys Asn Glu Ala Ala
   835 840 845
   Leu Arg Glu Leu Tyr Lys Trp Ala Leu
   850 855
<210> 9
   <211> 253
   <212> PRT
   <213> Artificial Sequence
<220>
   <223> Combinectin
<400> 9
   Gly Val Ser Asp Val Pro Arg Asp Leu Glu Val Val Ala Ala Thr Pro
   1               5                   10                  15
   Thr Ser Leu Leu Ile Ser Trp Asp Ala Pro Ala Val Thr Val His Ser
               20                  25                  30
   Tyr His Ile Gln Tyr Trp Pro Leu Gly Ser Tyr Gln Arg Tyr Gln Val
           35                  40                  45
   Phe Ser Val Pro Gly Ser Lys Ser Thr Ala Thr Ile Ser Gly Leu Lys
       50                  55                  60
   Pro Gly Val Glu Tyr Gln Ile Arg Val Tyr Ala Glu Thr Gly Gly Ala
   65                  70                  75                  80
   Asp Ser Asp Gln Ser Phe Gly Trp Ile Gln Ile Gly Tyr Arg Thr Pro
                   85                  90                  95
   Glu Ser Pro Glu Pro Glu Thr Pro Glu Asp Glu Gly Val Ser Asp Val
               100                 105                 110
   Pro Arg Asp Leu Glu Val Val Ala Ala Thr Pro Thr Ser Leu Leu Ile
           115                 120                 125
   Ser Trp Glu Tyr Lys Val His Pro Tyr Arg Tyr Tyr Arg Ile Thr Tyr
       130                 135                 140
   Gly Glu Thr Gly Gly Asn Ser Pro Val Gln Glu Phe Thr Val Pro Ser
   145                 150                 155                 160
   Val Leu Ser Thr Ala Glu Ile Ser Gly Leu Lys Pro Gly Val Asp Tyr
                   165                 170                 175
   Thr Ile Thr Val Tyr Ala Val Thr Arg Gly Val Asp Ser Ala Pro Ile
               180                 185                 190
   Ser Ile Asn Tyr Arg Thr Pro Gly Gly Gly Gly Ser Gly Gly Gly Gly
           195                 200                 205
   Ser Gly Gly Gly Gly Ser Gly Gly Gly Gly Thr Ile Ala Glu Tyr Ala
       210                 215                 220
   Ala Arg Ile Glu Ala Leu Ile Arg Ala Ala Gln Glu Gln Gln Glu Lys
   225                 230                 235                 240
   Asn Glu Ala Ala Leu Arg Glu Leu Tyr Lys Trp Ala Ser
                   245                 250
<210> 10
   <211> 863
   <212> PRT
   <213> Artificial Sequence
<220>
   <223> Combinectin
<400> 10
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Ala Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Lys Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385 390 395 400
   Tyr Lys Phe Gln Asn Ala Leu Leu Val Arg Tyr Thr Lys Lys Val Pro
   405 410 415
   Gln Val Ser Thr Pro Thr Leu Val Glu Val Ser Arg Asn Leu Gly Lys
   420 425 430
   Val Gly Ser Lys Cys Cys Lys His Pro Glu Ala Lys Arg Met Pro Cys
   435 440 445
   Ala Glu Asp Tyr Leu Ser Val Val Leu Asn Gln Leu Cys Val Leu His
   450 455 460
   Glu Lys Thr Pro Val Ser Asp Arg Val Thr Lys Cys Cys Thr Glu Ser
   465 470 475 480
   Leu Val Asn Arg Arg Pro Cys Phe Ser Ala Leu Glu Val Asp Glu Thr
   485 490 495
   Tyr Val Pro Lys Glu Phe Asn Ala Glu Thr Phe Thr Phe His Ala Asp
   500 505 510
   Ile Cys Thr Leu Ser Glu Lys Glu Arg Gln Ile Lys Lys Gln Thr Ala
   515 520 525
   Leu Val Glu Leu Val Lys His Lys Pro Lys Ala Thr Lys Glu Gln Leu
   530 535 540
   Lys Ala Val Met Asp Asp Phe Ala Ala Phe Val Glu Lys Cys Cys Lys
   545 550 555 560
   Ala Asp Asp Lys Glu Thr Cys Phe Ala Glu Glu Gly Lys Lys Leu Val
   565 570 575
   Ala Ala Ser Gln Ala Ala Leu Gly Leu Gly Gly Gly Gly Ser Gly Gly
   580 585 590
   Gly Gly Ser Gly Gly Gly Gly Ser Gly Gly Gly Gly Ser Gly Gly Gly
   595 600 605
   Gly Ser Gly Val Ser Asp Val Pro Arg Asp Leu Glu Val Val Ala Ala
   610 615 620
   Thr Pro Thr Ser Leu Leu Ile Ser Trp Asp Ala Pro Ala Val Thr Val
   625 630 635 640
   His Ser Tyr His Ile Gln Tyr Trp Pro Leu Gly Ser Tyr Gln Arg Tyr
   645 650 655
   Gln Val Phe Ser Val Pro Gly Ser Lys Ser Thr Ala Thr Ile Ser Gly
   660 665 670
   Leu Lys Pro Gly Val Glu Tyr Gln Ile Arg Val Tyr Ala Glu Thr Gly
   675 680 685
   Gly Ala Asp Ser Asp Gln Ser Phe Gly Trp Ile Gln Ile Gly Tyr Arg
   690 695 700
   Thr Pro Glu Ser Pro Glu Pro Glu Thr Pro Glu Asp Glu Gly Val Ser
   705 710 715 720
   Asp Val Pro Arg Asp Leu Glu Val Val Ala Ala Thr Pro Thr Ser Leu
   725 730 735
   Leu Ile Ser Trp Glu Tyr Lys Val His Pro Tyr Arg Tyr Tyr Arg Ile
   740 745 750
   Thr Tyr Gly Glu Thr Gly Gly Asn Ser Pro Val Gln Glu Phe Thr Val
   755 760 765
   Pro Ser Val Leu Ser Thr Ala Glu Ile Ser Gly Leu Lys Pro Gly Val
   770 775 780
   Asp Tyr Thr Ile Thr Val Tyr Ala Val Thr Arg Gly Val Asp Ser Ala
   785 790 795 800
   Pro Ile Ser Ile Asn Tyr Arg Thr Pro Gly Gly Gly Gly Ser Gly Gly
   805 810 815
   Gly Gly Ser Gly Gly Gly Gly Ser Gly Gly Gly Gly Thr Ile Ala Glu
   820 825 830
   Tyr Ala Ala Arg Ile Glu Ala Leu Ile Arg Ala Ala Gln Glu Gln Gln
   835 840 845
   Glu Lys Asn Glu Ala Ala Leu Arg Glu Leu Tyr Lys Trp Ala Ser
   850 855 860
<210> 11
   <211> 227
   <212> PRT
   <213> Artificial Sequence
<220>
   <223> human IgG1 immunoglobulin Fc domain
<400> 11
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Glu Glu Met Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 12
   <211> 16
   <212> PRT
   <213> Artificial Sequence
<220>
   <223> IgGlFc hinge region
<400> 12
   Asp Lys Thr His Thr Cys Pro Pro Cys Pro Ala Pro Glu Leu Leu Gly
   1               5                   10                  15
<210> 13
   <211> 19
   <212> PRT
   <213> Artificial Sequence
<220>
   <223> Adnectin
<400> 13
   His Ser Tyr His Ile Gln Tyr Trp Pro Leu Gly Ser Tyr Gln Arg Tyr
   1               5                   10                  15
   Gln Val Phe
<210> 14
   <211> 25
   <212> PRT
   <213> Artificial Sequence
<220>
   <223> Adnectin
<400> 14
   Glu Tyr Gln Ile Arg Val Tyr Ala Glu Thr Gly Gly Ala Asp Ser Asp
   1               5                   10                  15
   Gln Ser Met Gly Trp Ile Gln Ile Gly
               20                  25
<210> 15
   <211> 19
   <212> PRT
   <213> Artificial Sequence
<220>
   <223> Adnectin
<400> 15
   Leu Ser Tyr His Ile Gln Tyr Trp Pro Leu Gly Leu Tyr Gln Ala Tyr
   1               5                   10                  15
   Gln Val Phe
<210> 16
   <211> 25
   <212> PRT
   <213> Artificial Sequence
<220>
   <223> Adnectin
<400> 16
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 17
   <211> 19
   <212> PRT
   <213> Artificial Sequence
<220>
   <223> Adnectin
<400> 17
   His Ala Tyr His Ile Gln Tyr Trp Pro Leu Gly Phe Tyr Gln Gly Tyr
   1               5                   10                  15
   Gln Val Phe
<210> 18
   <211> 25
   <212> PRT
   <213> Artificial Sequence
<220>
   <223> Adnectin
<400> 18
   Glu Tyr Gln Ile Arg Val Tyr Ala Glu Thr Gly Leu Gly Asp Ala His
   1               5                   10                  15
   Gln Ser Leu Gly Trp Ile Gln Ile Gly
               20                  25
<210> 19
   <211> 19
   <212> PRT
   <213> Artificial Sequence
<220>
   <223> Adnectin
<400> 19
   Leu Ala Tyr His Ile Gln Tyr Trp Pro Leu Gly Trp Tyr Gln Arg Tyr
   1               5                   10                  15
   Gln Ile Phe
<210> 20
   <211> 25
   <212> PRT
   <213> Artificial Sequence
<220>
   <223> Adnectin
<400> 20
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 21
   <211> 19
   <212> PRT
   <213> Artificial Sequence
<220>
   <223> Adnectin
<400> 21
   Leu Ala Tyr His Ile Gln Tyr Trp Pro Leu Gly Trp Tyr Gln Arg Tyr
   1               5                   10                  15
   Gln Val Phe
<210> 22
   <211> 25
   <212> PRT
   <213> Artificial Sequence
<220>
   <223> Adnectin
<400> 22
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 23
   <211> 19
   <212> PRT
   <213> Artificial Sequence
<220>
   <223> Adnectin
<400> 23
   His Phe Tyr His Ile Gln Tyr Trp Pro Leu Gly Leu Tyr His Leu Tyr
   1               5                   10                  15
   Gln Val Phe
<210> 24
   <211> 25
   <212> PRT
   <213> Artificial Sequence
<220>
   <223> Adnectin
<400> 24
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 25
   <211> 19
   <212> PRT
   <213> Artificial Sequence
<220>
   <223> Adnectin
<400> 25
   Tyr Ser Tyr His Ile Gln Tyr Trp Pro Leu Gly Trp Tyr His Arg Tyr
   1               5                   10                  15
   Gln Val Phe
<210> 26
   <211> 25
   <212> PRT
   <213> Artificial Sequence
<220>
   <223> Adnectin
<400> 26
   Glu Tyr Gln Ile Arg Val Tyr Ala Glu Thr Gly Ala Asp Asp Pro Val
   1               5                   10                  15
   Gln Ala Leu Gly Trp Ile Gln Ile Gly
               20                  25
<210> 27
   <211> 19
   <212> PRT
   <213> Artificial Sequence
<220>
   <223> Adnectin
<400> 27
   Arg Cys Tyr His Ile Gln Tyr Trp Pro Leu Gly Leu Tyr Pro Leu Tyr
   1               5                   10                  15
   Gln Val Phe
<210> 28
   <211> 25
   <212> PRT
   <213> Artificial Sequence
<220>
   <223> Adnectin
<400> 28
   Glu Tyr Gln Ile Arg Val Tyr Ala Glu Thr Gly Asp Glu Ser Ser Val
   1               5                   10                  15
   Gln Pro Phe Gly Trp Ile Gln Ile Gly
               20                  25
<210> 29
   <211> 19
   <212> PRT
   <213> Artificial Sequence
<220>
   <223> Adnectin
<400> 29
   Tyr Ser Tyr His Ile Gln Tyr Trp Pro Leu Gly Trp Tyr Gln Arg Tyr
   1 5 10 15
   Gln Val Phe
<210> 30
   <211> 25
   <212> PRT
   <213> Artificial Sequence
<220>
   <223> Adnectin
<400> 30
   Glu Tyr Gln Ile Arg Val Tyr Ala Glu Thr Asp Gly Gly Arg Ser Gln
   1 5 10 15
   Gln Ser Phe Gly Trp Ile Gln Ile Gly
   20 25
<210> 31
   <211> 19
   <212> PRT
   <213> Artificial Sequence
<220>
   <223> Adnectin
<400> 31
   Ser Ser Tyr His Ile Gln Tyr Trp Pro Leu Gly Ala Tyr Gln Arg Tyr
   1 5 10 15
   Gln Val Phe
<210> 32
   <211> 25
   <212> PRT
   <213> Artificial Sequence
<220>
   <223> Adnectin
<400> 32
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 33
   <211> 19
   <212> PRT
   <213> Artificial Sequence
<220>
   <223> Adnectin
<400> 33
   His Ala Tyr His Ile Gln Tyr Trp Pro Leu Gly Leu Tyr Gln Arg Tyr
   1               5                   10                  15
   Gln Val Phe
<210> 34
   <211> 25
   <212> PRT
   <213> Artificial Sequence
<220>
   <223> Adnectin
<400> 34
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 35
   <211> 19
   <212> PRT
   <213> Artificial Sequence
<220>
   <223> Adnectin
<400> 35
   His Ala Tyr Tyr Ile Gln Tyr Trp Pro Leu Gly Ser Tyr Gln Phe Tyr
   1               5                   10                  15
   Gln Val Phe
<210> 36
   <211> 25
   <212> PRT
   <213> Artificial Sequence
<220>
   <223> Adnectin
<400> 36
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 37
   <211> 19
   <212> PRT
   <213> Artificial Sequence
<220>
   <223> Adnectin
<400> 37
   His Ser Tyr His Ile Gln Tyr Trp Pro Leu Gly Ser Tyr Leu Arg Tyr
   1 5 10 15
   Gln Val Phe
<210> 38
   <211> 25
   <212> PRT
   <213> Artificial Sequence
<220>
   <223> Adnectin
<400> 38
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 39
   <211> 19
   <212> PRT
   <213> Artificial Sequence
<220>
   <223> Adnectin
<400> 39
   Leu Ser Tyr His Ile Gln Tyr Trp Pro Leu Gly Phe Tyr Gln Arg Tyr
   1 5 10 15
   Gln Val Phe
<210> 40
   <211> 25
   <212> PRT
   <213> Artificial Sequence
<220>
   <223> Adnectin
<400> 40
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 41
   <211> 19
   <212> **PRT**
   <213> **Artificial** Sequence
<220>
   <223> Adnectin
<400> **41**
   Ser Ala Tyr His Ile Gln Tyr Trp Pro Leu Gly Trp Tyr His Arg Tyr
   1               5                   10                  15
   Gln Ile Phe
<210> 42
   <211> 25
   <212> PRT
   <213> Artificial Sequence
<220>
   <223> Adnectin
<400> 42
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 43
   <211> 19
   <212> PRT
   <213> Artificial Sequence
<220>
   <223> Adnectin
<400> 43
   Tyr Ser Tyr His Ile Gln Tyr Trp Pro Leu Gly Ala Tyr Ser Arg His
   1               5                   10                  15
   Gln Leu Phe
<210> 44
   <211> 25
   <212> PRT
   <213> Artificial Sequence
<220>
   <223> Adnectin
<400> 44
   Glu Tyr Gln Ile Arg Val Tyr Ala Glu Thr Gly Gly Asp Gly Ser Glu
   1               5                   10                  15
   Met Tyr Phe Gly Trp Ile Gln Ile Gly
               20                  25
<210> 45
   <211> 19
   <212> PRT
   <213> Artificial Sequence
<220>
   <223> Adnectin
<400> 45
   Leu Ala Tyr His Ile Gln Tyr Trp Pro Leu Gly Trp Tyr His Leu Tyr
   1               5                   10                  15
   Gln Val Phe
<210> 46
   <211> 25
   <212> PRT
   <213> Artificial Sequence
<220>
   <223> Adnectin
<400> 46
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 47
   <211> 19
   <212> PRT
   <213> Artificial Sequence
<220>
   <223> Adnectin
<400> 47
   Leu Ala Tyr His Ile Gln Tyr Trp Pro Leu Gly Trp Tyr Gln Leu Tyr
   1               5                   10                  15
   Lys Val Phe
<210> 48
   <211> 25
   <212> PRT
   <213> Artificial Sequence
<220>
   <223> Adnectin
<400> 48
   Asp Tyr Gln Ile Arg Val Tyr Ala Glu Thr Ser Gly Glu Ser Ser Glu
   1               5                   10                  15
   Gln Tyr Leu Gly Trp Ile Gln Ile Gly
               20                  25
<210> 49
   <211> 19
   <212> PRT
   <213> Artificial Sequence
<220>
   <223> Adnectin
<400> 49
   His Ser Tyr His Ile Gln Tyr Trp Pro Leu Gly Trp Tyr Gln Leu Tyr
   1               5                   10                  15
   Gln Val Phe
<210> 50
   <211> 25
   <212> PRT
   <213> Artificial Sequence
<220>
   <223> Adnectin
<400> 50
   Glu Tyr Gln Ile Arg Val Tyr Ala Glu Thr Glu Val Asp Ser Gly Gln
   1               5                   10                  15
   His Ser Phe Gly Trp Ile Gln Ile Gly
               20                  25
<210> 51
   <211> 19
   <212> PRT
   <213> Artificial Sequence
<220>
   <223> Adnectin
<400> 51
   Leu Ala Tyr His Ile Gln Tyr Trp Pro Leu Gly Trp Tyr Gln Arg Tyr
   1               5                   10                  15
   Gln Ile Phe
<210> 52
   <211> 25
   <212> PRT
   <213> Artificial Sequence
<220>
   <223> Adnectin
<400> 52
   Glu Tyr Gln Ile Arg Val Tyr Ala Glu Thr Gly Glu Ser Gly Ala Gln
   1               5                   10                  15
   Gln Ser Phe Gly Trp Ile Gln Ile Gly
               20                  25
<210> 53
   <211> 19
   <212> PRT
   <213> Artificial Sequence
<220>
   <223> Adnectin
<400> 53
   Gln Ser Tyr His Ile Gln Tyr Trp Pro Leu Gly Ala Tyr Gln Leu Tyr
   1               5                   10                  15
   Gln Leu Phe
<210> 54
   <211> 25
   <212> PRT
   <213> Artificial Sequence
<220>
   <223> Adnectin
<400> 54
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 55
   <211> 19
   <212> PRT
   <213> Artificial Sequence
<220>
   <223> Adnectin
<400> 55
   His Ala Tyr His Ile Gln Tyr Trp Pro Leu Gly Phe Tyr Gln Gly Tyr
   1               5                   10                  15
   Gln Val Phe
<210> 56
   <211> 25
   <212> PRT
   <213> Artificial Sequence
<220>
   <223> Adnectin
<400> 56
   Glu Tyr Gln Ile Arg Val Tyr Ala Asp Thr Gly Arg Gly Tyr Gln Leu
   1               5                   10                  15
   Ser Tyr Ser Trp Ile Gln Ile Gly Tyr
               20                  25
<210> 57
   <211> 19
   <212> PRT
   <213> Artificial Sequence
<220>
   <223> Adnectin
<400> 57
   Phe Arg Tyr His Ile Gln Tyr Trp Pro Leu Gly Gly Tyr Glu Arg Tyr
   1 5 10 15
   Gln Val Phe
<210> 58
   <211> 25
   <212> PRT
   <213> Artificial Sequence
<220>
   <223> Adnectin
<400> 58
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 59
   <211> 19
   <212> PRT
   <213> Artificial Sequence
<220>
   <223> Adnectin
<400> 59
   His Ser Tyr His Ile Gln Tyr Trp Pro Leu Gly Ser Tyr His Leu Tyr
   1 5 10 15
   Gln Leu Phe
<210> 60
   <211> 25
   <212> PRT
   <213> Artificial Sequence
<220>
   <223> Adnectin
<400> 60
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 61
   <211> 19
   <212> PRT
   <213> Artificial Sequence
<220>
   <223> Adnectin
<400> 61
   His Ser Tyr His Ile Gln Tyr Trp Pro Leu Gly Trp Tyr Gln Leu Tyr
   1               5                   10                  15
   Gln Val Phe
<210> 62
   <211> 25
   <212> PRT
   <213> Artificial Sequence
<220>
   <223> Adnectin
<400> 62
   Glu Tyr Gln Ile Arg Val Tyr Ala Glu Thr Gly Gly Phe Gly Ser Pro
   1               5                   10                  15
   Pro Asn Phe Gly Trp Ile Gln Ile Gly
               20                  25
<210> 63
   <211> 19
   <212> PRT
   <213> Artificial Sequence
<220>
   <223> Adnectin
<400> 63
   Gln Phe Tyr His Ile Gln Tyr Trp Pro Leu Gly Ser Tyr Gln Arg Tyr
   1               5                   10                  15
   Gln Val Phe
<210> 64
   <211> 25
   <212> PRT
   <213> Artificial Sequence
<220>
   <223> Adnectin
<400> 64
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 65
   <211> 19
   <212> PRT
   <213> Artificial Sequence
<220>
   <223> Adnectin
<400> 65
   Asn Ser Tyr His Ile Gln Tyr Trp Pro Leu Gly Trp Tyr His Arg Tyr
   1 5 10 15
   Gln Val Phe
<210> 66
   <211> 25
   <212> PRT
   <213> Artificial Sequence
<220>
   <223> Adnectin
<400> 66
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 67
   <211> 19
   <212> PRT
   <213> Artificial Sequence
<220>
   <223> Adnectin
<400> 67
   His Ser Tyr His Ile Gln Tyr Trp Pro Leu Gly Arg Tyr Gln Leu Tyr
   1 5 10 15
   Gln Val Phe
<210> 68
   <211> 25
   <212> PRT
   <213> Artificial Sequence
<220>
   <223> Adnectin
<400> 68
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 69
   <211> 19
   <212> PRT
   <213> Artificial Sequence
<220>
   <223> Adnectin
<400> 69
   Leu Ala Tyr His Ile Gln Tyr Trp Pro Leu Gly Trp Tyr His Leu Tyr
   1               5                   10                  15
   Gln Ile Phe
<210> 70
   <211> 25
   <212> PRT
   <213> Artificial Sequence
<220>
   <223> Adnectin
<400> 70
   Glu Tyr Gln Ile Arg Val Tyr Ala Glu Thr Gly Gly Val Gly Trp His
   1               5                   10                  15
   His Ser Phe Gly Trp Ile Gln Ile Gly
               20                  25
<210> 71
   <211> 19
   <212> PRT
   <213> Artificial Sequence
<220>
   <223> Adnectin
<400> 71
   His Val Tyr His Ile Gln Tyr Trp Pro Leu Gly Trp Tyr Pro Arg Tyr
   1 5 10 15
   Gln Val Phe
<210> 72
   <211> 25
   <212> PRT
   <213> Artificial Sequence
<220>
   <223> Adnectin
<400> 72
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 73
   <211> 19
   <212> PRT
   <213> Artificial Sequence
<220>
   <223> Adnectin
<400> 73
   His Ser Tyr His Ile Pro Tyr Trp Glu Leu Ala Trp Tyr Gln Arg Tyr
   1 5 10 15
   Gln Val Phe
<210> 74
   <211> 25
   <212> PRT
   <213> Artificial Sequence
<220>
   <223> Adnectin
<400> 74
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 75
   <211> 19
   <212> PRT
   <213> Artificial Sequence
<220>
   <223> Adnectin
<400> 75
   Glu Ser Tyr His Ile Gln Tyr Trp Pro Leu Gly Leu Tyr His Arg Tyr
   1               5                   10                  15
   Gln Val Phe
<210> 76
   <211> 25
   <212> PRT
   <213> Artificial Sequence
<220>
   <223> Adnectin
<400> 76
   Glu Tyr Gln Ile Arg Val Tyr Ala Glu Thr Gly Arg Gly Glu Ser Pro
   1               5                   10                  15
   Ala Ser Phe Gly Trp Ile Gln Ile Gly
               20                  25
<210> 77
   <211> 19
   <212> PRT
   <213> Artificial Sequence
<220>
   <223> Adnectin
<400> 77
   Leu Ala Tyr His Ile Gln Tyr Trp Pro Leu Gly Trp Tyr Gln Ala Tyr
   1               5                   10                  15
   Gln Val Phe
<210> 78
   <211> 25
   <212> PRT
   <213> Artificial Sequence
<220>
   <223> Adnectin
<400> 78
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 79
   <211> 19
   <212> PRT
   <213> Artificial Sequence
<220>
   <223> Adnectin
<400> 79
   Tyr Leu Tyr His Ile Gln Tyr Trp Pro Leu Gly Trp Tyr His Arg Tyr
   1 5 10 15
   Gln Val Phe
<210> 80
   <211> 25
   <212> PRT
   <213> Artificial Sequence
<220>
   <223> Adnectin
<400> 80
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 81
   <211> 19
   <212> PRT
   <213> Artificial Sequence
<220>
   <223> Adnectin
<400> 81
   Arg Phe Tyr His Ile Gln Tyr Trp Pro Leu Gly Trp Tyr His Cys Tyr
   1 5 10 15
   Gln Val Phe
<210> 82
   <211> 25
   <212> PRT
   <213> Artificial Sequence
<220>
   <223> Adnectin
<400> 82
   Glu Tyr Gln Ile Arg Val Tyr Ala Gln Thr Gly Asp Gly Ser Ser Gln
   1               5                   10                  15
   Glu Tyr Phe Gly Trp Ile Gln Ile Gly
               20                  25
<210> 83
   <211> 19
   <212> PRT
   <213> Artificial Sequence
<220>
   <223> Adnectin
<400> 83
   His Ser Tyr His Ile Gln Tyr Trp Pro Leu Gly Trp Tyr Tyr Arg Tyr
   1               5                   10                  15
   Gln Val Phe
<210> 84
   <211> 25
   <212> PRT
   <213> Artificial Sequence
<220>
   <223> Adnectin
<400> 84
   Glu Tyr Gln Ile Arg Val Tyr Ala Glu Thr Gly Gly Ser Gly Ser Gln
   1               5                   10                  15
   Gln Tyr Trp Gly Trp Ile Gln Ile Gly
               20                  25
<210> 85
   <211> 19
   <212> PRT
   <213> Artificial Sequence
<220>
   <223> Adnectin
<400> 85
   His Ala Tyr His Ile Gln Tyr Trp Pro Leu Gly Phe Tyr Gln Gly Tyr
   1 5 10 15
   Gln Val Phe
<210> 86
   <211> 25
   <212> PRT
   <213> Artificial Sequence
<220>
   <223> Adnectin
<400> 86
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 87
   <211> 19
   <212> PRT
   <213> Artificial Sequence
<220>
   <223> Adnectin
<400> 87
   His Ser Tyr His Ile Gln Tyr Trp Pro Leu Gly Leu Tyr Val Leu Tyr
   1 5 10 15
   Gln Val Phe
<210> 88
   <211> 25
   <212> PRT
   <213> Artificial Sequence
<220>
   <223> Adnectin
<400> 88
   Glu Tyr Gln Ile Arg Val Tyr Ala Glu Thr Gly Ala Gly Gly Ser Glu
   1 5 10 15
   His Ser Phe Gly Trp Ile Gln Ile Gly
   20 25
<210> 89
   <211> 19
   <212> PRT
   <213> Artificial Sequence
<220>
   <223> Adnectin
<400> 89
   Leu Ser Tyr His Ile Gln Tyr Trp Pro Leu Gly Arg Tyr Glu Arg Tyr
   1               5                   10                  15
   Gln Val Phe
<210> 90
   <211> 25
   <212> PRT
   <213> Artificial Sequence
<220>
   <223> Adnectin
<400> 90
   Glu Tyr Gln Ile Arg Val Tyr Ala Glu Thr Val Gly Gly Glu Ser Leu
   1               5                   10                  15
   Asp Ser Phe Ser Trp Ile Gln Ile Gly
               20                  25
<210> 91
   <211> 19
   <212> PRT
   <213> Artificial Sequence
<220>
   <223> Adnectin
<400> 91
   Leu Ser Tyr His Ile Gln Tyr Trp Pro Leu Gly Trp Tyr Gln Leu Tyr
   1               5                   10                  15
   Gln Val Phe
<210> 92
   <211> 25
   <212> PRT
   <213> Artificial Sequence
<220>
   <223> Adnectin
<400> 92
   Glu Tyr Gln Ile Arg Val Tyr Ala Glu Thr Arg Val Gly Gly Ser Val
   1 5 10 15
   Ala Ser Phe Gly Trp Ile Gln Ile Gly
   20 25
<210> 93
   <211> 19
   <212> PRT
   <213> Artificial Sequence
<220>
   <223> Adnectin
<400> 93
   Leu Ala Tyr His Ile Gln Tyr Trp Pro Leu Gly Arg Tyr Gln Leu Tyr
   1 5 10 15
   Gln Val Phe
<210> 94
   <211> 25
   <212> PRT
   <213> Artificial Sequence
<220>
   <223> Adnectin
<400> 94
   Glu Tyr Gln Ile Arg Val Tyr Ala Glu Thr Gly Arg Gly Glu Ser Pro
   1 5 10 15
   Ala Ser Phe Gly Trp Ile Gln Ile Gly
   20 25
<210> 95
   <211> 111
   <212> PRT
   <213> Artificial Sequence
<220>
   <223> Adnectin
<400> 95
   Met Ala Ser Thr Ser Gly Val Ser Asp Val Pro Arg Asp Leu Glu Val
   1               5                   10                  15
   Val Ala Ala Thr Pro Thr Ser Leu Leu Ile Ser Trp Asp Ala Pro Ala
               20                  25                  30
   Val Thr Val His Ser Tyr His Ile Gln Tyr Trp Pro Leu Gly Trp Tyr
           35                  40                  45
   Gln Arg Tyr Gln Val Phe Ser Val Pro Gly Ser Lys Ser Thr Ala Thr
       50                  55                  60
   Ile Ser Gly Leu Lys Pro Glu Val Glu Tyr Gln Ile Arg Val Tyr Ala
   65                  70                  75                  80
   Glu Thr Gly Gly Gly Gly Ser Gln Gln Ser Phe Gly Trp Ile Gln Ile
                   85                  90                  95
   Gly Tyr Arg Thr Glu Gly Ser Gly Ser His His His His His His
               100                 105                 110
<210> 96
   <211> 109
   <212> PRT
   <213> Artificial Sequence
<220>
   <223> Adnectin
<400> 96
   Met Gly Val Ser Asp Val Pro Arg Asp Leu Glu Val Val Ala Ala Thr
   1               5                   10                  15
   Pro Thr Ser Leu Leu Ile Ser Trp Asp Ala Pro Ala Val Thr Val Gln
               20                  25                  30
   Ser Tyr His Ile Gln Tyr Trp Pro Leu Gly Ser Tyr Gln Arg Tyr Gln
           35                  40                  45
   Val Phe Ser Val Pro Gly Ser Lys Ser Thr Ala Thr Ile Ser Gly Leu
       50                  55                  60
   Lys Pro Gly Val Glu Tyr Gln Ile Arg Val Tyr Ala Glu Thr Gly Gly
   65                  70                  75                  80
   Ala Asp Ser Asp Gln Ser Met Gly Trp Ile Gln Ile Gly Tyr Arg Thr
                   85                  90                  95
   Glu Gly Asp Lys Pro Ser Gln His His His His His His
               100                 105
<210> 97
   <211> 109
   <212> PRT
   <213> Artificial Sequence
<220>
   <223> Adnectin
<400> 97
   Met Gly Val Ser Asp Val Pro Arg Asp Leu Glu Val Val Ala Ala Thr
   1               5                   10                  15
   Pro Thr Ser Leu Leu Ile Ser Trp Asp Ala Pro Ala Val Thr Val His
               20                  25                  30
   Ser Tyr His Ile Gln Tyr Trp Pro Leu Gly Trp Tyr Gln Arg Tyr Gln
           35                  40                  45
   Val Phe Ser Val Pro Gly Ser Lys Ser Thr Ala Thr Ile Ser Gly Leu
       50                  55                  60
   Lys Pro Gly Val Glu Tyr Gln Ile Arg Val Tyr Ala Glu Thr Arg Ser
   65                  70                  75                  80
   Gly Leu Ala Asp Glu Ser Phe Gly Trp Ile Gln Ile Gly Tyr Arg Thr
                   85                  90                  95
   Glu Gly Asp Lys Pro Ser Gln His His His His His His
               100                 105
<210> 98
   <211> 109
   <212> PRT
   <213> Artificial Sequence
<220>
   <223> Adnectin
<400> 98
   Met Gly Val Ser Asp Val Pro Arg Asp Leu Glu Val Val Ala Ala Thr
   1               5                   10                  15
   Pro Thr Ser Leu Leu Ile Ser Trp Asp Ala Pro Ala Val Thr Val His
               20                  25                  30
   Ser Tyr His Ile Gln Tyr Trp Pro Leu Gly Ser Tyr Gln Arg Tyr Gln
           35                  40                  45
   Val Phe Ser Val Pro Gly Ser Lys Ser Thr Ala Thr Ile Ser Gly Leu
       50                  55                  60
   Lys Pro Gly Val Glu Tyr Gln Ile Arg Val Tyr Ala Glu Thr Gly Gly
   65                  70                  75                  80
   Ala Asp Ser Asp Gln Ser Met Gly Trp Ile Gln Ile Gly Tyr Arg Thr
   85 90 95
   Glu Gly Asp Lys Pro Ser Gln His His His His His His
   100 105
<210> 99
   <211> 107
   <212> PRT
   <213> Artificial Sequence
<220>
   <223> Adnectin
<400> 99
   Met Gly His His His His His His Gly Gly Val Ser Asp Val Pro Arg
   1 5 10 15
   Asp Leu Glu Val Val Ala Ala Thr Pro Thr Ser Leu Leu Ile Ser Trp
   20 25 30
   Asp Ala Pro Ala Val Thr Val His Ser Tyr His Ile Gln Tyr Trp Pro
   35 40 45
   Leu Gly Ser Tyr Gln Arg Tyr Gln Val Phe Ser Val Pro Gly Ser Lys
   50 55 60
   Ser Thr Ala Thr Ile Ser Gly Leu Lys Pro Gly Val Glu Tyr Gln Ile
   65 70 75 80
   Arg Val Tyr Ala Glu Thr Gly Gly Ala Asp Ser Asp Gln Ser Phe Gly
   85 90 95
   Trp Ile Gln Ile Gly Tyr Arg Thr Pro Glu Ser
   100 105
<210> 100
   <211> 115
   <212> PRT
   <213> Artificial Sequence
<220>
   <223> Adnectin
<400> 100
   Met Ala Ser Thr Ser Gly Ser Ser Ser Tyr Leu Met Pro Ser Asp Leu
   1 5 10 15
   Glu Val Val Ala Ala Thr Pro Thr Ser Leu Tyr Ile His Trp Tyr Pro
   20 25 30
   Ile Ala Ser Thr Ile Ile Asn Phe Arg Ile Thr Tyr Gly Glu Thr Gly
   35 40 45
   Gly Asn Ser Pro Val Gln Glu Phe Thr Val Pro Gly Ser Gln Val His
   50 55 60
   Ala Thr Ile Ser Gly Leu Lys Pro Gly Val Asp Tyr Thr Ile Thr Val
   65 70 75 80
   Tyr Ala Val His Tyr Glu His Lys Tyr Ser Glu Leu Trp Met Gly His
   85 90 95
   Pro Ile Ser Ile Asn Tyr Arg Thr Glu Gly Ser Gly Ser His His His
   100 105 110
   His His His
   115
<210> 101
   <211> 115
   <212> PRT
   <213> Artificial Sequence
<220>
   <223> Adnectin
<400> 101
   Met Ala Ser Thr Ser Gly Ser Ala Ser Tyr Leu Ile Pro Ser Asp Leu
   1               5                   10                  15
   Glu Val Val Ala Ala Thr Pro Thr Ser Leu Ser Ile Tyr Trp Tyr Pro
               20                  25                  30
   Val Ala Ser Thr Ile Ile Asn Phe Arg Ile Thr Tyr Val Glu Thr Gly
           35                  40                  45
   Gly Asn Ser Pro Val Gln Glu Phe Thr Val Pro Gly Ser Lys Ser Thr
       50                  55                  60
   Ala Thr Ile Ser Gly Leu Lys Pro Gly Val Asp Tyr Thr Ile Thr Val
   65                  70                  75                  80
   Tyr Ala Val His Tyr Glu Gln Lys Tyr Ser Glu Tyr Trp Ile Gly His
                   85                  90                  95
   Pro Ile Ser Ile Asn Tyr Arg Thr Glu Gly Ser Gly Ser His His His
               100                 105                 110
   His His His
       115
<210> 102
   <211> 115
   <212> PRT
   <213> Artificial Sequence
<220>
   <223> Adnectin
<400> 102
   Met Ala Ser Thr Ser Gly Ser Ser Pro Tyr Leu Met Pro Tyr Asp Leu
   1               5                   10                  15
   Glu Val Val Ala Ala Thr Pro Thr Ser Leu Phe Ile Arg Trp Tyr Gly
               20                  25                  30
   Ser Ala Ser Ser Ile Val Lys Phe Arg Ile Thr Tyr Gly Glu Thr Gly
           35                  40                  45
   Gly Asn Ser Pro Val Gln Glu Phe Thr Val Gly Gly Thr Gln Leu His
       50                  55                  60
   Ala Thr Ile Ser Gly Leu Lys Pro Gly Val Asp Tyr Thr Ile Thr Val
   65                  70                  75                  80
   Tyr Ala Val His Phe Glu His Lys Tyr Ser Glu Leu Trp Ile Gly His
                   85                  90                  95
   Pro Ile Ser Ile Asn Tyr Arg Thr Glu Gly Ser Gly Ser His His His
               100                 105                 110
   His His His
           115
<210> 103
   <211> 115
   <212> PRT
   <213> Artificial Sequence
<220>
   <223> Adnectin
<400> 103
   Met Ala Ser Thr Ser Gly Tyr Thr Ser Tyr Pro Ile Pro Tyr Asp Leu
   1               5                   10                  15
   Glu Val Val Ala Ala Thr Pro Thr Ser Leu Tyr Ile His Trp Tyr Trp
               20                  25                  30
   Ile Ala Ala Thr Ile Ile Ser Phe Arg Ile Thr Tyr Gly Glu Thr Gly
           35                  40                  45
   Gly Asn Ser Pro Val Gln Glu Phe Thr Val Pro Ala Gly Gln Asp His
       50                  55                  60
   Ala Thr Ile Ser Gly Leu Lys Pro Gly Val Asp Tyr Thr Ile Thr Val
   65                  70                  75                  80
   Tyr Ala Val His Tyr Glu Glu Glu Tyr Ser Glu Phe Trp Thr Gly His
                   85                  90                  95
   Pro Ile Ser Ile Asn Tyr Arg Thr Glu Gly Ser Gly Ser His His His
               100                 105                 110
   His His His
           115
<210> 104
   <211> 116
   <212> PRT
   <213> Artificial Sequence
<220>
   <223> Adnectin
<400> 104
   Met Ala Ser Thr Ser Gly Thr His Trp Phe Tyr Ser Ile Pro His Asp
   1               5                   10                  15
   Leu Glu Val Val Ala Ala Thr Pro Thr Ser Leu Thr Ile Ala Trp Glu
               20                  25                  30
   Pro Pro His His Thr Ala Met Gly Tyr Arg Ile Thr Tyr Gly Glu Thr
           35                  40                  45
   Gly Gly Asn Ser Pro Val Gln Glu Phe Thr Val Pro Gly Gly Tyr Thr
       50                  55                  60
   Thr Ala Tyr Ile Ser Gly Leu Lys Pro Gly Val Asp Tyr Thr Ile Thr
   65                  70                  75                  80
   Val Tyr Ala Ala Tyr Tyr Glu Arg Glu Tyr Ser Glu His Trp Ile Ser
                   85                  90                  95
   His Pro Ile Ser Ile Asn Tyr Arg Thr Glu Gly Ser Gly Ser His His
               100                 105                 110
   His His His His
       115
<210> 105
   <211> 118
   <212> PRT
   <213> Artificial Sequence
<220>
   <223> Adnectin
<400> 105
   Met Ala Ser Thr Ser Gly Glu Phe Tyr His Thr Lys Tyr Pro Tyr Asp
   1               5                   10                  15
   Leu Glu Val Val Ala Ala Thr Pro Thr Ser Leu Glu Ile Ser Trp Arg
               20                  25                  30
   Ser Pro Thr Arg Asp Trp Gln Trp Phe Arg Ile Thr Tyr Gly Glu Thr
           35                  40                  45
   Gly Gly Asn Ser Pro Val Gln Glu Phe Thr Val Ala Gly Pro Tyr Arg
       50                  55                  60
   Asn Ala Ile Ile Ser Gly Leu Lys Pro Gly Val Asp Tyr Thr Ile Thr
   65                  70                  75                  80
   Val Tyr Ala Asp Val Tyr Met Pro Ser Glu Gly Gly Leu Val Val Asp
                   85                  90                  95
   Thr Tyr His Pro Ile Ser Ile Asn Tyr Arg Thr Glu Gly Ser Gly Ser
               100                 105                 110
   His His His His His His
           115
<210> 106
   <211> 116
   <212> PRT
   <213> Artificial Sequence
<220>
   <223> Adnectin
<400> 106
   Met Ala Ser Thr Ser Gly Gln Ala Tyr Pro Glu Tyr Tyr Phe Val Asp
   1               5                   10                  15
   Leu Glu Val Val Ala Ala Thr Pro Thr Ser Leu Leu Ile Ser Trp Ser
               20                  25                  30
   Lys Pro Tyr Tyr Asn Ala Tyr Ser Tyr Arg Ile Thr Tyr Gly Glu Thr
   35 40 45
   Gly Gly Asn Ser Pro Val Gln Glu Phe Thr Val Leu Gly His Asp Thr
   50 55 60
   Arg Ala Val Ile Ser Gly Leu Lys Pro Gly Val Asp Tyr Thr Ile Thr
   65 70 75 80
   Val Tyr Ala Met Phe Ile Glu Tyr Ile Asp Gln Glu Ile Trp His Ala
   85 90 95
   His Pro Ile Ser Ile Asn Tyr Arg Thr Glu Gly Ser Gly Ser His His
   100 105 110
   His His His His
   115
<210> 107
   <211> 109
   <212> PRT
   <213> Artificial Sequence
<220>
   <223> Adnectin
<400> 107
   Met Gly Val Ser Asp Val Pro Arg Asp Leu Glu Val Val Ala Ala Thr
   1 5 10 15
   Pro Thr Ser Leu Leu Ile Ser Trp Asp Glu His Thr Asp Ile Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ala Met Glu His Thr Ala Thr Ile Ser Gly Leu
   50 55 60
   Lys Pro Gly Val Asp Tyr Thr Ile Thr Val Tyr Ala Val Thr His Val
   65 70 75 80
   Tyr Pro Ile Met Ile His Gln Tyr Pro Ile Ser Ile Asn Tyr Arg Thr
   85 90 95
   Glu Ile Asp Lys Pro Ser Gln His His His His His His
   100 105
<210> 108
   <211> 111
   <212> PRT
   <213> Artificial Sequence
<220>
   <223> Adnectin
<400> 108
   Met Ala Ser Thr Ser Gly Val Ser Asp Val Pro Arg Asp Leu Glu Val
   1               5                   10                  15
   Val Ala Ala Thr Pro Thr Ser Leu Leu Ile Ser Trp Asp Ala Pro Ala
               20                  25                  30
   Val Thr Val Leu Glu Tyr Gln Ile Asp Tyr His Pro Ala Ala Val Trp
           35                  40                  45
   His Ala Leu Gln Arg Phe Thr Val Pro Gly Ser Lys Ser Thr Ala Thr
       50                  55                  60
   Ile Ser Gly Leu Lys Pro Gly Val His Tyr Lys Ile Ser Val Thr Ala
   65                  70                  75                  80
   Thr Thr His Ala Asp Asn Glu Ser Ile Met Trp His Pro Ile Ser Ile
                   85                  90                  95
   Tyr Tyr Arg Thr Glu Gly Ser Gly Ser His His His His His His
               100                 105                 110
<210> 109
   <211> 109
   <212> PRT
   <213> Artificial Sequence
<220>
   <223> Adnectin
<400> 109
   Met Gly Val Ser Asp Val Pro Arg Asp Leu Glu Val Val Ala Ala Thr
   1               5                   10                  15
   Pro Thr Ser Leu Leu Ile Ser Trp Asp Tyr Pro Thr Val Thr Pro Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Glu Tyr Ile Gly Thr Ala Thr Ile Ser Gly Leu
       50                  55                  60
   Lys Pro Gly Val Asp Tyr Thr Ile Thr Val Tyr Ala Val Thr Asn Asp
   65                  70                  75                  80
   Thr Thr Ile Tyr Ser Ile Ser Arg Pro Ile Ser Ile Asn Tyr Arg Thr
                   85                  90                  95
   Glu Ile Asp Lys Pro Ser Gln His His His His His His
               100                 105
<210> 110
   <211> 107
   <212> PRT
   <213> Artificial Sequence
<220>
   <223> Adnectin
<400> 110
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Asp Ala Pro Ala Val Thr Val His Ser Tyr His Ile Gln Tyr Trp Pro
           35                  40                  45
   Leu Gly Ser Tyr Gln Arg Tyr Gln Val Phe Ser Val Pro Gly Ser Lys
       50                  55                  60
   Ser Thr Ala Thr Ile Ser Gly Leu Lys Pro Gly Val Glu Tyr Gln Ile
   65                  70                  75                  80
   Arg Val Tyr Ala Glu Thr Gly Arg Gly Glu Ser Asp Gln Ser Leu Gly
                   85                  90                  95
   Trp Ile Gln Ile Gly Tyr Arg Thr Glu Glu Ser
               100                 105
<210> 111
   <211> 107
   <212> PRT
   <213> Artificial Sequence
<220>
   <223> Adnectin
<400> 111
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
   20 25 30
   Asp Ala Pro Ala Val Thr Val His Ser Tyr His Ile Gln Tyr Trp Pro
   35 40 45
   Leu Gly Ser Tyr Gln Arg Tyr Gln Val Phe Ser Val Pro Gly Ser Lys
   50 55 60
   Ser Thr Ala Thr Ile Ser Gly Leu Lys Pro Gly Val Glu Tyr Gln Ile
   65 70 75 80
   Arg Val Tyr Ala Glu Thr Gly Arg Gly Glu Ser Asp Gln Ser Phe Gly
   85 90 95
   Trp Ile Gln Ile Gly Tyr Arg Thr Glu Glu Ser
   100 105
<210> 112
   <211> 107
   <212> PRT
   <213> Artificial Sequence
<220>
   <223> Adnectin
<400> 112
   Met Gly His His His His His His Gly Gly Val Ser Asp Val Pro Arg
   1 5 10 15
   Asp Leu Glu Val Val Ala Ala Thr Pro Thr Ser Leu Leu Ile Ser Trp
   20 25 30
   Asp Ala Pro Ala Val Thr Val His Ser Tyr His Ile Gln Tyr Trp Pro
   35 40 45
   Leu Gly Ser Tyr Gln Arg Tyr Gln Val Phe Ser Val Pro Gly Ser Lys
   50 55 60
   Ser Thr Ala Thr Ile Ser Gly Leu Lys Pro Gly Val Glu Tyr Gln Ile
   65 70 75 80
   Arg Val Tyr Ala Glu Thr Gly Arg Gly Glu Ser Asp Gln Ser Leu Gly
   85 90 95
   Trp Ile Gln Ile Gly Tyr Arg Thr Pro Glu Ser
   100 105
<210> 113
   <211> 107
   <212> PRT
   <213> Artificial Sequence
<220>
   <223> Adnectin
<400> 113
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Asp Ala Pro Ala Val Thr Val His Ser Tyr His Ile Gln Tyr Trp Pro
           35                  40                  45
   Leu Gly Ser Tyr Gln Arg Tyr Gln Val Phe Ser Val Pro Gly Ser Lys
       50                  55                  60
   Ser Thr Ala Thr Ile Ser Gly Leu Lys Pro Gly Val Glu Tyr Gln Ile
   65                  70                  75                  80
   Arg Val Tyr Ala Glu Thr Gly Arg Gly Glu Ser Asp Gln Ser Phe Gly
                   85                  90                  95
   Trp Ile Gln Ile Gly Tyr Arg Thr Pro Glu Ser
               100                 105
<210> 114
   <211> 107
   <212> PRT
   <213> Artificial Sequence
<220>
   <223> Adnectin
<400> 114
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Asp Ala Pro Ala Val Thr Val His Ala Tyr His Ile Gln Tyr Trp Pro
           35                  40                  45
   Leu Gly Phe Tyr Gln Gly Tyr Gln Val Phe Ser Val Pro Gly Ser Lys
       50                  55                  60
   Ser Thr Ala Thr Ile Ser Gly Leu Lys Pro Gly Val Glu Tyr Gln Ile
   65                  70                  75                  80
   Arg Val Tyr Ala Glu Thr Gly Leu Gly Asp Ala His Gln Ser Leu Gly
                   85                  90                  95
   Trp Ile Gln Ile Gly Tyr Arg Thr Pro Glu Ser
               100                 105
<210> 115
   <211> 105
   <212> PRT
   <213> Artificial Sequence
<220>
   <223> Adnectin
<400> 115
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Asn Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val His Ser Ser Pro Ile Ser Ile Asn Tyr Arg Thr Glu Ile Asp Lys
                   85                  90                  95
   Pro Ser Gln His His His His His His
               100                 105
<210> 116
   <211> 111
   <212> PRT
   <213> Artificial Sequence
<220>
   <223> Adnectin
<400> 116
   Met Ala Ser Thr Ser Gly Val Ser Asp Val Pro Arg Asp Leu Glu Val
   1               5                   10                  15
   Val Ala Ala Thr Pro Thr Ser Leu Leu Ile Ser Trp Asp Ala Pro Ala
   20 25 30
   Val Thr Val Glu Gln Tyr Tyr Ile Ala Tyr Tyr Val Glu Gly Glu Pro
   35 40 45
   Ser Ser Tyr Gln Tyr Phe Arg Val Pro Gly Ser Lys Ser Thr Ala Thr
   50 55 60
   Ile Ser Gly Leu Lys Pro Gly Val Leu Tyr His Ile Tyr Val Asn Ala
   65 70 75 80
   Val Thr Gly Ser Gly Leu Arg Pro Glu Phe Ser Leu Pro Ile Arg Ile
   85 90 95
   Lys Tyr Arg Thr Glu Gly Ser Gly Ser His His His His His His
   100 105 110
<210> 117
   <211> 104
   <212> PRT
   <213> Artificial Sequence
<220>
   <223> Adnectin
<400> 117
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 118
   <211> 105
   <212> PRT
   <213> Artificial Sequence
<220>
   <223> Adnectin
<400> 118
   Met Gly Val Ser Asp Val Pro Arg Asp Leu Glu Val Val Ala Ala Thr
   1               5                   10                  15
   Pro Thr Ser Leu Leu Ile Ser Trp Lys Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Leu Pro Ile Ser Ile Asn Tyr Arg Thr Glu Ile Asp Lys
                   85                  90                  95
   Pro Ser Gln His His His His His His
               100                 105
<210> 119
   <211> 107
   <212> PRT
   <213> Artificial Sequence
<220>
   <223> Adnectin
<400> 119
   Met Gly Val Ser Asp Val Pro Arg Asp Leu Glu Val Val Ala Ala Thr
   1               5                   10                  15
   Pro Thr Ser Leu Leu Ile Ser Trp Asp Ala Pro Ala Val Thr Val Gly
               20                  25                  30
   Trp Tyr His Ile Gly Tyr Asn Val Glu Gly Glu Pro Ala Ser Tyr Gln
           35                  40                  45
   Tyr Phe Arg Val Pro Gly Ser Lys Ser Thr Ala Thr Ile Ser Gly Leu
       50                  55                  60
   Lys Pro Gly Val Glu Tyr Met Ile Phe Val Asn Ala Val Thr Gly Ser
   65                  70                  75                  80
   Gly Ala Arg Glu Glu Phe Ser Leu Pro Ile Ser Ile Asn Tyr Arg Thr
                   85                  90                  95
   Glu Gly Ser Gly Ser His His His His His His
               100                 105
<210> 120
   <211> 103
   <212> PRT
   <213> Artificial Sequence
<220>
   <223> Adnectin
<400> 120
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Glu Tyr Asn Val Asn Pro Tyr Arg Tyr Tyr Arg Ile Thr Tyr Gly Glu
           35                  40                  45
   Thr Gly Gly Asn Ser Pro Val Gln Glu Phe Thr Val Pro Ser Val Leu
       50                  55                  60
   Ser Ser Ala Gln Ile Ser Gly Leu Lys Pro Gly Val Asp Tyr Thr Ile
   65                  70                  75                  80
   Thr Val Tyr Ala Val Thr Arg Gly Val Asp Ser Ala Pro Ile Ser Ile
                   85                  90                  95
   Asn Tyr Arg Thr Pro Gly Gly
               100
<210> 121
   <211> 103
   <212> PRT
   <213> Artificial Sequence
<220>
   <223> Adnectin
<400> 121
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Glu Tyr Asn Val Asn Pro Tyr Arg Tyr Tyr Arg Ile Thr Tyr Gly Glu
           35                  40                  45
   Thr Gly Gly Asn Ser Pro Val Gln Glu Phe Thr Val Pro Ser Val Leu
       50                  55                  60
   Ser Thr Ala Glu Ile Ser Gly Leu Lys Pro Gly Val Asp Tyr Thr Ile
   65                  70                  75                  80
   Thr Val Tyr Ala Val Thr Tyr Gly Val Asp Ser Asp Pro Ile Ser Ile
                   85                  90                  95
   Asn Tyr Arg Thr Pro Gly Gly
               100
<210> 122
   <211> 103
   <212> PRT
   <213> Artificial Sequence
<220>
   <223> Adnectin
<400> 122
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Gln Tyr Lys Val His Pro Tyr Arg Tyr Tyr Arg Ile Thr Tyr Gly Glu
           35                  40                  45
   Thr Gly Gly Asn Ser Pro Val Gln Glu Phe Thr Val Pro Ser Val Leu
       50                  55                  60
   Ser Thr Ala Glu Ile Ser Gly Leu Lys Pro Gly Val Asp Tyr Thr Ile
   65                  70                  75                  80
   Thr Val Tyr Ala Val Thr Arg Gly Val Asp Ser Ala Pro Ile Ser Ile
                   85                  90                  95
   Asn Tyr Arg Thr Pro Gly Gly
               100
<210> 123
   <211> 105
   <212> PRT
   <213> Artificial Sequence
<220>
   <223> Adnectin
<400> 123
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Arg Gly
   65                  70                  75                  80
   Val Asp Ser Ala Pro Ile Ser Ile Asn Tyr Arg Thr Glu Ile Asp Lys
                   85                  90                  95
   Pro Ser Gln His His His His His His
               100                 105
<210> 124
   <211> 105
   <212> PRT
   <213> Artificial Sequence
<220>
   <223> Adnectin
<400> 124
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Glu Ile Ser Gly Leu
   50 55 60
   Lys Pro Gly Val Asp Tyr Thr Ile Thr Val Tyr Ala Val Thr Arg Gly
   65 70 75 80
   Val Asp Ser Ala Pro Ile Ser Ile Asn Tyr Arg Thr Pro Ile Asp Lys
   85 90 95
   Pro Ser Gln His His His His His His
   100 105
<210> 125
   <211> 103
   <212> PRT
   <213> Artificial Sequence
<220>
   <223> Adnectin
<400> 125
   Met Gly His His His His His His Gly Gly Val Ser Asp Val Pro Arg
   1 5 10 15
   Asp Leu Glu Val Val Ala Ala Thr Pro Thr Ser Leu Leu Ile Ser Trp
   20 25 30
   Glu Tyr Lys Val His Pro Tyr Arg Tyr Tyr Arg Ile Thr Tyr Gly Glu
   35 40 45
   Thr Gly Gly Asn Ser Pro Val Gln Glu Phe Thr Val Pro Ser Val Leu
   50 55 60
   Ser Thr Ala Thr Ile Ser Gly Leu Lys Pro Gly Val Asp Tyr Thr Ile
   65 70 75 80
   Thr Val Tyr Ala Val Thr Tyr Gly Val Gln Ser Asp Pro Ile Ser Ile
   85 90 95
   Asn Tyr Arg Thr Pro Gly Gly
   100
<210> 126
   <211> 105
   <212> PRT
   <213> Artificial Sequence
<220>
   <223> Adnectin
<400> 126
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Gln Ser Pro Pro Ile Ser Ile Asn Tyr Arg Thr Glu Ile Asp Lys
                   85                  90                  95
   Pro Ser Gln His His His His His His
               100                 105
<210> 127
   <211> 103
   <212> PRT
   <213> Artificial Sequence
<220>
   <223> Adnectin
<400> 127
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Gln Tyr Lys Val His Pro Tyr Arg Tyr Tyr Arg Ile Thr Tyr Gly Glu
           35                  40                  45
   Thr Gly Gly Asn Ser Pro Val Gln Glu Phe Thr Val Pro Ser Val Leu
       50                  55                  60
   Ser Ser Ala Gln Ile Ser Gly Leu Lys Pro Gly Val Asp Tyr Thr Ile
   65                  70                  75                  80
   Thr Val Tyr Ala Val Thr Tyr Gly Ile Glu Ser Ser Pro Ile Ser Ile
                   85                  90                  95
   Asn Tyr Arg Thr Pro Gly Gly
           100
<210> 128
   <211> 103
   <212> PRT
   <213> Artificial Sequence
<220>
   <223> Adnectin
<400> 128
   Met Gly His His His His His His Gly Gly Val Ser Asp Val Pro Arg
   1               5                   10                  15
   Asp Leu Glu Val Val Ala Ala Thr Pro Thr Ser Leu Leu Ile Ser Trp
               20                  25                  30
   Glu Tyr Lys Val His Pro Tyr Arg Tyr Tyr Arg Ile Thr Tyr Gly Glu
           35                  40                  45
   Thr Gly Gly Asn Ser Pro Val Gln Glu Phe Thr Val Pro Ser Val Leu
       50                  55                  60
   Ser Ser Ala Glu Ile Ser Gly Leu Lys Pro Gly Val Asp Tyr Thr Ile
   65                  70                  75                  80
   Thr Val Tyr Ala Val Thr Tyr Gly Ile Asp Ser Ser Pro Ile Ser Ile
                   85                  90                  95
   Asn Tyr Arg Thr Pro Gly Gly
               100
<210> 129
   <211> 105
   <212> PRT
   <213> Artificial Sequence
<220>
   <223> Adnectin
<400> 129
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Ser Ala Glu Ile Ser Gly Leu
       50                  55                  60
   Lys Pro Gly Val Asp Tyr Thr Ile Thr Val Tyr Ala Val Thr Tyr Gly
   65                  70                  75                  80
   Ile Asp Ser Pro Pro Ile Ser Ile Asn Tyr Arg Thr Glu Ile Asp Lys
                   85                  90                  95
   Pro Ser Gln His His His His His His
               100                 105
<210> 130
   <211> 104
   <212> PRT
   <213> Artificial Sequence
<220>
   <223> Adnectin
<400> 130
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Asn Val His Tyr Asp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Asp Gly Val His Ser Ser Pro Ile Ser
                   85                  90                  95
   Ile Asn Tyr Arg Thr Glu Ile Asp
               100
<210> 131
   <211> 104
   <212> PRT
   <213> Artificial Sequence
<220>
   <223> Adnectin
<400> 131
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Leu Ser Ala Pro Ile Ser
                   85                  90                  95
   Ile Asn Tyr Arg Thr Glu Ile Asp
               100
<210> 132
   <211> 108
   <212> PRT
   <213> Artificial Sequence
<220>
   <223> Adnectin
<400> 132
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Asp Ala Pro Ala Val Thr Val Glu Glu Tyr Tyr Ile Gly Tyr Tyr
           35                  40                  45
   Val Glu Phe Glu Pro Ser Ser Tyr Gln Trp Phe Thr Val Pro Gly Ser
       50                  55                  60
   Lys Ser Thr Ala Thr Ile Ser Gly Leu Lys Pro Gly Val Glu Tyr Ser
   65                  70                  75                  80
   Ile Tyr Val Asn Ala Val Thr Gly Met Gly Met Gln Pro Glu Met Ser
                   85                  90                  95
   Leu Pro Ile Ser Ile Asn Tyr Arg Thr Glu Gly Ser
               100                 105
<210> 133
   <211> 104
   <212> PRT
   <213> Artificial Sequence
<220>
   <223> Adnectin
<400> 133
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Tyr Asp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val Asp Ser Asp Pro Ile Ser
   85 90 95
   Ile Asn Tyr Arg Thr Glu Ile Asp
   100
<210> 134
   <211> 108
   <212> PRT
   <213> Artificial Sequence
<220>
   <223> Adnectin
<400> 134
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Asp Ala Pro Ala Val Thr Val Glu Gln Tyr Tyr Ile Ala Tyr Tyr
   35 40 45
   Asp Glu Lys Glu Pro Ser Ser Tyr Gln Tyr Phe Arg Val Pro Gly Ser
   50 55 60
   Lys Ser Thr Ala Thr Ile Ser Gly Leu Lys Pro Gly Val Glu Tyr Ala
   65 70 75 80
   Ile Phe Val Asn Ala Val Thr Arg Ser Gly Val Leu Pro Glu Phe Ser
   85 90 95
   Leu Pro Ile Ser Ile Asn Tyr Arg Thr Glu Gly Ser
   100 105
<210> 135
   <211> 104
   <212> PRT
   <213> Artificial Sequence
<220>
   <223> Adnectin
<400> 135
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Lys Tyr Asn Val Asn Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val His Ser Ser Pro Ile Ser
   85 90 95
   Ile Asn Tyr Arg Thr Glu Ile Asp
   100
<210> 136
   <211> 108
   <212> PRT
   <213> Artificial Sequence
<220>
   <223> Adnectin
<400> 136
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Asp Ala Pro Ala Val Thr Val Glu Gln Tyr Tyr Ile Gly Tyr Tyr
           35                  40                  45
   Val Glu Ala Glu Pro Ser Ser Tyr Gln Tyr Phe Phe Val Pro Gly Ser
       50                  55                  60
   Lys Ser Thr Ala Thr Ile Ser Gly Leu Lys Pro Gly Val Asp Tyr Ala
   65                  70                  75                  80
   Ile Phe Val Asn Ala Val Thr Ala Ser Gly Arg Gly Pro Glu Tyr Ser
                   85                  90                  95
   Leu Pro Ile Ser Ile Asn Tyr Arg Thr Glu Gly Ser
               100                 105
<210> 137
   <211> 109
   <212> PRT
   <213> Artificial Sequence
<220>
   <223> Adnectin
<400> 137
   Met Gly Val Ser Asp Val Pro Arg Asp Leu Glu Val Val Ala Ala Thr
   1               5                   10                  15
   Pro Thr Ser Leu Leu Ile Ser Trp His Asp Gly Ile Gly Glu Glu Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Met Asp Asp Ile Thr Ala Thr Ile Ser Gly Leu
       50                  55                  60
   Lys Pro Gly Val Asp Tyr Thr Ile Thr Val Tyr Ala Val Thr Val Gly
   65                  70                  75                  80
   Asp Val Ile Ser Val Leu His Glu Pro Ile Ser Ile Asn Tyr Arg Thr
                   85                  90                  95
   Glu Ile Asp Lys Pro Ser Gln His His His His His His
               100                 105
<210> 138
   <211> 108
   <212> PRT
   <213> Artificial Sequence
<220>
   <223> Adnectin
<400> 138
   Met Gly Val Ser Asp Val Pro Arg Asp Leu Glu Val Val Ala Ala Thr
   1 5 10 15
   Pro Thr Ser Leu Leu Ile Ser Trp His Tyr Pro Phe Glu Gly Tyr Val
   20 25 30
   Thr Tyr Tyr Arg Ile Thr Tyr Gly Glu Thr Gly Gly Asn Ser His Val
   35 40 45
   Gln Glu Phe Thr Val Pro Val Gly Tyr Thr Thr Ala Thr Ile Ser Gly
   50 55 60
   Leu Lys Pro Gly Val Asp Tyr Thr Ile Thr Val Tyr Ala Val Thr Ser
   65 70 75 80
   Ser Lys Gly Tyr Val Tyr Phe Pro Ile Ser Ile Asn Tyr Arg Thr Glu
   85 90 95
   Ile Asp Lys Pro Ser Gln His His His His His His
   100 105
<210> 139
   <211> 109
   <212> PRT
   <213> Artificial Sequence
<220>
   <223> Adnectin
<400> 139
   Met Gly Val Ser Asp Val Pro Arg Asp Leu Glu Val Val Ala Ala Thr
   1 5 10 15
   Pro Thr Ser Leu Leu Ile Ser Trp Glu Asp Pro Glu Ala Ala Val Arg
   20 25 30
   Tyr Tyr Arg Ile Thr Tyr Gly Glu Thr Gly Gly Asn Ser Pro Val Gln
   35 40 45
   Glu Phe Thr Val Pro Ile Asn Asp Leu His Ser Tyr Leu Ser Gly Leu
   50 55 60
   Lys Pro Gly Val Asp Tyr Thr Ile Thr Val Tyr Ala Val Thr Glu Ala
   65 70 75 80
   Thr Val Met Tyr Val Leu Asp Glu Pro Ile Ser Ile Asn Tyr Arg Thr
   85 90 95
   Glu Ile Asp Lys Pro Ser Gln His His His His His His
   100 105
<210> 140
   <211> 109
   <212> **PRT**
   <213> **Artificial** Sequence
<220>
   <223> Adnectin
<400> **140**
   Met Gly Val Ser Asp Val Pro Arg Asp Leu Glu Val Val Ala Ala Thr
   1 5 10 15
   Pro Thr Ser Leu Leu Ile Ser Trp Asp Leu Leu Glu Asp Met Ser Arg
   20 25 30
   Tyr Tyr Arg Ile Thr Tyr Gly Glu Thr Gly Gly Asn Ser Pro Val Gln
   35 40 45
   Glu Phe Thr Val Pro Thr Asp Ala Tyr Thr Ala Thr Ile Ser Gly Leu
   50 55 60
   Lys Pro Gly Val Asp Tyr Thr Ile Thr Val Tyr Ala Val Thr Gln Asp
   65 70 75 80
   Ser His Val Ile Glu Leu Ser Tyr Pro Ile Ser Ile Asn Tyr Arg Thr
   85 90 95
   Glu Ile Asp Lys Pro Ser Gln His His His His His His
   100 105
<210> 141
   <211> 105
   <212> PRT
   <213> Artificial Sequence
<220>
   <223> Adnectin
<400> **141**
   Met Gly Val Ser Asp Val Pro Arg Asp Leu Glu Val Val Ala Ala Thr
   1               5                   10                  15
   Pro Thr Ser Leu Leu Ile Ser Trp Gln Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Gln Ser Pro Pro Ile Ser Ile Asn Tyr Arg Thr Glu Ile Asp Lys
                   85                  90                  95
   Pro Ser Gln His His His His His His
               100                 105
<210> 142
   <211> 105
   <212> **PRT**
   <213> **Artificial** Sequence
<220>
   <223> Adnectin
<400> 142
   Met Gly Val Ser Asp Val Pro Arg Asp Leu Glu Val Val Ala Ala Thr
   1               5                   10                  15
   Pro Thr Ser Leu Leu Ile Ser Trp Arg Tyr Arg Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Asp Gly
   65                  70                  75                  80
   Val Gln Ser Ser Pro Ile Ser Ile Asn Tyr Arg Thr Glu Ile Asp Lys
                   85                  90                  95
   Pro Ser Gln His His His His His His
               100                 105
<210> 143
   <211> 105
   <212> PRT
   <213> Artificial Sequence
<220>
   <223> Adnectin
<400> 143
   Met Gly Val Ser Asp Val Pro Arg Asp Leu Glu Val Val Ala Ala Thr
   1 5 10 15
   Pro Thr Ser Leu Leu Ile Ser Trp Glu Tyr Asn Val Asn Pro Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Ile Glu Ser Ser Pro Ile Ser Ile Asn Tyr Arg Thr Glu Ile Asp Lys
   85 90 95
   Pro Ser Gln His His His His His His
   100 105
<210> 144
   <211> 105
   <212> PRT
   <213> Artificial Sequence
<220>
   <223> Adnectin
<400> 144
   Met Gly Val Ser Asp Val Pro Arg Asp Leu Glu Val Val Ala Ala Thr
   1 5 10 15
   Pro Thr Ser Leu Leu Ile Ser Trp Gln Tyr Lys Val His Pro Tyr Arg
   20 25 30
   Tyr Tyr Arg Ile Thr Tyr Gly Glu Thr Gly Gly Asn Ser Pro Val Gln
               35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Glu Ser Ser Pro Ile Ser Ile Asn Tyr Arg Thr Glu Ile Asp Lys
                   85                  90                  95
   Pro Ser Gln His His His His His His
               100                 105
<210> 145
   <211> 105
   <212> PRT
   <213> Artificial Sequence
<220>
   <223> Adnectin
<400> 145
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Leu Pro Ile Ser Ile Asn Tyr Arg Thr Glu Ile Asp Lys
                   85                  90                  95
   Pro Ser Gln His His His His His His
               100                 105
<210> 146
   <211> 105
   <212> PRT
   <213> Artificial Sequence
<220>
   <223> Adnectin
<400> 146
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Asp Ser Pro Pro Ile Ser Ile Asn Tyr Arg Thr Glu Ile Asp Lys
                   85                  90                  95
   Pro Ser Gln His His His His His His
               100                 105
<210> 147
   <211> 105
   <212> **PRT**
   <213> **Artificial** Sequence
<220>
   <223> Adnectin
<400> 147
   Met Gly Val Ser Asp Val Pro Arg Asp Leu Glu Val Val Ala Ala Thr
   1 5 10 15
   Pro Thr Ser Leu Leu Ile Ser Trp Glu Tyr Lys Val His Pro Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Val Asp Ser Asp Pro Ile Ser Ile Asn Tyr Arg Thr Glu Ile Asp Lys
   85 90 95
   Pro Ser Gln His His His His His His
   100 105
<210> 148
   <211> 105
   <212> PRT
   <213> Artificial Sequence
<220>
   <223> Adnectin
<400> 148
   Met Gly Val Ser Asp Val Pro Arg Asp Leu Glu Val Val Ala Ala Thr
   1 5 10 15
   Pro Thr Ser Leu Leu Ile Ser Trp Glu Tyr Asn Val Asn Pro Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Ile Asp Ser Pro Pro Ile Ser Ile Asn Tyr Arg Thr Glu Ile Asp Lys
   85 90 95
   Pro Ser Gln His His His His His His
   100 105
<210> 149
   <211> 104
   <212> PRT
   <213> Artificial Sequence
<220>
   <223> Adnectin
<400> 149
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Lys Asp Ser Ala Pro Ile Ser
   85 90 95
   Ile Asn Tyr Arg Thr Glu Ile Asp
   100
<210> 150
   <211> 105
   <212> PRT
   <213> Artificial Sequence
<220>
   <223> Adnectin
<400> 150
   Met Gly Val Ser Asp Val Pro Arg Asp Leu Glu Val Val Ala Ala Thr
   1 5 10 15
   Pro Thr Ser Leu Leu Ile Ser Trp Glu Tyr Asn Val Asn Pro Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Val Glu Ser Ser Pro Ile Ser Ile Asn Tyr Arg Thr Glu Ile Asp Lys
   85 90 95
   Pro Ser Gln His His His His His His
   100 105
<210> 151
   <211> 105
   <212> PRT
   <213> Artificial Sequence
<220>
   <223> Adnectin
<400> 151
   Met Gly Val Ser Asp Val Pro Arg Asp Leu Glu Val Val Ala Ala Thr
   1 5 10 15
   Pro Thr Ser Leu Leu Ile Ser Trp Glu Tyr Lys Val His Pro Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Ile Asp Ser Ser Pro Ile Ser Ile Asn Tyr Arg Thr Glu Ile Asp Lys
   85 90 95
   Pro Ser Gln His His His His His His
   100 105
<210> 152
   <211> 105
   <212> PRT
   <213> Artificial Sequence
<220>
   <223> Adnectin
<400> 152
   Met Gly Val Ser Asp Val Pro Arg Asp Leu Glu Val Val Ala Ala Thr
   1 5 10 15
   Pro Thr Ser Leu Leu Ile Ser Trp Glu Tyr Asn Val Asn Pro Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Val Gln Ser Asp Pro Ile Ser Ile Asn Tyr Arg Thr Glu Ile Asp Lys
   85 90 95
   Pro Ser Gln His His His His His His
   100 105
<210> 153
   <211> 104
   <212> PRT
   <213> Artificial Sequence
<220>
   <223> Adnectin
<400> 153
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Gln Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 154
   <211> 105
   <212> PRT
   <213> Artificial Sequence
<220>
   <223> Adnectin
<400> 154
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Asn Val Asn Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Leu Pro Ile Ser Ile Asn Tyr Arg Thr Glu Ile Asp Lys
                   85                  90                  95
   Pro Ser Gln His His His His His His
               100                 105
<210> 155
   <211> 105
   <212> PRT
   <213> Artificial Sequence
<220>
   <223> Adnectin
<400> 155
   Met Gly Val Ser Asp Val Pro Arg Asp Leu Glu Val Val Ala Ala Thr
   1               5                   10                  15
   Pro Thr Ser Leu Leu Ile Ser Trp Gln Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr His Gly
   65                  70                  75                  80
   Val His Ser Ala Pro Ile Ser Ile Asn Tyr Arg Thr Glu Ile Asp Lys
                   85                  90                  95
   Pro Ser Gln His His His His His His
               100                 105
<210> 156
   <211> 105
   <212> PRT
   <213> Artificial Sequence
<220>
   <223> Adnectin
<400> 156
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Pro Trp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Glu Ser Ser Pro Ile Ser Ile Asn Tyr Arg Thr Glu Ile Asp Lys
                   85                  90                  95
   Pro Ser Gln His His His His His His
               100                 105
<210> 157
   <211> 105
   <212> PRT
   <213> Artificial Sequence
<220>
   <223> Adnectin
<400> 157
   Met Gly Val Ser Asp Val Pro Arg Asp Leu Glu Val Val Ala Ala Thr
   1               5                   10                  15
   Pro Thr Ser Leu Leu Ile Ser Trp Gln Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Ile Pro Ile Ser Ile Asn Tyr Arg Thr Glu Ile Asp Lys
                   85                  90                  95
   Pro Ser Gln His His His His His His
               100                 105
<210> 158
   <211> 105
   <212> PRT
   <213> Artificial Sequence
<220>
   <223> Adnectin
<400> 158
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Tyr Asp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Gln Ser Pro Pro Ile Ser Ile Asn Tyr Arg Thr Glu Ile Asp Lys
                   85                  90                  95
   Pro Ser Gln His His His His His His
               100                 105
<210> 159
   <211> 104
   <212> PRT
   <213> Artificial Sequence
<220>
   <223> Adnectin
<400> 159
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Pro Tyr Arg Thr Pro Gly Gly
               100
<210> 160
   <211> 105
   <212> PRT
   <213> Artificial Sequence
<220>
   <223> Adnectin
<400> 160
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asp Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Glu Ser Ser Pro Ile Ser Ile Asn Tyr Arg Thr Glu Ile Asp Lys
                   85                  90                  95
   Pro Ser Gln His His His His His His
               100                 105
<210> 161
   <211> 104
   <212> PRT
   <213> Artificial Sequence
<220>
   <223> Adnectin
<400> 161
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Glu Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 162
   <211> 104
   <212> PRT
   <213> Artificial Sequence
<220>
   <223> Adnectin
<400> 162
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Gln Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 163
   <211> 104
   <212> PRT
   <213> Artificial Sequence
<220>
   <223> Adnectin
<400> 163
   Met Gly His His His His His His Gly Gly Ser Val Ser Asp Val Pro
   1 5 10 15
   Arg Tyr Leu Glu Val Val Ala Ala Thr Pro Thr Ser Leu Leu Ile Ser
   20 25 30
   Trp Gln Tyr Lys Val His Pro Tyr Arg Trp Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val Asn Ser Ile Pro Ile Ser
   85 90 95
   Ile Asn Tyr Arg Thr Glu Ile Asp
   100
<210> 164
   <211> 104
   <212> PRT
   <213> Artificial Sequence
<220>
   <223> Adnectin
<400> 164
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Lys Tyr Gln Val His Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val Tyr Ser Ala Pro Ile Ser
   85 90 95
   Ile Asn Tyr Arg Thr Glu Ile Asp
   100
<210> 165
   <211> 104
   <212> PRT
   <213> Artificial Sequence
<220>
   <223> Adnectin
<400> 165
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asn Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val His Ser Ser Pro Ile Ser
   85 90 95
   Ile Asn Tyr Arg Thr Glu Ile Asp
   100
<210> 166
   <211> 104
   <212> PRT
   <213> Artificial Sequence
<220>
   <223> Adnectin
<400> 166
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Lys Tyr Asn Leu His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ile Ile Ser Glu Pro Ile Ser
                   85                  90                  95
   Ile Asn Tyr Arg Thr Glu Ile Asp
               100
<210> 167
   <211> 104
   <212> PRT
   <213> Artificial Sequence
<220>
   <223> Adnectin
<400> 167
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Arg Val Asn Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val Gln Ser Pro Pro Ile Ser
                   85                  90                  95
   Ile Asn Tyr Arg Thr Glu Ile Asp
               100
<210> 168
   <211> 104
   <212> PRT
   <213> Artificial Sequence
<220>
   <223> Adnectin
<400> 168
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val Leu Ser Pro Pro Ile Ser
                   85                  90                  95
   Ile Asn Tyr Arg Thr Glu Ile Asp
               100
<210> 169
   <211> 104
   <212> PRT
   <213> Artificial Sequence
<220>
   <223> Adnectin
<400> 169
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Asn Val Asn Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Arg Gly Val Asp Ser Ala Pro Ile Ser
   85 90 95
   Ile Asn Tyr Arg Thr Glu Ile Asp
   100
<210> 170
   <211> 104
   <212> PRT
   <213> Artificial Sequence
<220>
   <223> Adnectin
<400> 170
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ser Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Phe Gly Ile Arg Ser Ser Pro Ile Ser
   85 90 95
   Ile Asn Tyr Arg Thr Glu Ile Asp
   100
<210> 171
   <211> 104
   <212> PRT
   <213> Artificial Sequence
<220>
   <223> Adnectin
<400> 171
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Lys Tyr Gln Val His Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ile Ile Ser Glu Pro Ile Ser
                   85                  90                  95
   Ile Asn Tyr Arg Thr Glu Ile Asp
               100
<210> 172
   <211> 104
   <212> PRT
   <213> Artificial Sequence
<220>
   <223> Adnectin
<400> 172
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ile Asp Ser Ser Pro Ile Ser
                   85                  90                  95
   Ile Asn Tyr Arg Thr Glu Ile Asp
               100
<210> 173
   <211> 104
   <212> PRT
   <213> Artificial Sequence
<220>
   <223> Adnectin
<400> 173
   Met Gly His His His His His His Gly Gly Ala Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Pro Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val His Ser Ser Pro Ile Ser
                   85                  90                  95
   Ile Asn Tyr Arg Thr Glu Ile Asp
               100
<210> 174
   <211> 104
   <212> PRT
   <213> Artificial Sequence
<220>
   <223> Adnectin
<400> 174
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Asn Val Asn Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Ile Ile Ser Glu Pro Ile Ser
   85 90 95
   Ile Asn Tyr Arg Thr Glu Ile Asp
   100
<210> 175
   <211> 104
   <212> PRT
   <213> Artificial Sequence
<220>
   <223> Adnectin
<400> 175
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Tyr Asp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val Gln Ser Thr Pro Ile Ser
   85 90 95
   Ile Asn Tyr Arg Thr Glu Ile Asp
   100
<210> 176
   <211> 104
   <212> PRT
   <213> Artificial Sequence
<220>
   <223> Adnectin
<400> 176
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His His Asp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ile Glu Ser Ser Pro Ile Ser
                   85                  90                  95
   Ile Asn Tyr Arg Thr Glu Ile Asp
               100
<210> 177
   <211> 104
   <212> PRT
   <213> Artificial Sequence
<220>
   <223> Adnectin
<400> 177
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Pro Tyr Arg Thr Pro Gly Gly
   100
<210> 178
   <211> 104
   <212> PRT
   <213> Artificial Sequence
<220>
   <223> Adnectin
<220>
   <221> misc_feature
   <222> (90)..(90)
   <223> Xaa can be any naturally occurring amino acid
<220>
   <221> misc_feature
   <222> (93)..(93)
   <223> Xaa can be any naturally occurring amino acid
<400> 178
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Xaa Asp Ser Xaa Pro Ile Ser
                   85                  90                  95
   Ile Asn Tyr Arg Thr Glu Ile Asp
               100
<210> 179
   <211> 104
   <212> PRT
   <213> Artificial Sequence
<220>
   <223> Adnectin
<400> 179
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Arg Pro Ile Ser
                   85                  90                  95
   Ile Asn Tyr Arg Thr Glu Ile Asp
               100
<210> 180
   <211> 104
   <212> PRT
   <213> Artificial Sequence
<220>
   <223> Adnectin
<400> 180
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Lys Asp Ser Ala Pro Ile Ser
   85 90 95
   Ile Asn Tyr Arg Thr Glu Ile Asp
   100
<210> 181
   <211> 104
   <212> PRT
   <213> Artificial Sequence
<220>
   <223> Adnectin
<400> 181
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp His Ser Ala Pro Ile Ser
   85 90 95
   Ile Asn Tyr Arg Thr Glu Ile Asp
   100
<210> 182
   <211> 104
   <212> PRT
   <213> Artificial Sequence
<220>
   <223> Adnectin
<400> 182
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Gln Ser Ala Pro Ile Ser
                   85                  90                  95
   Ile Asn Tyr Arg Thr Glu Ile Asp
               100
<210> 183
   <211> 104
   <212> PRT
   <213> Artificial Sequence
<220>
   <223> Adnectin
<400> 183
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ile Asp Ser Ala Pro Ile Ser
                   85                  90                  95
   Ile Asn Tyr Arg Thr Glu Ile Asp
               100
<210> 184
   <211> 104
   <212> PRT
   <213> Artificial Sequence
<220>
   <223> Adnectin
<400> 184
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Val Ser Ala Pro Ile Ser
                   85                  90                  95
   Ile Asn Tyr Arg Thr Glu Ile Asp
               100
<210> 185
   <211> 104
   <212> PRT
   <213> Artificial Sequence
<220>
   <223> Adnectin
<220>
   <221> misc_feature
   <222> (90)..(90)
   <223> Xaa can be any naturally occurring amino acid
<400> 185
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Xaa Asp Ser Ala Pro Ile Ser
                   85                  90                  95
   Ile Asn Tyr Arg Thr Glu Ile Asp
               100
<210> 186
   <211> 104
   <212> PRT
   <213> Artificial Sequence
<220>
   <223> Adnectin
<400> 186
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Glu Asp Ser Ala Pro Ile Ser
                   85                  90                  95
   Ile Asn Tyr Arg Thr Glu Ile Asp
               100
<210> 187
   <211> 104
   <212> PRT
   <213> Artificial Sequence
<220>
   <223> Adnectin
<400> 187
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Lys Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 188
   <211> 104
   <212> PRT
   <213> Artificial Sequence
<220>
   <223> Adnectin
<400> 188
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Leu Ser Ala Pro Ile Ser
   85 90 95
   Ile Asn Tyr Arg Thr Glu Ile Asp
   100
<210> 189
   <211> 104
   <212> PRT
   <213> Artificial Sequence
<220>
   <223> Adnectin
<400> 189
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Lys Asp Ser Ala Pro Ile Ser
   85 90 95
   Ile Asn Tyr Arg Thr Glu Ile Asp
   100
<210> 190
   <211> 104
   <212> PRT
   <213> Artificial Sequence
<220>
   <223> Adnectin
<400> 190
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Ala Asp Ser Ala Pro Ile Ser
   85 90 95
   Ile Asn Tyr Arg Thr Glu Ile Asp
   100
<210> 191
   <211> 104
   <212> PRT
   <213> Artificial Sequence
<220>
   <223> Adnectin
<400> 191
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Thr Asp Ser Ala Pro Ile Ser
   85 90 95
   Ile Asn Tyr Arg Thr Glu Ile Asp
   100
<210> 192
   <211> 104
   <212> PRT
   <213> Artificial Sequence
<220>
   <223> Adnectin
<400> 192
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Thr Asp Ser Ala Pro Ile Ser
                   85                  90                  95
   Ile Asn Tyr Arg Thr Glu Ile Asp
               100
<210> 193
   <211> 104
   <212> PRT
   <213> Artificial Sequence
<220>
   <223> Adnectin
<400> 193
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Lys Asp Ser Ala Pro Ile Ser
   85 90 95
   Ile Asn Tyr Arg Thr Glu Ile Asp
   100
<210> 194
   <211> 104
   <212> PRT
   <213> Artificial Sequence
<220>
   <223> Adnectin
<400> 194
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Pro Ser Ala Pro Ile Ser
   85 90 95
   Ile Asn Tyr Arg Thr Glu Ile Asp
   100
<210> 195
   <211> 104
   <212> PRT
   <213> Artificial Sequence
<220>
   <223> Adnectin
<400> 195
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Gln Pro Ile Ser
                   85                  90                  95
   Ile Asn Tyr Arg Thr Glu Ile Asp
               100
<210> 196
   <211> 104
   <212> PRT
   <213> Artificial Sequence
<220>
   <223> Adnectin
<400> 196
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Val Ser Ala Pro Ile Ser
                   85                  90                  95
   Ile Asn Tyr Arg Thr Glu Ile Asp
               100
<210> 197
   <211> 104
   <212> PRT
   <213> Artificial Sequence
<220>
   <223> Adnectin
<400> 197
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Gly Pro Ile Ser
                   85                  90                  95
   Ile Asn Tyr Arg Thr Glu Ile Asp
               100
<210> 198
   <211> 104
   <212> PRT
   <213> Artificial Sequence
<220>
   <223> Adnectin
<400> 198
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
   85 90 95
   Ile Gln Tyr Arg Thr Glu Ile Asp
   100
<210> 199
   <211> 104
   <212> PRT
   <213> Artificial Sequence
<220>
   <223> Adnectin
<400> 199
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Gln Pro Ile Ser
   85 90 95
   Ile Asn Tyr Arg Thr Glu Ile Asp
   100
<210> 200
   <211> 104
   <212> PRT
   <213> Artificial Sequence
<220>
   <223> Adnectin
<400> 200
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Thr
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 201
   <211> 104
   <212> PRT
   <213> Artificial Sequence
<220>
   <223> Adnectin
<400> 201
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Gln Ser Ala Pro Ile Ser
                   85                  90                  95
   Ile Asn Tyr Arg Thr Glu Ile Asp
               100
<210> 202
   <211> 104
   <212> PRT
   <213> Artificial Sequence
<220>
   <223> Adnectin
<400> 202
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Lys Pro Ile Ser
                   85                  90                  95
   Ile Asn Tyr Arg Thr Glu Ile Asp
               100
<210> 203
   <211> 104
   <212> PRT
   <213> Artificial Sequence
<220>
   <223> Adnectin
<400> 203
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Gly Pro Ile Ser
   85 90 95
   Ile Asn Tyr Arg Thr Glu Ile Asp
   100
<210> 204
   <211> 104
   <212> PRT
   <213> Artificial Sequence
<220>
   <223> Adnectin
<400> 204
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Arg
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 205
   <211> 104
   <212> PRT
   <213> Artificial Sequence
<220>
   <223> Adnectin
<400> 205
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Asp Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 206
   <211> 104
   <212> PRT
   <213> Artificial Sequence
<220>
   <223> Adnectin
<400> 206
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Lys Pro Ile Ser
   85 90 95
   Ile Asn Tyr Arg Thr Glu Ile Asp
   100
<210> 207
   <211> 104
   <212> PRT
   <213> Artificial Sequence
<220>
   <223> Adnectin
<400> 207
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Trp
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 208
   <211> 104
   <212> PRT
   <213> Artificial Sequence
<220>
   <223> Adnectin
<400> 208
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Thr Pro Ile Ser
                   85                  90                  95
   Ile Asn Tyr Arg Thr Glu Ile Asp
               100
<210> 209
   <211> 104
   <212> PRT
   <213> Artificial Sequence
<220>
   <223> Adnectin
<400> 209
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Thr Pro Ile Ser
                   85                  90                  95
   Ile Asn Tyr Arg Thr Glu Ile Asp
               100
<210> 210
   <211> 104
   <212> PRT
   <213> Artificial Sequence
<220>
   <223> Adnectin
<400> 210
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ser Pro Ile Ser
   85 90 95
   Ile Asn Tyr Arg Thr Glu Ile Asp
   100
<210> 211
   <211> 104
   <212> PRT
   <213> Artificial Sequence
<220>
   <223> Adnectin
<400> 211
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Arg Gly Asp Asp Ser Ala Pro Ile Ser
   85 90 95
   Ile Asn Tyr Arg Thr Glu Ile Asp
   100
<210> 212
   <211> 104
   <212> PRT
   <213> Artificial Sequence
<220>
   <223> Adnectin
<400> 212
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr His Gly Asp Asp Ser Ala Pro Ile Ser
                   85                  90                  95
   Ile Asn Tyr Arg Thr Glu Ile Asp
               100
<210> 213
   <211> 104
   <212> PRT
   <213> Artificial Sequence
<220>
   <223> Adnectin
<400> 213
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Trp Pro Ile Ser
   85 90 95
   Ile Asn Tyr Arg Thr Glu Ile Asp
   100
<210> 214
   <211> 104
   <212> PRT
   <213> Artificial Sequence
<220>
   <223> Adnectin
<400> 214
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr His Gly Asp Asp Ser Ala Pro Ile Ser
                   85                  90                  95
   Ile Asn Tyr Arg Thr Glu Ile Asp
               100
<210> 215
   <211> 104
   <212> PRT
   <213> Artificial Sequence
<220>
   <223> Adnectin
<400> 215
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Lys Tyr Asp Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 216
   <211> 104
   <212> PRT
   <213> Artificial Sequence
<220>
   <223> Adnectin
<400> 216
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Asp Leu Lys Pro Gly Val Asp Tyr Thr
   65 70 75 80
   Ile Thr Val Tyr Ala Val Thr Tyr Gly Asp Asp Ser His Pro Ile Ser
   85 90 95
   Ile Asn Tyr Arg Thr Glu Ile Asp
   100
<210> 217
   <211> 104
   <212> PRT
   <213> Artificial Sequence
<220>
   <223> Adnectin
<400> 217
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Arg Gly Asp Asp Ser Ala Pro Ile Ser
                   85                  90                  95
   Ile Asn Tyr Arg Thr Glu Ile Asp
               100
<210> 218
   <211> 104
   <212> PRT
   <213> Artificial Sequence
<220>
   <223> Adnectin
<400> 218
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Arg Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 219
   <211> 104
   <212> PRT
   <213> Artificial Sequence
<220>
   <223> Adnectin
<400> 219
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Lys Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 220
   <211> 104
   <212> PRT
   <213> Artificial Sequence
<220>
   <223> Adnectin
<400> 220
   Met Gly His His His His His His Cys Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val Ala His Asn Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 221
   <211> 104
   <212> PRT
   <213> Artificial Sequence
<220>
   <223> Adnectin
<400> 221
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Tyr Gly Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 222
   <211> 104
   <212> PRT
   <213> Artificial Sequence
<220>
   <223> Adnectin
<400> 222
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp His Gln Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 223
   <211> 104
   <212> PRT
   <213> Artificial Sequence
<220>
   <223> Adnectin
<400> 223
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Tyr Arg Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 224
   <211> 104
   <212> PRT
   <213> Artificial Sequence
<220>
   <223> Adnectin
<400> 224
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala Tyr Asp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 225
   <211> 104
   <212> PRT
   <213> Artificial Sequence
<220>
   <223> Adnectin
<400> 225
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Thr Ser Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 226
   <211> 103
   <212> PRT
   <213> Artificial Sequence
<220>
   <223> Adnectin
<400> 226
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Ile His Glu
           35                  40                  45
   Thr Gly Gly Asn Ser Pro Val Gln Glu Phe Thr Val Pro Ser Val Leu
       50                  55                  60
   Ser Thr Ala Thr Ile Ser Gly Leu Lys Pro Gly Val Asp Tyr Thr Ile
   65                  70                  75                  80
   Thr Val Tyr Ala Val Thr Tyr Gly Asp Asp Ser Ala Pro Ile Ser Ile
                   85                  90                  95
   Asn Tyr Arg Thr Glu Ile Asp
               100
<210> 227
   <211> 105
   <212> PRT
   <213> Artificial Sequence
<220>
   <223> Adnectin
<400> 227
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Lys Glu Ala Glu Leu
               100                 105
<210> 228
   <211> 104
   <212> PRT
   <213> Artificial Sequence
<220>
   <223> Adnectin
<400> 228
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn His Gln Arg Tyr Tyr Arg Ile Thr Tyr Gly
           35                  40                  45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 229
   <211> 104
   <212> PRT
   <213> Artificial Sequence
<220>
   <223> Adnectin
<400> 229
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asp His Arg Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 230
   <211> 104
   <212> PRT
   <213> Artificial Sequence
<220>
   <223> Adnectin
<400> 230
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asp Ser Asp Pro Ile Ser Ile Asn Tyr Arg Thr Asp Asp Lys Pro
                   85                  90                  95
   Ser Gln His His His His His His
               100
<210> 231
   <211> 105
   <212> PRT
   <213> Artificial Sequence
<220>
   <223> Adnectin
<400> 231
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Ile Pro Ile Ser Ile Asn Tyr Arg Thr Glu Ile Asp Lys
                   85                  90                  95
   Pro Ser Gln His His His His His His
                       100                 105
<210> 232
   <211> 105
   <212> PRT
   <213> Artificial Sequence
<220>
   <223> Adnectin
<400> 232
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Ala Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Glu Ser Ser Pro Ile Ser Ile Asn Tyr Arg Thr Glu Ile Asp Lys
                   85                  90                  95
   Pro Ser Gln His His His His His His
               100                 105
<210> 233
   <211> 105
   <212> PRT
   <213> Artificial Sequence
<220>
   <223> Adnectin
<400> 233
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Asp Gly
   65 70 75 80
   Val Gln Ser Ser Pro Ile Ser Ile Asn Tyr Arg Thr Glu Ile Asp Lys
   85 90 95
   Pro Ser Gln His His His His His His
   100 105
<210> 234
   <211> 105
   <212> PRT
   <213> Artificial Sequence
<220>
   <223> Adnectin
<400> 234
   Met Gly Val Ser Asp Val Pro Arg Asp Leu Glu Val Val Ala Ala Thr
   1 5 10 15
   Pro Thr Ser Leu Leu Ile Ser Trp Glu Tyr Lys Val Asn Ala Tyr Arg
   20 25 30
   Tyr Tyr Arg Ile Thr Tyr Gly Glu Thr Gly Gly Asn Ser Pro Val Gln
   35 40 45
   Glu Phe Thr Val Pro Ser Val Leu Ser Thr Ala Thr Ile Ser Gly Leu
   50 55 60
   Lys Pro Gly Val Asp Tyr Thr Ile Thr Val Tyr Ala Val Thr Tyr Gly
   65 70 75 80
   Ile Asp Ser Ser Pro Ile Ser Ile Asn Tyr Arg Thr Glu Ile Asp Lys
   85 90 95
   Pro Ser Gln His His His His His His
   100 105
<210> 235
   <211> 105
   <212> PRT
   <213> Artificial Sequence
<220>
   <223> Adnectin
<400> 235
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Tyr Asp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Asn Gly
   65                  70                  75                  80
   Val Leu Ser Ser Pro Ile Ser Ile Asn Tyr Arg Thr Glu Ile Asp Lys
                   85                  90                  95
   Pro Ser Gln His His His His His His
               100                 105
<210> 236
   <211> 105
   <212> PRT
   <213> Artificial Sequence
<220>
   <223> Adnectin
<400> 236
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Pro Trp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Arg Gly
   65                  70                  75                  80
   Val Asp Ser Ala Pro Ile Ser Ile Asn Tyr Arg Thr Glu Ile Asp Lys
                   85                  90                  95
   Pro Ser Gln His His His His His His
               100                 105
<210> 237
   <211> 105
   <212> PRT
   <213> Artificial Sequence
<220>
   <223> Adnectin
<220>
   <221> misc_feature
   <222> (31)..(31)
   <223> Xaa can be any naturally occurring amino acid
<220>
   <221> misc_feature
   <222> (84)..(84)
   <223> Xaa can be any naturally occurring amino acid
<400> 237
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val His Pro Xaa Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asx Ser Xaa Pro Ile Ser Ile Asn Tyr Arg Thr Glu Ile Asp Lys
                   85                  90                  95
   Pro Ser Gln His His His His His His
               100                 105
<210> 238
   <211> 105
   <212> PRT
   <213> Artificial Sequence
<220>
   <223> Adnectin
<400> 238
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Pro Trp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Ile Gln Ser Pro Pro Ile Ser Ile Asn Tyr Arg Thr Glu Ile Asp Lys
                   85                  90                  95
   Pro Ser Gln His His His His His His
               100                 105
<210> 239
   <211> 105
   <212> PRT
   <213> Artificial Sequence
<220>
   <223> Adnectin
<400> 239
   Met Gly Val Ser Asp Val Pro Arg Asp Leu Glu Val Val Ala Ala Thr
   1               5                   10                  15
   Pro Thr Ser Leu Leu Ile Ser Trp Gln Tyr Lys Val His Pro Tyr Arg
               20                  25                  30
   Trp Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Ile Pro Ile Ser Ile Asn Tyr Arg Thr Glu Ile Asp Lys
                   85                  90                  95
   Pro Ser Gln His His His His His His
                       100                 105
<210> 240
   <211> 105
   <212> PRT
   <213> Artificial Sequence
<220>
   <223> Adnectin
<400> 240
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Ser Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Ile Pro Ile Ser Ile Asn Tyr Arg Thr Glu Ile Asp Lys
                   85                  90                  95
   Pro Ser Gln His His His His His His
               100                 105
<210> 241
   <211> 105
   <212> PRT
   <213> Artificial Sequence
<220>
   <223> Adnectin
<400> 241
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Pro Trp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asn Ser Leu Pro Ile Ser Ile Asn Tyr Arg Thr Glu Ile Asp Lys
                   85                  90                  95
   Pro Ser Gln His His His His His His
               100                 105
<210> 242
   <211> 105
   <212> PRT
   <213> Artificial Sequence
<220>
   <223> Adnectin
<400> 242
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Asn Val His Tyr Asp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asp Ser Asp Pro Ile Ser Ile Asn Tyr Arg Thr Glu Ile Asp Lys
                   85                  90                  95
   Pro Ser Gln His His His His His His
               100                 105
<210> 243
   <211> 105
   <212> PRT
   <213> Artificial Sequence
<220>
   <223> Adnectin
<400> 243
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asn Pro Trp Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asp Ser Asp Pro Ile Ser Ile Asn Tyr Arg Thr Glu Ile Asp Lys
                   85                  90                  95
   Pro Ser Gln His His His His His His
               100                 105
<210> 244
   <211> 105
   <212> PRT
   <213> Artificial Sequence
<220>
   <223> Adnectin
<400> 244
   Met Gly Val Ser Asp Val Pro Arg Asp Leu Glu Val Val Ala Ala Thr
   1               5                   10                  15
   Pro Thr Ser Leu Leu Ile Ser Trp Glu Tyr Lys Val Asp Pro Tyr Arg
               20                  25                  30
   Tyr Tyr Arg Ile Thr Tyr Gly Glu Thr Gly Gly Asn Ser Pro Val Gln
           35                  40                  45
   Glu Phe Thr Val Pro Ser Val Leu Ser Thr Ala Thr Ile Ser Gly Leu
       50                  55                  60
   Lys Pro Gly Val Asp Tyr Thr Ile Thr Val Tyr Ala Val Thr Tyr Gly
   65                  70                  75                  80
   Val Asp Ser Asp Pro Ile Ser Ile Asn Tyr Arg Thr Glu Ile Asp Lys
                   85                  90                  95
   Pro Ser Gln His His His His His His
               100                 105
<210> 245
   <211> 104
   <212> PRT
   <213> Artificial Sequence
<220>
   <223> Adnectin
<400> 245
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Lys Tyr Gln Val His Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Asn Gly Leu Lys Pro Gly Val Asp Tyr Thr
   65 70 75 80
   Ile Thr Val Tyr Ala Val Thr Tyr Gly Ile Ile Ser Glu Pro Ile Ser
   85 90 95
   Ile Asn Tyr Arg Thr Glu Ile Asp
   100
<210> 246
   <211> 104
   <212> PRT
   <213> Artificial Sequence
<220>
   <223> Adnectin
<400> 246
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asn Tyr Asn Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Glu Gly Val Gln Ser Ala Pro Ile Ser
   85 90 95
   Ile Asn Tyr Arg Thr Glu Ile Asp
   100
<210> 247
   <211> 104
   <212> PRT
   <213> Artificial Sequence
<220>
   <223> Adnectin
<400> 247
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Pro Gly Gly
   100
<210> 248
   <211> 104
   <212> PRT
   <213> Artificial Sequence
<220>
   <223> Adnectin
<400> 248
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Thr Pro Val Gln Glu Phe Arg Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Ala Asp Ala Ser Ala Pro Ile Ser
                   85                  90                  95
   Ile Asn Tyr Arg Thr Glu Ile Asp
               100
<210> 249
   <211> 104
   <212> PRT
   <213> Artificial Sequence
<220>
   <223> Adnectin
<400> 249
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Thr Leu Asn Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 250
   <211> 104
   <212> PRT
   <213> Artificial Sequence
<220>
   <223> Adnectin
<400> 250
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Lys Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 251
   <211> 104
   <212> PRT
   <213> Artificial Sequence
<220>
   <223> Adnectin
<400> 251
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Asn Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 252
   <211> 104
   <212> PRT
   <213> Artificial Sequence
<220>
   <223> Adnectin
<400> 252
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ala His Arg Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 253
   <211> 104
   <212> PRT
   <213> Artificial Sequence
<220>
   <223> Adnectin
<400> 253
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 254
   <211> 104
   <212> PRT
   <213> Artificial Sequence
<220>
   <223> Adnectin
<400> 254
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Ser Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 255
   <211> 104
   <212> PRT
   <213> Artificial Sequence
<220>
   <223> Adnectin
<400> 255
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 256
   <211> 104
   <212> PRT
   <213> Artificial Sequence
<220>
   <223> Adnectin
<400> 256
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Ala Ser Ala Pro Ile Ser
   85 90 95
   Ile Asn Tyr Arg Thr Glu Ile Asp
   100
<210> 257
   <211> 104
   <212> PRT
   <213> Artificial Sequence
<220>
   <223> Adnectin
<400> 257
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Asn Gly Asp Asp Ser Ala Pro Ile Ser
   85 90 95
   Ile Asn Tyr Arg Thr Glu Ile Asp
   100
<210> 258
   <211> 104
   <212> PRT
   <213> Artificial Sequence
<220>
   <223> Adnectin
<400> 258
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Thr Ile Arg Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 259
   <211> 104
   <212> PRT
   <213> Artificial Sequence
<220>
   <223> Adnectin
<400> 259
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Lys Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 260
   <211> 104
   <212> PRT
   <213> Artificial Sequence
<220>
   <223> Adnectin
<400> 260
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Arg Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 261
   <211> 104
   <212> PRT
   <213> Artificial Sequence
<220>
   <223> Adnectin
<400> 261
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala Pro Trp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 262
   <211> 104
   <212> PRT
   <213> Artificial Sequence
<220>
   <223> Adnectin
<400> 262
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asp Gly Trp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 263
   <211> 104
   <212> PRT
   <213> Artificial Sequence
<220>
   <223> Adnectin
<400> 263
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Thr Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 264
   <211> 104
   <212> PRT
   <213> Artificial Sequence
<220>
   <223> Adnectin
<220>
   <221> misc_feature
   <222> (28)..(28)
   <223> Xaa can be any naturally occurring amino acid
<400> 264
   Met Gly His His His His His His Gly Gly Ser Val Ser Asp Val Pro
   1               5                   10                  15
   Arg Asp Leu Glu Val Val Ala Ala Thr Pro Thr Xaa Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 265
   <211> 104
   <212> PRT
   <213> Artificial Sequence
<220>
   <223> Adnectin
<400> 265
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Ala Asp Ser Ala Pro Ile Ser
   85 90 95
   Ile Asn Tyr Arg Thr Glu Ile Asp
   100
<210> 266
   <211> 104
   <212> PRT
   <213> Artificial Sequence
<220>
   <223> Adnectin
<400> 266
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Arg Gly Asp Asp Ser Ala Pro Ile Ser
   85 90 95
   Ile Asn Tyr Arg Thr Glu Ile Asp
   100
<210> 267
   <211> 104
   <212> PRT
   <213> Artificial Sequence
<220>
   <223> Adnectin
<400> 267
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Asp Phe Thr Val Pro Ser Val
       50                  55                  60
   Leu Ser Thr Ala Thr Ile Arg Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 268
   <211> 104
   <212> PRT
   <213> Artificial Sequence
<220>
   <223> Adnectin
<400> 268
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Asn Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 269
   <211> 104
   <212> PRT
   <213> Artificial Sequence
<220>
   <223> Adnectin
<400> 269
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Ala Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 270
   <211> 104
   <212> PRT
   <213> Artificial Sequence
<220>
   <223> Adnectin
<400> 270
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ala Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 271
   <211> 104
   <212> PRT
   <213> Artificial Sequence
<220>
   <223> Adnectin
<400> 271
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Gly Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 272
   <211> 104
   <212> PRT
   <213> Artificial Sequence
<220>
   <223> Adnectin
<400> 272
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Thr Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
           100
<210> 273
   <211> 104
   <212> PRT
   <213> Artificial Sequence
<220>
   <223> Adnectin
<400> 273
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Glu Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 274
   <211> 104
   <212> PRT
   <213> Artificial Sequence
<220>
   <223> Adnectin
<400> 274
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Arg Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 275
   <211> 104
   <212> PRT
   <213> Artificial Sequence
<220>
   <223> Adnectin
<400> 275
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Ser Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ala Asp Ser Ala Pro Ile Ser
                   85                  90                  95
   Ile Asn Tyr Arg Thr Glu Ile Asp
               100
<210> 276
   <211> 104
   <212> PRT
   <213> Artificial Sequence
<220>
   <223> Adnectin
<400> 276
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Arg Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 277
   <211> 104
   <212> PRT
   <213> Artificial Sequence
<220>
   <223> Adnectin
<400> 277
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Asp Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 278
   <211> 104
   <212> PRT
   <213> Artificial Sequence
<220>
   <223> Adnectin
<400> 278
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ala Arg Trp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 279
   <211> 104
   <212> PRT
   <213> Artificial Sequence
<220>
   <223> Adnectin
<400> 279
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asp His Gln Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 280
   <211> 104
   <212> PRT
   <213> Artificial Sequence
<220>
   <223> Adnectin
<400> 280
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asp Tyr Gly Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 281
   <211> 104
   <212> PRT
   <213> Artificial Sequence
<220>
   <223> Adnectin
<400> 281
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Lys Tyr Gly Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 282
   <211> 104
   <212> PRT
   <213> Artificial Sequence
<220>
   <223> Adnectin
<400> 282
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Ser Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 283
   <211> 104
   <212> PRT
   <213> Artificial Sequence
<220>
   <223> Adnectin
<400> 283
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Asp Gly Asp Asp Ser Ala Pro Ile Ser
                   85                  90                  95
   Ile Asn Tyr Arg Thr Glu Ile Asp
               100
<210> 284
   <211> 104
   <212> PRT
   <213> Artificial Sequence
<220>
   <223> Adnectin
<400> 284
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Ser Gly Asp Asp Ser Ala Pro Ile Ser
   85 90 95
   Ile Asn Tyr Arg Thr Glu Ile Asp
   100
<210> 285
   <211> 104
   <212> PRT
   <213> Artificial Sequence
<220>
   <223> Adnectin
<400> 285
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Ser Ala Thr Leu Arg Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 286
   <211> 104
   <212> PRT
   <213> Artificial Sequence
<220>
   <223> Adnectin
<400> 286
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala Ala Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 287
   <211> 104
   <212> PRT
   <213> Artificial Sequence
<220>
   <223> Adnectin
<400> 287
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala Thr Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 288
   <211> 104
   <212> PRT
   <213> Artificial Sequence
<220>
   <223> Adnectin
<400> 288
   Met Gly His His His His His His Gly Gly Ser Val Ser Asp Val Pro
   1 5 10 15
   Arg Asp Leu Glu Val Val Ala Ala Thr Thr Thr Ser Leu Leu Ile Ser
   20 25 30
   Trp Glu Tyr Lys Val Asp Tyr His Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 289
   <211> 104
   <212> PRT
   <213> Artificial Sequence
<220>
   <223> Adnectin
<400> 289
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Gly Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 290
   <211> 104
   <212> PRT
   <213> Artificial Sequence
<220>
   <223> Adnectin
<220>
   <221> misc_feature
   <222> (36)..(36)
   <223> Xaa can be any naturally occurring amino acid
<400> 290
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Lys Tyr Xaa Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 291
   <211> 104
   <212> PRT
   <213> Artificial Sequence
<220>
   <223> Adnectin
<400> 291
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Gly Gly Asp Asp Ser Ala Pro Ile Ser
                   85                  90                  95
   Ile Asn Tyr Arg Thr Glu Ile Asp
               100
<210> 292
   <211> 104
   <212> PRT
   <213> Artificial Sequence
<220>
   <223> Adnectin
<400> 292
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile His Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 293
   <211> 104
   <212> PRT
   <213> Artificial Sequence
<220>
   <223> Adnectin
<400> 293
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Leu Arg Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 294
   <211> 104
   <212> PRT
   <213> Artificial Sequence
<220>
   <223> Adnectin
<400> 294
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Ser Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 295
   <211> 104
   <212> PRT
   <213> Artificial Sequence
<220>
   <223> Adnectin
<400> 295
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ala Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 296
   <211> 104
   <212> PRT
   <213> Artificial Sequence
<220>
   <223> Adnectin
<400> 296
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala Thr Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 297
   <211> 104
   <212> PRT
   <213> Artificial Sequence
<220>
   <223> Adnectin
<400> 297
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Pro Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 298
   <211> 104
   <212> PRT
   <213> Artificial Sequence
<220>
   <223> Adnectin
<400> 298
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Tyr Gln Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 299
   <211> 104
   <212> PRT
   <213> Artificial Sequence
<220>
   <223> Adnectin
<400> 299
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Lys Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 300
   <211> 104
   <212> PRT
   <213> Artificial Sequence
<220>
   <223> Adnectin
<220>
   <221> misc_feature
   <222> (36)..(36)
   <223> Xaa can be any naturally occurring amino acid
<400> 300
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Xaa Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 301
   <211> 105
   <212> PRT
   <213> Artificial Sequence
<220>
   <223> Adnectin
<400> 301
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr His Gly Asp Asp Phe Ala Pro Ile Ser
   85 90 95
   Ile Asn Tyr Arg Thr Glu Met Leu Ile
   100 105
<210> 302
   <211> 104
   <212> PRT
   <213> Artificial Sequence
<220>
   <223> Adnectin
<400> 302
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Thr Ile Asn Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 303
   <211> 104
   <212> PRT
   <213> Artificial Sequence
<220>
   <223> Adnectin
<400> 303
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Lys Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 304
   <211> 104
   <212> PRT
   <213> Artificial Sequence
<220>
   <223> Adnectin
<400> 304
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala His Asp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 305
   <211> 104
   <212> PRT
   <213> Artificial Sequence
<220>
   <223> Adnectin
<400> 305
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 306
   <211> 104
   <212> PRT
   <213> Artificial Sequence
<220>
   <223> Adnectin
<400> 306
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asp Tyr Arg Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 307
   <211> 104
   <212> PRT
   <213> Artificial Sequence
<220>
   <223> Adnectin
<400> 307
   Met Gly His His His His His His Gly Gly Ser Val Ser Asp Val Pro
   1               5                   10                  15
   Arg Asp Leu Glu Val Val Ala Ala Thr Pro Thr Gly Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 308
   <211> 104
   <212> PRT
   <213> Artificial Sequence
<220>
   <223> Adnectin
<400> 308
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 309
   <211> 104
   <212> PRT
   <213> Artificial Sequence
<220>
   <223> Adnectin
<400> 309
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr His Gly Asp Asp Ser Ala Pro Ile Ser
                   85                  90                  95
   Ile Asn Tyr Arg Thr Glu Ile Asp
               100
<210> 310
   <211> 104
   <212> PRT
   <213> Artificial Sequence
<220>
   <223> Adnectin
<400> 310
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Asn Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 311
   <211> 104
   <212> PRT
   <213> Artificial Sequence
<220>
   <223> Adnectin
<400> 311
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Glu Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 312
   <211> 104
   <212> PRT
   <213> Artificial Sequence
<220>
   <223> Adnectin
<400> 312
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Leu Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 313
   <211> 104
   <212> PRT
   <213> Artificial Sequence
<220>
   <223> Adnectin
<400> 313
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ala His Gly Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 314
   <211> 104
   <212> **PRT**
   <213> Artificial Sequence
<220>
   <223> Adnectin
<400> 314
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ala Tyr Lys Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 315
   <211> 104
   <212> PRT
   <213> Artificial Sequence
<220>
   <223> Adnectin
<400> 315
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asp Arg Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 316
   <211> 104
   <212> PRT
   <213> Artificial Sequence
<220>
   <223> Adnectin
<400> 316
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Ala Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 317
   <211> 104
   <212> PRT
   <213> Artificial Sequence
<220>
   <223> Adnectin
<400> 317
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val Asn Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 318
   <211> 104
   <212> PRT
   <213> Artificial Sequence
<220>
   <223> Adnectin
<400> 318
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asn Gly Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 319
   <211> 104
   <212> PRT
   <213> Artificial Sequence
<220>
   <223> Adnectin
<400> 319
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ser His Gly Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 320
   <211> 104
   <212> PRT
   <213> Artificial Sequence
<220>
   <223> Adnectin
<400> 320
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Thr Thr Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 321
   <211> 104
   <212> PRT
   <213> Artificial Sequence
<220>
   <223> Adnectin
<400> 321
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Glx Leu Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 322
   <211> 104
   <212> PRT
   <213> Artificial Sequence
<220>
   <223> Adnectin
<400> 322
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Thr Asp Ser Ala Pro Ile Ser
   85 90 95
   Ile Asn Tyr Arg Thr Glu Ile Asp
   100
<210> 323
   <211> 104
   <212> **PRT**
   <213> **Artificial** Sequence
<220>
   <223> Adnectin
<400> 323
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Gln Ser Ala Pro Ile Ser
   85 90 95
   Ile Asn Tyr Arg Thr Glu Ile Asp
   100
<210> 324
   <211> 104
   <212> **PRT**
   <213> **Artificial** Sequence
<220>
   <223> Adnectin
<400> 324
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Asn Pro Ile Ser
                   85                  90                  95
   Ile Asn Tyr Arg Thr Glu Ile Asp
               100
<210> 325
   <211> 104
   <212> PRT
   <213> Artificial Sequence
<220>
   <223> Adnectin
<400> 325
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn His Asp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 326
   <211> 104
   <212> PRT
   <213> Artificial Sequence
<220>
   <223> Adnectin
<400> 326
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ser His Arg Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 327
   <211> 104
   <212> PRT
   <213> Artificial Sequence
<220>
   <223> Adnectin
<400> 327
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val Thr Tyr Glu Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 328
   <211> 104
   <212> PRT
   <213> Artificial Sequence
<220>
   <223> Adnectin
<220>
   <221> misc_feature
   <222> (70)..(71)
   <223> Xaa can be any naturally occurring amino acid
<400> 328
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Ser Ala Thr Xaa Xaa Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 329
   <211> 104
   <212> PRT
   <213> Artificial Sequence
<220>
   <223> Adnectin
<400> 329
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Val Ser Ala Pro Ile Ser
   85 90 95
   Ile Asn Tyr Arg Thr Glu Ile Asp
   100
<210> 330
   <211> 104
   <212> **PRT**
   <213> **Artificial** Sequence
<220>
   <223> Adnectin
<400> 330
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Pro Pro Ile Ser
   85 90 95
   Ile Asn Tyr Arg Thr Glu Ile Asp
   100
<210> 331
   <211> 104
   <212> PRT
   <213> Artificial Sequence
<220>
   <223> Adnectin
<220>
   <221> misc_feature
   <222> (93)..(93)
   <223> Xaa can be any naturally occurring amino acid
<400> 331
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Xaa Pro Ile Ser
   85 90 95
   Ile Asn Tyr Arg Thr Glu Ile Asp
   100
<210> 332
   <211> 104
   <212> **PRT**
   <213> **Artificial** Sequence
<220>
   <223> Adnectin
<400> 332
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Arg Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 333
   <211> 104
   <212> **PRT**
   <213> **Artificial** Sequence
<220>
   <223> Adnectin
<400> 333
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ser Pro Trp Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 334
   <211> 104
   <212> PRT
   <213> Artificial Sequence
<220>
   <223> Adnectin
<400> 334
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Thr Tyr Arg Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 335
   <211> 104
   <212> **PRT**
   <213> **Artificial** Sequence
<220>
   <223> Adnectin
<400> 335
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Glu Ser Ala Pro Ile Ser
   85 90 95
   Ile Asn Tyr Arg Thr Glu Ile Asp
   100
<210> 336
   <211> 104
   <212> **PRT**
   <213> **Artificial** Sequence
<220>
   <223> Adnectin
<400> 336
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Asp Pro Ile Ser
                   85                  90                  95
   Ile Asn Tyr Arg Thr Glu Ile Asp
               100
<210> 337
   <211> 104
   <212> **PRT**
   <213> **Artificial** Sequence
<220>
   <223> Adnectin
<400> 337
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Tyr Pro Ile Ser
                   85                  90                  95
   Ile Asn Tyr Arg Thr Glu Ile Asp
               100
<210> 338
   <211> 104
   <212> **PRT**
   <213> **Artificial** Sequence
<220>
   <223> Adnectin
<400> 338
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Arg Tyr Arg Tyr Tyr Arg Ile Thr Tyr Gly
           35                  40                  45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 339
   <211> 104
   <212> **PRT**
   <213> **Artificial** Sequence
<220>
   <223> Adnectin
<400> 339
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ser Arg Trp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 340
   <211> 104
   <212> PRT
   <213> Artificial Sequence
<220>
   <223> Adnectin
<220>
   <221> misc_feature
   <222> (38)..(39)
   <223> Xaa can be any naturally occurring amino acid
<400> 340
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Xaa Xaa Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 341
   <211> 104
   <212> **PRT**
   <213> **Artificial** Sequence
<220>
   <223> Adnectin
<400> 341
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Ser Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 342
   <211> 104
   <212> **PRT**
   <213> **Artificial** Sequence
<220>
   <223> Adnectin
<400> 342
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Ile Asp Ser Ala Pro Ile Ser
                   85                  90                  95
   Ile Asn Tyr Arg Thr Glu Ile Asp
               100
<210> 343
   <211> 104
   <212> PRT
   <213> Artificial Sequence
<220>
   <223> Adnectin
<400> 343
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp His Ser Ala Pro Ile Ser
   85 90 95
   Ile Asn Tyr Arg Thr Glu Ile Asp
   100
<210> 344
   <211> 104
   <212> **PRT**
   <213> **Artificial Sequence**
<220>
   <223> Adnectin
<400> 344
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Glu Pro Ile Ser
   85 90 95
   Ile Asn Tyr Arg Thr Glu Ile Asp
   100
<210> 345
   <211> 104
   <212> **PRT**
   <213> Artificial Sequence
<220>
   <223> Adnectin
<400> 345
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Arg Pro Ile Ser
                   85                  90                  95
   Ile Asn Tyr Arg Thr Glu Ile Asp
               100
<210> 346
   <211> 104
   <212> **PRT**
   <213> **Artificial** Sequence
<220>
   <223> Adnectin
<400> 346
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
   85 90 95
   Ile Gln Tyr Arg Thr Glu Ile Asp
   100
<210> 347
   <211> 104
   <212> **PRT**
   <213> **Artificial** Sequence
<220>
   <223> Adnectin
<400> 347
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asn Tyr Arg Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 348
   <211> 104
   <212> PRT
   <213> Artificial Sequence
<220>
   <223> Adnectin
<400> 348
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ser Thr Trp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 349
   <211> 104
   <212> **PRT**
   <213> **Artificial** Sequence
<220>
   <223> Adnectin
<400> 349
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Tyr Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 350
   <211> 104
   <212> PRT
   <213> Artificial Sequence
<220>
   <223> Adnectin
<400> 350
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Thr Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 351
   <211> 104
   <212> PRT
   <213> Artificial Sequence
<220>
   <223> Adnectin
<400> 351
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Ala Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Lys Asp Ser Ala Pro Ile Ser
                   85                  90                  95
   Ile Asn Tyr Arg Thr Glu Ile Asp
               100
<210> 352
   <211> 104
   <212> PRT
   <213> Artificial Sequence
<220>
   <223> Adnectin
<400> 352
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Leu Ser Ala Pro Ile Ser
                   85                  90                  95
   Ile Asn Tyr Arg Thr Glu Ile Asp
               100
<210> 353
   <211> 104
   <212> **PRT**
   <213> **Artificial** Sequence
<220>
   <223> Adnectin
<400> 353
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Gly Pro Ile Ser
   85 90 95
   Ile Asn Tyr Arg Thr Glu Ile Asp
   100
<210> 354
   <211> 104
   <212> **PRT**
   <213> **Artificial** Sequence
<220>
   <223> Adnectin
<400> 354
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ser Ala Trp Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 355
   <211> 104
   <212> PRT
   <213> Artificial Sequence
<220>
   <223> Adnectin
<400> 355
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ser Thr Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 356
   <211> 104
   <212> PRT
   <213> Artificial Sequence
<220>
   <223> Adnectin
<400> 356
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Glu Leu Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 357
   <211> 104
   <212> PRT
   <213> Artificial Sequence
<220>
   <223> Adnectin
<400> 357
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Leu Ser Ala Pro Ile Ser
                   85                  90                  95
   Ile Asn Tyr Arg Thr Glu Ile Asp
               100
<210> 358
   <211> 104
   <212> PRT
   <213> Artificial Sequence
<220>
   <223> Adnectin
<400> 358
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Thr Pro Ile Ser
   85 90 95
   Ile Asn Tyr Arg Thr Glu Ile Asp
   100
<210> 359
   <211> 104
   <212> PRT
   <213> Artificial Sequence
<220>
   <223> Adnectin
<400> 359
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Ser Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp
   100
<210> 360
   <211> 104
   <212> PRT
   <213> Artificial Sequence
<220>
   <223> Adnectin
<400> 360
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Thr Gly Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 361
   <211> 104
   <212> PRT
   <213> Artificial Sequence
<220>
   <223> Adnectin
<400> 361
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Tyr His Gly Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 362
   <211> 104
   <212> PRT
   <213> Artificial Sequence
<220>
   <223> Adnectin
<400> 362
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Lys Leu Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 363
   <211> 104
   <212> **PRT**
   <213> **Artificial** Sequence
<220>
   <223> Adnectin
<400> 363
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asn Asp Ser Ala Pro Ile Ser
   85 90 95
   Ile Asn Tyr Arg Thr Glu Ile Asp
   100
<210> 364
   <211> 104
   <212> **PRT**
   <213> **Artificial** Sequence
<220>
   <223> Adnectin
<400> 364
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Pro Ser Ala Pro Ile Ser
   85 90 95
   Ile Asn Tyr Arg Thr Glu Ile Asp
   100
<210> 365
   <211> 104
   <212> PRT
   <213> Artificial Sequence
<220>
   <223> Adnectin
<400> 365
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Ser Gly Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 366
   <211> 104
   <212> PRT
   <213> Artificial Sequence
<220>
   <223> Adnectin
<400> 366
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Tyr His Asn Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 367
   <211> 104
   <212> PRT
   <213> Artificial Sequence
<220>
   <223> Adnectin
<400> 367
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Ser Ala Gln Leu Ser Gly Leu Lys Pro Gly Val Asp Tyr Thr
   65                  70                  75                  80
   Ile Thr Val Tyr Ala Val Thr Tyr Gly Asp Asp Ser Ala Pro Ile Ser
                   85                  90                  95
   Ile Asn Tyr Arg Thr Glu Ile Asp
               100
<210> 368
   <211> 104
   <212> PRT
   <213> Artificial Sequence
<220>
   <223> Adnectin
<400> 368
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Thr Asp Ser Ala Pro Ile Ser
                   85                  90                  95
   Ile Asn Tyr Arg Thr Glu Ile Asp
               100
<210> 369
   <211> 104
   <212> PRT
   <213> Artificial Sequence
<220>
   <223> Adnectin
<400> 369
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Pro Ser Ala Pro Ile Ser
                   85                  90                  95
   Ile Asn Tyr Arg Thr Glu Ile Asp
               100
<210> 370
   <211> 104
   <212> PRT
   <213> Artificial Sequence
<220>
   <223> Adnectin
<400> 370
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Asp Asp Ser Lys Pro Ile Ser
                   85                  90                  95
   Ile Asn Tyr Arg Thr Glu Ile Asp
               100
<210> 371
   <211> 104
   <212> PRT
   <213> Artificial Sequence
<220>
   <223> Adnectin
<400> 371
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val His Pro Tyr Arg Tyr Tyr Arg Ile Thr Tyr Gly
           35                  40                  45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Ile Asp Ser Thr Pro Ile Ser
   85 90 95
   Ile Asn Tyr Arg Thr Glu Ile Asp
   100
<210> 372
   <211> 33
   <212> **PRT**
   <213> **Artificial** Sequence
<220>
   <223> HIV fusion peptide inhibitor
<400> 372
   Ser Glu Tyr Glu Ala Arg Ile Glu Ala Leu Ile Arg Ala Ala Gln Glu
   1 5 10 15
   Gln Gln Glu Lys Asn Glu Ala Ala Leu Arg Glu Leu Tyr Lys Trp Ala
   20 25 30
   Leu
<210> 373
   <211> 33
   <212> **PRT**
   <213> **Artificial** Sequence
<220>
   <223> HIV fusion peptide inhibitor
<400> 373
   Ser Glu Tyr Glu Ala Arg Ile Glu Ala Leu Ile Arg Ala Ala Gln Glu
   1 5 10 15
   Gln Gln Glu Lys Asn Glu Ala Ala Leu Arg Glu Leu Trp Lys Trp Ala
   20 25 30
   Ser
<210> 374
   <211> 35
   <212> PRT
   <213> Artificial Sequence
<220>
   <223> HIV fusion peptide inhibitor
<400> 374
   Thr Ile Ala Glu Tyr Ala Ala Arg Ile Glu Ala Leu Ile Arg Ala Ala
   1               5                   10                  15
   Gln Glu Gln Gln Glu Lys Asn Glu Ala Ala Leu Arg Glu Leu Tyr Lys
               20                  25                  30
   Trp Ala Ser
           35
<210> 375
   <211> 36
   <212> PRT
   <213> Artificial Sequence
<220>
   <223> HIV fusion peptide inhibitor
<400> 375
   Ala Arg Ile Glu Glu Tyr Ala Ala Arg Ile Glu Ala Leu Ile Arg Ala
   1               5                   10                  15
   Ala Gln Glu Gln Gln Glu Lys Asn Glu Ala Ala Leu Arg Glu Leu Tyr
               20                  25                  30
   Lys Trp Ala Ser
           35
<210> 376
   <211> 33
   <212> PRT
   <213> Artificial Sequence
<220>
   <223> HIV fusion peptide inhibitor
<400> 376
   Ser Glu Tyr Glu Ala Arg Ile Glu Ala Leu Ile Arg Ala Ala Gln Glu
   1               5                   10                  15
   Gln Gln Glu Lys Asn Glu Ala Ala Leu Arg Glu Leu Tyr Lys Trp Ala
               20                  25                  30
   Ser
<210> 377
   <211> 36
   <212> PRT
   <213> Artificial Sequence
<220>
   <223> HIV fusion peptide inhibitor
<400> 377
   Thr Glu Tyr Glu Ala Arg Ile Glu Ala Leu Ile Arg Ala Ala Gln Glu
   1               5                   10                  15
   Gln Gln Glu Lys Asn Glu Ala Ala Leu Arg Glu Leu Lys Glu Trp Ala
               20                  25                  30
   Ser Ile Trp Asn
           35
<210> 378
   <211> 41
   <212> **PRT**
   <213> **Artificial** Sequence
<220>
   <223> HIV fusion peptide inhibitor
<400> 378
   Ser Glu Tyr Glu Ala Arg Ile Glu Ala Leu Ile Arg Ala Ala Gln Glu
   1               5                   10                  15
   Gln Gln Glu Lys Asn Glu Ala Ala Leu Arg Glu Leu Asp Lys Trp Thr
               20                  25                  30
   Gly Val Trp Gly Asn Tyr Glu Lys Val
           35                  40
<210> 379
   <211> 29
   <212> PRT
   <213> Artificial Sequence
<220>
   <223> HIV fusion peptide inhibitor
<400> 379
   Ser Arg Ile Glu Ala Leu Ile Arg Ala Ala Gln Glu Gln Gln Glu Lys
   1               5                   10                  15
   Asn Glu Ala Ala Leu Arg Glu Leu Phe Lys Trp Ala Ser
               20                  25
<210> 380
   <211> 29
   <212> **PRT**
   <213> **Artificial** Sequence
<220>
   <223> HIV fusion peptide inhibitor
<400> 380
   Ser Arg Ile Glu Ala Leu Ile Arg Ala Ala Gln Glu Gln Gln Gln Lys
   1 5 10 15
   Asn Glu Ala Ala Leu Arg Glu Leu Asp Lys Trp Ala Ser
   20 25
<210> 381
   <211> 29
   <212> PRT
   <213> Artificial Sequence
<220>
   <223> HIV fusion peptide inhibitor
<400> 381
   Ser Arg Ile Glu Ala Leu Ile Arg Ala Ala Gln Glu Gln Gln Glu Lys
   1 5 10 15
   Asn Glu Ala Ala Leu Arg Glu Leu Tyr Lys Trp Ala Ser
   20 25
<210> 382
   <211> 29
   <212> PRT
   <213> Artificial Sequence
<220>
   <223> HIV fusion peptide inhibitor
<400> 382
   Ser Arg Ile Glu Ala Leu Ile Arg Ala Ala Gln Glu Gln Gln Glu Lys
   1 5 10 15
   Asn Glu Ala Ala Leu Arg Glu Leu Leu Lys Trp Ala Ser
   20 25
<210> 383
   <211> 29
   <212> PRT
   <213> Artificial Sequence
<220>
   <223> HIV fusion peptide inhibitor
<400> 383
   Ser Arg Ile Glu Ala Leu Ile Arg Ala Ala Gln Glu Gln Gln Glu Lys
   1 5 10 15
   Asn Glu Ala Ala Leu Arg Glu Leu Gln Lys Trp Ala Ser
   20 25
<210> 384
   <211> 29
   <212> PRT
   <213> Artificial Sequence
<220>
   <223> HIV fusion peptide inhibitor
<400> 384
   Ser Arg Ile Glu Ala Leu Ile Arg Ala Ala Gln Glu Gln Gln Glu Lys
   1 5 10 15
   Asn Glu Ala Ala Leu Arg Glu Leu Asp Lys Trp Ala Ser
   20 25
<210> 385
   <211> 35
   <212> PRT
   <213> Artificial Sequence
<220>
   <223> HIV fusion peptide inhibitor
<400> 385
   Ala Ile Ala Glu Tyr Ala Ala Arg Ile Glu Ala Leu Ile Arg Ala Ala
   1 5 10 15
   Gln Glu Gln Gln Glu Lys Asn Glu Ala Ala Leu Arg Glu Leu Asp Lys
   20 25 30
   Trp Ala Ser
   35
<210> 386
   <211> 30
   <212> PRT
   <213> Artificial Sequence
<220>
   <223> HIV fusion peptide inhibitor
<400> 386
   Thr Glu Tyr Glu Ala Arg Ile Glu Ala Leu Ile Arg Ala Ala Gln Glu
   1               5                   10                  15
   Gln Gln Glu Lys Asn Glu Ala Ala Leu Arg Glu Leu Asp Lys
               20                  25                  30
<210> 387
   <211> 29
   <212> PRT
   <213> Artificial Sequence
<220>
   <223> HIV fusion peptide inhibitor
<400> 387
   Ser Arg Ile Glu Ala Leu Ile Arg Ala Ala Gln Glu Gln Gln Glu Lys
   1               5                   10                  15
   Asn Glu Ala Ala Leu Arg Glu Leu Tyr Lys Trp Thr Ser
               20                  25
<210> 388
   <211> 32
   <212> PRT
   <213> Artificial Sequence
<220>
   <223> HIV fusion peptide inhibitor
<400> 388
   Ser Arg Ile Glu Ala Leu Ile Arg Ala Ala Gln Glu Gln Gln Glu Lys
   1               5                   10                  15
   Asn Glu Ala Ala Leu Arg Glu Leu Tyr Lys Trp Ala Ser Leu Trp Ile
               20                  25                  30
<210> 389
   <211> 32
   <212> PRT
   <213> Artificial Sequence
<220>
   <223> HIV fusion peptide inhibitor
<400> 389
   Ser Arg Ile Glu Ala Leu Ile Arg Ala Ala Gln Glu Gln Gln Glu Lys
   1               5                   10                  15
   Asn Glu Ala Ala Leu Arg Glu Leu Tyr Lys Trp Ala Ser Arg Trp Asn
               20                  25                  30
<210> 390
   <211> 32
   <212> PRT
   <213> Artificial Sequence
<220>
   <223> HIV fusion peptide inhibitor
<400> 390
   Ser Arg Ile Glu Ala Leu Ile Arg Ala Ala Gln Glu Gln Gln Glu Lys
   1               5                   10                  15
   Asn Glu Ala Ala Leu Arg Glu Leu Tyr Lys Trp Ala Ser Ser Trp Asn
               20                  25                  30
<210> 391
   <211> 29
   <212> PRT
   <213> Artificial Sequence
<220>
   <223> HIV fusion peptide inhibitor
<400> 391
   Ser Arg Ile Glu Ala Leu Ile Arg Ala Ala Gln Glu Gln Gln Glu Lys
   1               5                   10                  15
   Asn Glu Ala Ala Leu Arg Glu Leu Tyr Lys Trp Gly Ser
               20                  25
<210> 392
   <211> 26
   <212> PRT
   <213> Artificial Sequence
<220>
   <223> HIV fusion peptide inhibitor
<400> 392
   Ser Arg Ile Glu Ala Leu Ile Arg Ala Ala Gln Glu Gln Gln Glu Lys
   1               5                   10                  15
   Asn Glu Ala Ala Leu Arg Glu Leu Asp Lys
               20                  25
<210> 393
   <211> 14
   <212> PRT
   <213> Artificial Sequence
<220>
   <223> Linker (GS)7
<400> 393
   Gly Ser Gly Ser Gly Ser Gly Ser Gly Ser Gly Ser Gly Ser
   1 5 10
<210> 394
   <211> 13
   <212> PRT
   <213> Artificial Sequence
<220>
   <223> Linker G(GS)6
<400> 394
   Gly Gly Ser Gly Ser Gly Ser Gly Ser Gly Ser Gly Ser
   1 5 10
<210> 395
   <211> 16
   <212> PRT
   <213> Artificial Sequence
<220>
   <223> Linker G(GS)7G
<400> 395
   Gly Gly Ser Gly Ser Gly Ser Gly Ser Gly Ser Gly Ser Gly Ser Gly
   1 5 10 15
<210> 396
   <211> 15
   <212> PRT
   <213> Artificial Sequence
<220>
   <223> Linker (GSE)5
<400> 396
   Gly Ser Glu Gly Ser Glu Gly Ser Glu Gly Ser Glu Gly Ser Glu
   1 5 10 15
<210> 397
   <211> 8
   <212> PRT
   <213> Artificial Sequence
<220>
   <223> Linker GGSEGGSE
<400> 397
   Gly Gly Ser Glu Gly Gly Ser Glu
   1 5
<210> 398
   <211> 8
   <212> PRT
   <213> Artificial Sequence
<220>
   <223> Linker (GS)4
<400> 398
   Gly Ser Gly Ser Gly Ser Gly Ser
   1               5
<210> 399
   <211> 35
   <212> PRT
   <213> Artificial Sequence
<220>
   <223> Linker (GGGGS)7
<400> 399
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
   Gly Gly Ser
           35
<210> 400
   <211> 25
   <212> PRT
   <213> Artificial Sequence
<220>
   <223> Linker (GGGGS)5
<400> 400
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser
               20                  25
<210> 401
   <211> 20
   <212> PRT
   <213> Artificial Sequence
<220>
   <223> Linker (GGGGS)4
<400> 401
   Gly Gly Gly Gly Ser Gly Gly Gly Gly Ser Gly Gly Gly Gly Ser Gly
   1 5 10 15
   Gly Gly Gly Ser
   20
<210> 402
   <211> 16
   <212> PRT
   <213> Artificial Sequence
<220>
   <223> Linker (GGGGS)3G
<400> 402
   Gly Gly Gly Gly Ser Gly Gly Gly Gly Ser Gly Gly Gly Gly Ser Gly
   1 5 10 15
<210> 403
   <211> 7
   <212> PRT
   <213> Artificial Sequence
<220>
   <223> Linker (GP)3G
<400> 403
   Gly Pro Gly Pro Gly Pro Gly
   1 5
<210> 404
   <211> 11
   <212> PRT
   <213> Artificial Sequence
<220>
   <223> Linker (GP)5G
<400> 404
   Gly Pro Gly Pro Gly Pro Gly Pro Gly Pro Gly
   1 5 10
<210> 405
   <211> 6
   <212> PRT
   <213> Artificial Sequence
<220>
   <223> Linker (PA)3
<400> 405
   Pro Ala Pro Ala Pro Ala
   1 5
<210> 406
   <211> 12
   <212> PRT
   <213> Artificial Sequence
<220>
   <223> Linker (PA)6
<400> **406**
   Pro Ala Pro Ala Pro Ala Pro Ala Pro Ala Pro Ala
   1 5 10
<210> 407
   <211> 18
   <212> **PRT**
   <213> **Artificial** Sequence
<220>
   <223> Linker (PA)9
<400> **407**
   Pro Ala Pro Ala Pro Ala Pro Ala Pro Ala Pro Ala Pro Ala Pro Ala
   1 5 10 15
   Pro Ala
<210> 408
   <211> 11
   <212> **PRT**
   <213> **Artificial** Sequence
<220>
   <223> **Linker ESPEPETPEDE**
<400> **408**
   Glu Ser Pro Glu Pro Glu Thr Pro Glu Asp Glu
   1 5 10
<210> 409
   <211> 21
   <212> **PRT**
   <213> **Artificial** Sequence
<220>
   <223> **Linker (ESPEPETPED)2E**
<400> **409**
   Glu Ser Pro Glu Pro Glu Thr Pro Glu Asp Glu Ser Pro Glu Pro Glu
   1               5                   10                  15
   Thr Pro Glu Asp Glu
               20
<210> 410
   <211> 159
   <212> **PRT**
   <213> **Artificial** Sequence
<220>
   <223> Anti-N17 Adnectin - HIV fusion peptide inhibitor Combinectin
<400> 410
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val Asp Ser Asp Pro Ile Ser
                   85                  90                  95
   Ile Asn Tyr Arg Thr Glu Ile Asp Gly Gly Gly Gly Ser Gly Gly Gly
               100                 105                 110
   Gly Ser Gly Gly Gly Gly Ser Gly Gly Gly Gly Ala Arg Ile Glu Glu
           115                 120                 125
   Tyr Ala Ala Arg Ile Glu Ala Leu Ile Arg Ala Ala Gln Glu Gln Gln
       130                 135                 140
   Glu Lys Asn Glu Ala Ala Leu Arg Glu Leu Tyr Lys Trp Ala Ser
   145                 150                 155
<210> 411
   <211> 158
   <212> PRT
   <213> Artificial Sequence
<220>
   <223> Anti-N17 Adnectin - HIV fusion peptide inhibitor Combinectin
<400> 411
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val Asp Ser Asp Pro Ile Ser
                   85                  90                  95
   Ile Asn Tyr Arg Thr Glu Ile Asp Gly Gly Gly Gly Ser Gly Gly Gly
               100                 105                 110
   Gly Ser Gly Gly Gly Gly Ser Gly Gly Gly Gly Thr Ile Ala Glu Tyr
           115                 120                 125
   Ala Ala Arg Ile Glu Ala Leu Ile Arg Ala Ala Gln Glu Gln Gln Glu
       130                 135                 140
   Lys Asn Glu Ala Ala Leu Arg Glu Leu Tyr Lys Trp Ala Ser
   145                 150                 155
<210> 412
   <211> 156
   <212> **PRT**
   <213> Artificial Sequence
<220>
   <223> Anti-N17 Adnectin - HIV fusion peptide inhibitor Combinectin
<400> 412
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val Asp Ser Asp Pro Ile Ser
                   85                  90                  95
   Ile Asn Tyr Arg Thr Glu Ile Asp Gly Gly Gly Gly Ser Gly Gly Gly
               100                 105                 110
   Gly Ser Gly Gly Gly Gly Ser Gly Gly Gly Gly Ser Glu Tyr Glu Ala
           115                 120                 125
   Arg Ile Glu Ala Leu Ile Arg Ala Ala Gln Glu Gln Gln Glu Lys Asn
       130                 135                 140
   Glu Ala Ala Leu Arg Glu Leu Tyr Lys Trp Ala Ser
   145                 150                 155
<210> 413
   <211> 157
   <212> **PRT**
   <213> **Artificial** Sequence
<220>
   <223> Anti-N17 Adnectin - HIV fusion peptide inhibitor Combinectin
<400> 413
   Met Gly His His His His His His Gly Val Ser Asp Val Pro Arg Asp
   1               5                   10                  15
   Leu Glu Val Val Ala Ala Thr Pro Thr Ser Leu Leu Ile Ser Trp Lys
               20                  25                  30
   Tyr Lys Val His Pro Tyr Arg Tyr Tyr Arg Ile Thr Tyr Gly Glu Thr
           35                  40                  45
   Gly Gly Asn Ser Pro Val Gln Glu Phe Thr Val Pro Ser Val Leu Ser
       50                  55                  60
   Thr Ala Thr Ile Ser Gly Leu Lys Pro Gly Val Asp Tyr Thr Ile Thr
   65                  70                  75                  80
   Val Tyr Ala Val Thr Tyr Gly Val Asn Ser Leu Pro Ile Ser Ile Asn
                   85                  90                  95
   Tyr Arg Thr Glu Ile Asp Gly Gly Gly Gly Ser Gly Gly Gly Gly Ser
               100                 105                 110
   Gly Gly Gly Gly Ser Gly Gly Gly Gly Thr Glu Tyr Glu Ala Arg Ile
           115                 120                 125
   Glu Ala Leu Ile Arg Ala Ala Gln Glu Gln Gln Glu Lys Asn Glu Ala
       130                 135                 140
   Ala Leu Arg Glu Leu Lys Glu Trp Ala Ser Ile Trp Asn
   145                 150                 155
<210> 414
   <211> 168
   <212> PRT
   <213> Artificial Sequence
<220>
   <223> Anti-N17 Adnectin - HIV fusion peptide inhibitor Combinectin
<400> 414
   Met Gly His His His His His His Thr Ser Gly Val Ser Asp Val Pro
   1               5                   10                  15
   Arg Asp Leu Glu Val Val Ala Ala Thr Pro Thr Ser Leu Leu Ile Ser
               20                  25                  30
   Trp Asp Ala Pro Ala Val Thr Val Gly Trp Tyr His Ile Gly Tyr Asn
           35                  40                  45
   Val Glu Gly Glu Pro Ala Ser Tyr Gln Tyr Phe Arg Val Pro Gly Ser
       50                  55                  60
   Lys Ser Thr Ala Thr Ile Ser Gly Leu Lys Pro Gly Val Glu Tyr Met
   65                  70                  75                  80
   Ile Phe Val Asn Ala Val Thr Gly Ser Gly Ala Arg Glu Glu Phe Ser
                   85                  90                  95
   Leu Pro Ile Ser Ile Asn Tyr Arg Thr Glu Ile Asp Gly Gly Gly Gly
               100                 105                 110
   Ser Gly Gly Gly Gly Ser Gly Gly Gly Gly Ser Gly Gly Gly Gly Ser
           115                 120                 125
   Glu Tyr Glu Ala Arg Ile Glu Ala Leu Ile Arg Ala Ala Gln Glu Gln
       130                 135                 140
   Gln Glu Lys Asn Glu Ala Ala Leu Arg Glu Leu Asp Lys Trp Thr Gly
   145                 150                 155                 160
   Val Trp Gly Asn Tyr Glu Lys Val
                   165
<210> 415
   <211> 154
   <212> PRT
   <213> Artificial Sequence
<220>
   <223> Anti-N17 Adnectin - HIV fusion peptide inhibitor Combinectin
<400> 415
   Met Gly His His His His His His Gly Val Ser Asp Val Pro Arg Asp
   1 5 10 15
   Leu Glu Val Val Ala Ala Thr Pro Thr Ser Leu Leu Ile Ser Trp Asp
   20 25 30
   Ala Pro Ala Val Thr Val Arg Tyr Tyr Arg Ile Thr Tyr Gly Glu Thr
   35 40 45
   Gly Gly Asn Ser Pro Val Gln Glu Phe Thr Val Pro Gly Ser Lys Ser
   50 55 60
   Thr Ala Thr Ile Ser Gly Leu Lys Pro Gly Val Asp Tyr Thr Ile Thr
   65 70 75 80
   Val Tyr Ala Val Thr Gly Arg Gly Glu Ser Pro Ala Ser Ser Lys Pro
   85 90 95
   Ile Ser Ile Asn Tyr Arg Thr Glu Ile Asp Gly Gly Gly Gly Ser Gly
   100 105 110
   Gly Gly Gly Ser Gly Gly Gly Gly Ser Gly Gly Gly Gly Ser Arg Ile
   115 120 125
   Glu Ala Leu Ile Arg Ala Ala Gln Glu Gln Gln Glu Lys Asn Glu Ala
   130 135 140
   Ala Leu Arg Glu Leu Phe Lys Trp Ala Ser
   145 150
<210> 416
   <211> 154
   <212> PRT
   <213> Artificial Sequence
<220>
   <223> Anti-N17 Adnectin - HIV fusion peptide inhibitor Combinectin
<400> 416
   Met Gly His His His His His His Gly Val Ser Asp Val Pro Arg Asp
   1 5 10 15
   Leu Glu Val Val Ala Ala Thr Pro Thr Ser Leu Leu Ile Ser Trp Asp
   20 25 30
   Ala Pro Ala Val Thr Val Arg Tyr Tyr Arg Ile Thr Tyr Gly Glu Thr
   35 40 45
   Gly Gly Asn Ser Pro Val Gln Glu Phe Thr Val Pro Gly Ser Lys Ser
   50 55 60
   Thr Ala Thr Ile Ser Gly Leu Lys Pro Gly Val Asp Tyr Thr Ile Thr
   65 70 75 80
   Val Tyr Ala Val Thr Gly Arg Gly Glu Ser Pro Ala Ser Ser Lys Pro
   85 90 95
   Ile Ser Ile Asn Tyr Arg Thr Glu Ile Asp Gly Gly Gly Gly Ser Gly
   100 105 110
   Gly Gly Gly Ser Gly Gly Gly Gly Ser Gly Gly Gly Gly Ser Arg Ile
   115 120 125
   Glu Ala Leu Ile Arg Ala Ala Gln Glu Gln Gln Gln Lys Asn Glu Ala
   130 135 140
   Ala Leu Arg Glu Leu Asp Lys Trp Ala Ser
   145 150
<210> 417
   <211> 154
   <212> PRT
   <213> Artificial Sequence
<220>
   <223> Anti-N17 Adnectin - HIV fusion peptide inhibitor Combinectin
<400> 417
   Met Gly His His His His His His Gly Val Ser Asp Val Pro Arg Asp
   1 5 10 15
   Leu Glu Val Val Ala Ala Thr Pro Thr Ser Leu Leu Ile Ser Trp Asp
   20 25 30
   Ala Pro Ala Val Thr Val Arg Tyr Tyr Arg Ile Thr Tyr Gly Glu Thr
   35 40 45
   Gly Gly Asn Ser Pro Val Gln Glu Phe Thr Val Pro Gly Ser Lys Ser
   50 55 60
   Thr Ala Thr Ile Ser Gly Leu Lys Pro Gly Val Asp Tyr Thr Ile Thr
   65 70 75 80
   Val Tyr Ala Val Thr Gly Arg Gly Glu Ser Pro Ala Ser Ser Lys Pro
   85 90 95
   Ile Ser Ile Asn Tyr Arg Thr Glu Ile Asp Gly Gly Gly Gly Ser Gly
   100 105 110
   Gly Gly Gly Ser Gly Gly Gly Gly Ser Gly Gly Gly Gly Ser Arg Ile
   115 120 125
   Glu Ala Leu Ile Arg Ala Ala Gln Glu Gln Gln Glu Lys Asn Glu Ala
   130 135 140
   Ala Leu Arg Glu Leu Tyr Lys Trp Ala Ser
   145 150
<210> 418
   <211> 154
   <212> **PRT**
   <213> **Artificial** Sequence
<220>
   <223> Anti-N17 Adnectin - HIV fusion peptide inhibitor Combinectin
<400> **418**
   Met Gly His His His His His His Gly Val Ser Asp Val Pro Arg Asp
   1               5                   10                  15
   Leu Glu Val Val Ala Ala Thr Pro Thr Ser Leu Leu Ile Ser Trp Asp
               20                  25                  30
   Ala Pro Ala Val Thr Val Arg Tyr Tyr Arg Ile Thr Tyr Gly Glu Thr
           35                  40                  45
   Gly Gly Asn Ser Pro Val Gln Glu Phe Thr Val Pro Gly Ser Lys Ser
       50                  55                  60
   Thr Ala Thr Ile Ser Gly Leu Lys Pro Gly Val Asp Tyr Thr Ile Thr
   65                  70                  75                  80
   Val Tyr Ala Val Thr Gly Arg Gly Glu Ser Pro Ala Ser Ser Lys Pro
                   85                  90                  95
   Ile Ser Ile Asn Tyr Arg Thr Glu Ile Asp Gly Gly Gly Gly Ser Gly
               100                 105                 110
   Gly Gly Gly Ser Gly Gly Gly Gly Ser Gly Gly Gly Gly Ser Arg Ile
           115                 120                 125
   Glu Ala Leu Ile Arg Ala Ala Gln Glu Gln Gln Glu Lys Asn Glu Ala
               130                 135                 140
   Ala Leu Arg Glu Leu Leu Lys Trp Ala Ser
   145                 150
<210> 419
   <211> 154
   <212> **PRT**
   <213> **Artificial** Sequence
<220>
   <223> Anti-N17 Adnectin - HIV fusion peptide inhibitor Combinectin
<400> **419**
   Met Gly His His His His His His Gly Val Ser Asp Val Pro Arg Asp
   1               5                   10                  15
   Leu Glu Val Val Ala Ala Thr Pro Thr Ser Leu Leu Ile Ser Trp Asp
               20                  25                  30
   Ala Pro Ala Val Thr Val Arg Tyr Tyr Arg Ile Thr Tyr Gly Glu Thr
           35                  40                  45
   Gly Gly Asn Ser Pro Val Gln Glu Phe Thr Val Pro Gly Ser Lys Ser
       50                  55                  60
   Thr Ala Thr Ile Ser Gly Leu Lys Pro Gly Val Asp Tyr Thr Ile Thr
   65                  70                  75                  80
   Val Tyr Ala Val Thr Gly Arg Gly Glu Ser Pro Ala Ser Ser Lys Pro
                   85                  90                  95
   Ile Ser Ile Asn Tyr Arg Thr Glu Ile Asp Gly Gly Gly Gly Ser Gly
               100                 105                 110
   Gly Gly Gly Ser Gly Gly Gly Gly Ser Gly Gly Gly Gly Ser Arg Ile
           115                 120                 125
   Glu Ala Leu Ile Arg Ala Ala Gln Glu Gln Gln Glu Lys Asn Glu Ala
       130                 135                 140
   Ala Leu Arg Glu Leu Gln Lys Trp Ala Ser
   145                 150
<210> 420
   <211> 152
   <212> PRT
   <213> Artificial Sequence
<220>
   <223> Anti-N17 Adnectin - HIV fusion peptide inhibitor Combinectin
<400> 420
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val His Pro Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Asp Asp Ser Ala Pro Ile Ser
   85 90 95
   Ile Asn Tyr Arg Thr Glu Ile Asp Gly Gly Gly Gly Ser Gly Gly Gly
   100 105 110
   Gly Ser Gly Gly Gly Gly Ser Gly Gly Gly Gly Ser Arg Ile Glu Ala
   115 120 125
   Leu Ile Arg Ala Ala Gln Glu Gln Gln Glu Lys Asn Glu Ala Ala Leu
   130 135 140
   Arg Glu Leu Asp Lys Trp Ala Ser
   145 150
<210> 421
   <211> 158
   <212> PRT
   <213> Artificial Sequence
<220>
   <223> Anti-N17 Adnectin - HIV fusion peptide inhibitor Combinectin
<400> 421
   Met Gly His His His His His His Gly Val Ser Asp Val Pro Arg Asp
   1 5 10 15
   Leu Glu Val Val Ala Ala Thr Pro Thr Ser Leu Leu Ile Ser Trp Val
   20 25 30
   Ala Gly Ala Glu Asp Tyr Gln Tyr Tyr Arg Ile Thr Tyr Gly Glu Thr
   35 40 45
   Gly Gly Asn Ser Pro Val Gln Glu Phe Thr Val Pro His Asp Leu Val
   50 55 60
   Thr Ala Thr Ile Ser Gly Leu Lys Pro Gly Val Asp Tyr Thr Ile Thr
   65 70 75 80
   Val Tyr Ala Val Thr Asp Met Met His Val Glu Tyr Thr Glu His Pro
   85 90 95
   Ile Ser Ile Asn Tyr Arg Thr Glu Ile Asp Gly Gly Gly Gly Ser Gly
   100 105 110
   Gly Gly Gly Ser Gly Gly Gly Gly Ser Gly Gly Ala Ile Ala Glu Tyr
   115 120 125
   Ala Ala Arg Ile Glu Ala Leu Ile Arg Ala Ala Gln Glu Gln Gln Glu
   130 135 140
   Lys Asn Glu Ala Ala Leu Arg Glu Leu Asp Lys Trp Ala Ser
   145 150 155
<210> 422
   <211> 151
   <212> PRT
   <213> Artificial Sequence
<220>
   <223> Anti-N17 Adnectin - HIV fusion peptide inhibitor Combinectin
<400> 422
   Met Gly His His His His His His Gly Val Ser Asp Val Pro Arg Asp
   1               5                   10                  15
   Leu Glu Val Val Ala Ala Thr Pro Thr Ser Leu Leu Ile Ser Trp Lys
               20                  25                  30
   Tyr Lys Val His Pro Tyr Arg Tyr Tyr Arg Ile Thr Tyr Gly Glu Thr
           35                  40                  45
   Gly Gly Asn Ser Pro Val Gln Glu Phe Thr Val Pro Ser Val Leu Ser
       50                  55                  60
   Thr Ala Thr Ile Ser Gly Leu Lys Pro Gly Val Asp Tyr Thr Ile Thr
   65                  70                  75                  80
   Val Tyr Ala Val Thr Tyr Gly Val Asn Ser Leu Pro Ile Ser Ile Asn
                   85                  90                  95
   Tyr Arg Thr Glu Ile Asp Gly Gly Gly Gly Ser Gly Gly Gly Gly Ser
               100                 105                 110
   Gly Gly Gly Gly Ser Gly Gly Gly Gly Thr Glu Tyr Glu Ala Arg Ile
           115                 120                 125
   Glu Ala Leu Ile Arg Ala Ala Gln Glu Gln Gln Glu Lys Asn Glu Ala
       130                 135                 140
   Ala Leu Arg Glu Leu Asp Lys
   145                 150
<210> 423
   <211> 154
   <212> **PRT**
   <213> **Artificial** Sequence
<220>
   <223> Anti-N17 Adnectin - HIV fusion peptide inhibitor Combinectin
<400> 423
   Met Gly His His His His His His Gly Val Ser Asp Val Pro Arg Asp
   1               5                   10                  15
   Leu Glu Val Val Ala Ala Thr Pro Thr Ser Leu Leu Ile Ser Trp Asp
               20                  25                  30
   Ala Pro Ala Val Thr Val Arg Tyr Tyr Arg Ile Thr Tyr Gly Glu Thr
           35                  40                  45
   Gly Gly Asn Ser Pro Val Gln Glu Phe Thr Val Pro Gly Ser Lys Ser
       50                  55                  60
   Thr Ala Thr Ile Ser Gly Leu Lys Pro Gly Val Asp Tyr Thr Ile Thr
   65                  70                  75                  80
   Val Tyr Ala Val Thr Gly Arg Gly Glu Ser Pro Ala Ser Ser Lys Pro
                   85                  90                  95
   Ile Ser Ile Asn Tyr Arg Thr Glu Ile Asp Gly Gly Gly Gly Ser Gly
               100                 105                 110
   Gly Gly Gly Ser Gly Gly Gly Gly Ser Gly Gly Gly Gly Ser Arg Ile
           115                 120                 125
   Glu Ala Leu Ile Arg Ala Ala Gln Glu Gln Gln Glu Lys Asn Glu Ala
       130                 135                 140
   Ala Leu Arg Glu Leu Tyr Lys Trp Thr Ser
   145                 150
<210> 424
   <211> 159
   <212> PRT
   <213> Artificial Sequence
<220>
   <223> Anti-N17 Adnectin - HIV fusion peptide inhibitor Combinectin
<400> 424
   Met Gly His His His His His His Gly Gly Ser Val Ser Asp Val Pro
   1 5 10 15
   Arg Asp Leu Glu Val Val Ala Ala Thr Pro Thr Ser Leu Leu Ile Ser
   20 25 30
   Trp Glu Tyr Lys Val Asn Ala Tyr Arg Tyr Tyr Arg Ile Thr Tyr Gly
   35 40 45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val Asp Ser Asp Pro Ile Ser
   85 90 95
   Ile Asn Tyr Arg Thr Glu Ile Asp Gly Gly Gly Gly Ser Gly Gly Gly
   100 105 110
   Gly Ser Gly Gly Gly Gly Ser Gly Gly Gly Gly Ala Arg Ile Glu Glu
   115 120 125
   Tyr Ala Ala Arg Ile Glu Ala Leu Ile Arg Ala Ala Gln Glu Gln Gln
   130 135 140
   Glu Lys Asn Glu Ala Ala Leu Arg Glu Leu Tyr Lys Trp Ala Ser
   145 150 155
<210> 425
   <211> 158
   <212> PRT
   <213> Artificial Sequence
<220>
   <223> Anti-N17 Adnectin - HIV fusion peptide inhibitor Combinectin
<400> 425
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
       50                  55                  60
   Leu Ser Thr Ala Thr Ile Ser Gly Leu Lys Pro Gly Val Asp Tyr Thr
   65                  70                  75                  80
   Ile Thr Val Tyr Ala Val Thr Tyr Gly Val Asp Ser Asp Pro Ile Ser
                   85                  90                  95
   Ile Asn Tyr Arg Thr Glu Ile Asp Gly Gly Gly Gly Ser Gly Gly Gly
               100                 105                 110
   Gly Ser Gly Gly Gly Gly Ser Gly Gly Gly Gly Thr Ile Ala Glu Tyr
           115                 120                 125
   Ala Ala Arg Ile Glu Ala Leu Ile Arg Ala Ala Gln Glu Gln Gln Glu
       130                 135                 140
   Lys Asn Glu Ala Ala Leu Arg Glu Leu Tyr Lys Trp Ala Ser
   145                 150                 155
<210> 426
   <211> 156
   <212> PRT
   <213> Artificial Sequence
<220>
   <223> Anti-N17 Adnectin - HIV fusion peptide inhibitor Combinectin
<400> 426
   Met Gly His His His His His His Gly Gly Ser Val Ser Asp Val Pro
   1               5                   10                  15
   Arg Asp Leu Glu Val Val Ala Ala Thr Pro Thr Ser Leu Leu Ile Ser
               20                  25                  30
   Trp Glu Tyr Lys Val Asn Ala Tyr Arg Tyr Tyr Arg Ile Thr Tyr Gly
           35                  40                  45
   Glu Thr Gly Gly Asn Ser Pro Val Gln Glu Phe Thr Val Pro Ser Val
   50 55 60
   Leu Ser Thr Ala Thr Ile Ser Gly Leu Lys Pro Gly Val Asp Tyr Thr
   65 70 75 80
   Ile Thr Val Tyr Ala Val Thr Tyr Gly Val Asp Ser Asp Pro Ile Ser
   85 90 95
   Ile Asn Tyr Arg Thr Glu Ile Asp Gly Gly Gly Gly Ser Gly Gly Gly
   100 105 110
   Gly Ser Gly Gly Gly Gly Ser Gly Gly Gly Gly Ser Glu Tyr Glu Ala
   115 120 125
   Arg Ile Glu Ala Leu Ile Arg Ala Ala Gln Glu Gln Gln Glu Lys Asn
   130 135 140
   Glu Ala Ala Leu Arg Glu Leu Tyr Lys Trp Ala Ser
   145 150 155
<210> 427
   <211> 157
   <212> PRT
   <213> Artificial Sequence
<220>
   <223> Anti-N17 Adnectin - HIV fusion peptide inhibitor Combinectin
<400> 427
   Met Gly His His His His His His Gly Val Ser Asp Val Pro Arg Asp
   1 5 10 15
   Leu Glu Val Val Ala Ala Thr Pro Thr Ser Leu Leu Ile Ser Trp Lys
   20 25 30
   Tyr Lys Val His Pro Tyr Arg Tyr Tyr Arg Ile Thr Tyr Gly Glu Thr
   35 40 45
   Gly Gly Asn Ser Pro Val Gln Glu Phe Thr Val Pro Ser Val Leu Ser
   50 55 60
   Thr Ala Thr Ile Ser Gly Leu Lys Pro Gly Val Asp Tyr Thr Ile Thr
   65 70 75 80
   Val Tyr Ala Val Thr Tyr Gly Val Asn Ser Leu Pro Ile Ser Ile Asn
   85 90 95
   Tyr Arg Thr Glu Ile Asp Gly Gly Gly Gly Ser Gly Gly Gly Gly Ser
   100 105 110
   Gly Gly Gly Gly Ser Gly Gly Gly Gly Thr Glu Tyr Glu Ala Arg Ile
   115 120 125
   Glu Ala Leu Ile Arg Ala Ala Gln Glu Gln Gln Glu Lys Asn Glu Ala
   130 135 140
   Ala Leu Arg Glu Leu Lys Glu Trp Ala Ser Ile Trp Asn
   145 150 155
<210> 428
   <211> 168
   <212> PRT
   <213> Artificial Sequence
<220>
   <223> Anti-N17 Adnectin - HIV fusion peptide inhibitor Combinectin
<400> 428
   Met Gly His His His His His His Thr Ser Gly Val Ser Asp Val Pro
   1               5                   10                  15
   Arg Asp Leu Glu Val Val Ala Ala Thr Pro Thr Ser Leu Leu Ile Ser
               20                  25                  30
   Trp Asp Ala Pro Ala Val Thr Val Gly Trp Tyr His Ile Gly Tyr Asn
           35                  40                  45
   Val Glu Gly Glu Pro Ala Ser Tyr Gln Tyr Phe Arg Val Pro Gly Ser
       50                  55                  60
   Lys Ser Thr Ala Thr Ile Ser Gly Leu Lys Pro Gly Val Glu Tyr Met
   65                  70                  75                  80
   Ile Phe Val Asn Ala Val Thr Gly Ser Gly Ala Arg Glu Glu Phe Ser
                   85                  90                  95
   Leu Pro Ile Ser Ile Asn Tyr Arg Thr Glu Ile Asp Gly Gly Gly Gly
               100                 105                 110
   Ser Gly Gly Gly Gly Ser Gly Gly Gly Gly Ser Gly Gly Gly Gly Ser
           115                 120                 125
   Glu Tyr Glu Ala Arg Ile Glu Ala Leu Ile Arg Ala Ala Gln Glu Gln
       130                 135                 140
   Gln Glu Lys Asn Glu Ala Ala Leu Arg Glu Leu Asp Lys Trp Thr Gly
   145 150 155 160
   Val Trp Gly Asn Tyr Glu Lys Val
   165